(19) Europäisches Patentamt
European Patent Office
Office européen des brevets

(11) EP 2 054 431 B1

(12) **EUROPEAN PATENT SPECIFICATION**

(45) Date of publication and mention of the grant of the patent:
**31.08.2011 Bulletin 2011/35**

(21) Application number: **07875131.0**

(22) Date of filing: **11.06.2007**

(51) Int Cl.:
***C07K 14/315*** (2006.01) ***A61K 39/09*** (2006.01)
***C07K 16/12*** (2006.01) ***G01N 33/577*** (2006.01)
***G01N 33/569*** (2006.01) ***A61K 39/40*** (2006.01)
***A61P 31/04*** (2006.01)

(86) International application number:
**PCT/IB2007/004640**

(87) International publication number:
**WO 2009/030978 (12.03.2009 Gazette 2009/11)**

(54) **CONFORMERS OF BACTERIAL ADHESINS**

BAKTERIELLE ADHÄSINE KONFORMERE

CONFORMÈRES D'ADHÉSINES BACTÉRIENNES

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **09.06.2006 US 812145 P**

(43) Date of publication of application:
**06.05.2009 Bulletin 2009/19**

(73) Proprietor: **Novartis AG**
**4056 Basel (CH)**

(72) Inventors:
- **MAIONE, Domenico**
  **Emeryville, CA 94662-8097 (US)**
- **NORAIS, Nathalie**
  **Emeryville, CA 94662-8097 (US)**
- **GRANDI, Guido**
  **Emeryville, CA 94662-8097 (US)**
- **NARDI-DEI, Vincenzo**
  **53100 Siena (IT)**

(74) Representative: **Bullett, Rachel Margaret et al**
**Carpmaels & Ransford**
**One Southampton Row**
**London**
**WC1B 5HA (GB)**

(56) References cited:
**WO-A2-2005/028618**

- **DOMENICO MAIONE ET AL.: "Identification of a universal Group B Streptococcus vaccine by multiple genome screening" SCIENCE, vol. 309, 1 July 2005 (2005-07-01), pages 148-150, XP002523293**
- **PETER LAUER ET AL.: "Genome analysis reveals pili in Group B Streptococcus" SCIENCE, vol. 309, 1 July 2005 (2005-07-01), page 105, XP002523294**
- **VERDONCK F ET AL: "Oral immunization of piglets with recombinant F4 fimbrial adhesin FaeG monomers induces a mucosal and systemic F4-specific immune response" VACCINE, vol. 22, no. 31-32, 22 October 2004 (2004-10-22), pages 4291-4299, XP002523295 ISSN: 0264-410X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> NOVARTIS AG
Maione, Domenico
Norais, Nathalie
Grandi, Guido

<120> CONFORMERS OF BACTERIAL ADHESINS

<130> 22300-31014.00

<140> PCT/
<141> 11 June 2007

<ism> 60/812145
<151> 09 June 2006

<160> 13

<170> FastSEQ for Windows Version 4.0

<210> 1
<211> 1662
<212> DNA
<213> Streptococcus Agalactiae

<400> 1

```
atgaaattat cgaagaagtt attgttttcg gctgctgttt taacaatggt ggcggggtca 60
actgttgaac cagtagctca gtttgcgact ggaatgagta ttgtaagagc tgcagaagtg 120
tcacaagaac gcccagcgaa aacaacagta aatatctata aattacaagc tgatagttat 180
aaatcggaaa ttacttctaa tggtggtatc gagaataaag acggcgaagt aatatctaac 240
tatgctaaac ttggtgacaa tgtaaaaggt ttgcaaggtg tacagtttaa acgttataaa 300
gtcaagacgg atatttctgt tgatgaattg aaaaaattga caacagttga agcagcagat 360
gcaaaagttg gaacgattct tgaagaaggt gtcagtctac ctcaaaaaac taatgctcaa 420
ggtttggtcg tcgatgctct ggattcaaaa agtaatgtga gatacttgta tgtagaagat 480
ttaaagaatt caccttcaaa cattaccaaa gcttatgctg taccgtttgt gttggaatta 540
ccagttgcta actctacagg tacaggtttc ctttctgaaa ttaatattta ccctaaaaac 600
gttgtaactg atgaaccaaa aacagataaa gatgttaaaa aattaggtca ggacgatgca 660
ggttatacga ttggtgaaga attcaaatgg ttcttgaaat ctacaatccc tgccaattta 720
ggtgactatg aaaaatttga aattactgat aaatttgcag atggcttgac ttataaatct 780
gttggaaaaa tcaagattgg ttcgaaaaca ctgaatagag atgagcacta cactattgat 840
gaaccaacag ttgataacca aaatacatta aaaattacgt ttaaaccaga gaaatttaaa 900
gaaattgctg agctacttaa aggaatgacc cttgttaaaa atcaagatgc tcttgataaa 960
gctactgcaa atacagatga tgcggcattt ttggaaattc cagttgcatc aactattaat 1020
gaaaaagcag ttttaggaaa agcaattgaa aatacttttg aacttcaata tgaccatact 1080
cctgataaag ctgacaatcc aaaaccatct aatcctccaa gaaaaccaga agttcatact 1140
ggtgggaaac gatttgtaaa gaaagactca acagaaacac aaacactagg tggtgctgag 1200
tttgatttgt tggcttctga tgggacagca gtaaaatgga cagatgctct tattaaagcg 1260
aatactaata aaaactatat tgctggagaa gctgttactg ggcaaccaat caaattgaaa 1320
tcacatacag acggtacgtt tgagattaaa ggtttggctt atgcagttga tgcgaatgca 1380
gagggtacag cagtaactta caaattaaaa gaaacaaaag caccagaagg ttatgtaatc 1440
cctgataaag aaatcgagtt tacagtatca caaacatctt ataatacaaa accaactgac 1500
atcacggttg atagtgctga tgcaacacct gatacaatta aaaacaacaa acgtccttca 1560
atccctaata ctggtggtat tggtacggct atctttgtcg ctatcggtgc tgcggtgatg 1620
gcttttgctg ttaaggggat gaagcgtcgt acaaaagata ac                    1662
```

<210> 2
<211> 554
<212> PRT
<213> Streptococcus Agalactiae

<400> 2

```
Met Lys Leu Ser Lys Lys Leu Leu Phe Ser Ala Ala Val Leu Thr Met
1               5                   10                  15
Val Ala Gly Ser Thr Val Glu Pro Val Ala Gln Phe Ala Thr Gly Met
            20                  25                  30
Ser Ile Val Arg Ala Ala Glu Val Ser Gln Glu Arg Pro Ala Lys Thr
        35                  40                  45
Thr Val Asn Ile Tyr Lys Leu Gln Ala Asp Ser Tyr Lys Ser Glu Ile
    50                  55                  60
Thr Ser Asn Gly Gly Ile Glu Asn Lys Asp Gly Glu Val Ile Ser Asn
65                  70                  75                  80
Tyr Ala Lys Leu Gly Asp Asn Val Lys Gly Leu Gln Gly Val Gln Phe
                85                  90                  95
Lys Arg Tyr Lys Val Lys Thr Asp Ile Ser Val Asp Glu Leu Lys Lys
            100                 105                 110
Leu Thr Thr Val Glu Ala Ala Asp Ala Lys Val Gly Thr Ile Leu Glu
        115                 120                 125
Glu Gly Val Ser Leu Pro Gln Lys Thr Asn Ala Gln Gly Leu Val Val
    130                 135                 140
Asp Ala Leu Asp Ser Lys Ser Asn Val Arg Tyr Leu Tyr Val Glu Asp
145                 150                 155                 160
Leu Lys Asn Ser Pro Ser Asn Ile Thr Lys Ala Tyr Ala Val Pro Phe
                165                 170                 175
Val Leu Glu Leu Pro Val Ala Asn Ser Thr Gly Thr Gly Phe Leu Ser
            180                 185                 190
Glu Ile Asn Ile Tyr Pro Lys Asn Val Val Thr Asp Glu Pro Lys Thr
        195                 200                 205
Asp Lys Asp Val Lys Lys Leu Gly Gln Asp Asp Ala Gly Tyr Thr Ile
    210                 215                 220
Gly Glu Glu Phe Lys Trp Phe Leu Lys Ser Thr Ile Pro Ala Asn Leu
225                 230                 235                 240
Gly Asp Tyr Glu Lys Phe Glu Ile Thr Asp Lys Phe Ala Asp Gly Leu
                245                 250                 255
Thr Tyr Lys Ser Val Gly Lys Ile Lys Ile Gly Ser Lys Thr Leu Asn
            260                 265                 270
Arg Asp Glu His Tyr Thr Ile Asp Glu Pro Thr Val Asp Asn Gln Asn
        275                 280                 285
Thr Leu Lys Ile Thr Phe Lys Pro Glu Lys Phe Lys Glu Ile Ala Glu
    290                 295                 300
Leu Leu Lys Gly Met Thr Leu Val Lys Asn Gln Asp Ala Leu Asp Lys
305                 310                 315                 320
Ala Thr Ala Asn Thr Asp Asp Ala Ala Phe Leu Glu Ile Pro Val Ala
                325                 330                 335
Ser Thr Ile Asn Glu Lys Ala Val Leu Gly Lys Ala Ile Glu Asn Thr
            340                 345                 350
Phe Glu Leu Gln Tyr Asp His Thr Pro Asp Lys Ala Asp Asn Pro Lys
        355                 360                 365
Pro Ser Asn Pro Pro Arg Lys Pro Glu Val His Thr Gly Gly Lys Arg
    370                 375                 380
Phe Val Lys Lys Asp Ser Thr Glu Thr Gln Thr Leu Gly Gly Ala Glu
385                 390                 395                 400
```

Phe Asp Leu Leu Ala Ser Asp Gly Thr Ala Val Lys Trp Thr Asp Ala
405 410 415
Leu Ile Lys Ala Asn Thr Asn Lys Asn Tyr Ile Ala Gly Glu Ala Val
420 425 430
Thr Gly Gln Pro Ile Lys Leu Lys Ser His Thr Asp Gly Thr Phe Glu
435 440 445
Ile Lys Gly Leu Ala Tyr Ala Val Asp Ala Asn Ala Glu Gly Thr Ala
450 455 460
Val Thr Tyr Lys Leu Lys Glu Thr Lys Ala Pro Glu Gly Tyr Val Ile
465 470 475 480
Pro Asp Lys Glu Ile Glu Phe Thr Val Ser Gln Thr Ser Tyr Asn Thr
485 490 495
Lys Pro Thr Asp Ile Thr Val Asp Ser Ala Asp Ala Thr Pro Asp Thr
500 505 510
Ile Lys Asn Asn Lys Arg Pro Ser Ile Pro Asn Thr Gly Gly Ile Gly
515 520 525
Thr Ala Ile Phe Val Ala Ile Gly Ala Ala Val Met Ala Phe Ala Val
530 535 540
Lys Gly Met Lys Arg Arg Thr Lys Asp Asn
545 550

<210> 3
<211> 517
<212> PRT
<213> Streptococcus Agalactiae

<400> 3

```
Ala Glu Val Ser Gln Glu Arg Pro Ala Lys Thr Thr Val Asn Ile Tyr
 1               5                  10                  15
Lys Leu Gln Ala Asp Ser Tyr Lys Ser Glu Ile Thr Ser Asn Gly Gly
            20                  25                  30
Ile Glu Asn Lys Asp Gly Glu Val Ile Ser Asn Tyr Ala Lys Leu Gly
        35                  40                  45
Asp Asn Val Lys Gly Leu Gln Gly Val Gln Phe Lys Arg Tyr Lys Val
    50                  55                  60
Lys Thr Asp Ile Ser Val Asp Glu Leu Lys Lys Leu Thr Thr Val Glu
65                  70                  75                  80
Ala Ala Asp Ala Lys Val Gly Thr Ile Leu Glu Glu Gly Val Ser Leu
                85                  90                  95
Pro Gln Lys Thr Asn Ala Gln Gly Leu Val Val Asp Ala Leu Asp Ser
            100                 105                 110
Lys Ser Asn Val Arg Tyr Leu Tyr Val Glu Asp Leu Lys Asn Ser Pro
        115                 120                 125
Ser Asn Ile Thr Lys Ala Tyr Ala Val Pro Phe Val Leu Glu Leu Pro
    130                 135                 140
Val Ala Asn Ser Thr Gly Thr Gly Phe Leu Ser Glu Ile Asn Ile Tyr
145                 150                 155                 160
Pro Lys Asn Val Val Thr Asp Glu Pro Lys Thr Asp Lys Asp Val Lys
                165                 170                 175
Lys Leu Gly Gln Asp Asp Ala Gly Tyr Thr Ile Gly Glu Glu Phe Lys
            180                 185                 190
Trp Phe Leu Lys Ser Thr Ile Pro Ala Asn Leu Gly Asp Tyr Glu Lys
        195                 200                 205
Phe Glu Ile Thr Asp Lys Phe Ala Asp Gly Leu Thr Tyr Lys Ser Val
    210                 215                 220
Gly Lys Ile Lys Ile Gly Ser Lys Thr Leu Asn Arg Asp Glu His Tyr
```

```
225                 230                 235                 240
Thr Ile Asp Glu Pro Thr Val Asp Asn Gln Asn Thr Leu Lys Ile Thr
                245                 250                 255
Phe Lys Pro Glu Lys Phe Lys Glu Ile Ala Glu Leu Leu Lys Gly Met
            260                 265                 270
Thr Leu Val Lys Asn Gln Asp Ala Leu Asp Lys Ala Thr Ala Asn Thr
        275                 280                 285
Asp Asp Ala Ala Phe Leu Glu Ile Pro Val Ala Ser Thr Ile Asn Glu
    290                 295                 300
Lys Ala Val Leu Gly Lys Ala Ile Glu Asn Thr Phe Glu Leu Gln Tyr
305                 310                 315                 320
Asp His Thr Pro Asp Lys Ala Asp Asn Pro Lys Pro Ser Asn Pro Pro
                325                 330                 335
Arg Lys Pro Glu Val His Thr Gly Gly Lys Arg Phe Val Lys Lys Asp
            340                 345                 350
Ser Thr Glu Thr Gln Thr Leu Gly Gly Ala Glu Phe Asp Leu Leu Ala
        355                 360                 365
Ser Asp Gly Thr Ala Val Lys Trp Thr Asp Ala Leu Ile Lys Ala Asn
    370                 375                 380
Thr Asn Lys Asn Tyr Ile Ala Gly Glu Ala Val Thr Gly Gln Pro Ile
385                 390                 395                 400
Lys Leu Lys Ser His Thr Asp Gly Thr Phe Glu Ile Lys Gly Leu Ala
                405                 410                 415
Tyr Ala Val Asp Ala Asn Ala Glu Gly Thr Ala Val Thr Tyr Lys Leu
            420                 425                 430
Lys Glu Thr Lys Ala Pro Glu Gly Tyr Val Ile Pro Asp Lys Glu Ile
        435                 440                 445
Glu Phe Thr Val Ser Gln Thr Ser Tyr Asn Thr Lys Pro Thr Asp Ile
    450                 455                 460
Thr Val Asp Ser Ala Asp Ala Thr Pro Asp Thr Ile Lys Asn Asn Lys
465                 470                 475                 480
Arg Pro Ser Ile Pro Asn Thr Gly Gly Ile Gly Thr Ala Ile Phe Val
                485                 490                 495
Ala Ile Gly Ala Ala Val Met Ala Phe Ala Val Lys Gly Met Lys Arg
            500                 505                 510
Arg Thr Lys Asp Asn
        515
```

<210> 4
<211> 525
<212> PRT
<213> Streptococcus Agalactiae

<400> 4

```
Met Lys Leu Ser Lys Lys Leu Leu Phe Ser Ala Ala Val Leu Thr Met
 1               5                  10                  15
Val Ala Gly Ser Thr Val Glu Pro Val Ala Gln Phe Ala Thr Gly Met
            20                  25                  30
Ser Ile Val Arg Ala Ala Glu Val Ser Gln Glu Arg Pro Ala Lys Thr
        35                  40                  45
Thr Val Asn Ile Tyr Lys Leu Gln Ala Asp Ser Tyr Lys Ser Glu Ile
    50                  55                  60
Thr Ser Asn Gly Gly Ile Glu Asn Lys Asp Gly Glu Val Ile Ser Asn
65                  70                  75                  80
Tyr Ala Lys Leu Gly Asp Asn Val Lys Gly Leu Gln Gly Val Gln Phe
                85                  90                  95
```

```
Lys Arg Tyr Lys Val Lys Thr Asp Ile Ser Val Asp Glu Leu Lys Lys
            100                 105                 110
Leu Thr Thr Val Glu Ala Ala Asp Ala Lys Val Gly Thr Ile Leu Glu
        115                 120                 125
Glu Gly Val Ser Leu Pro Gln Lys Thr Asn Ala Gln Gly Leu Val Val
    130                 135                 140
Asp Ala Leu Asp Ser Lys Ser Asn Val Arg Tyr Leu Tyr Val Glu Asp
145                 150                 155                 160
Leu Lys Asn Ser Pro Ser Asn Ile Thr Lys Ala Tyr Ala Val Pro Phe
                165                 170                 175
Val Leu Glu Leu Pro Val Ala Asn Ser Thr Gly Thr Gly Phe Leu Ser
            180                 185                 190
Glu Ile Asn Ile Tyr Pro Lys Asn Val Val Thr Asp Glu Pro Lys Thr
        195                 200                 205
Asp Lys Asp Val Lys Lys Leu Gly Gln Asp Asp Ala Gly Tyr Thr Ile
    210                 215                 220
Gly Glu Glu Phe Lys Trp Phe Leu Lys Ser Thr Ile Pro Ala Asn Leu
225                 230                 235                 240
Gly Asp Tyr Glu Lys Phe Glu Ile Thr Asp Lys Phe Ala Asp Gly Leu
                245                 250                 255
Thr Tyr Lys Ser Val Gly Lys Ile Lys Ile Gly Ser Lys Thr Leu Asn
            260                 265                 270
Arg Asp Glu His Tyr Thr Ile Asp Glu Pro Thr Val Asp Asn Gln Asn
        275                 280                 285
Thr Leu Lys Ile Thr Phe Lys Pro Glu Lys Phe Lys Glu Ile Ala Glu
    290                 295                 300
Leu Leu Lys Gly Met Thr Leu Val Lys Asn Gln Asp Ala Leu Asp Lys
305                 310                 315                 320
Ala Thr Ala Asn Thr Asp Asp Ala Ala Phe Leu Glu Ile Pro Val Ala
                325                 330                 335
Ser Thr Ile Asn Glu Lys Ala Val Leu Gly Lys Ala Ile Glu Asn Thr
            340                 345                 350
Phe Glu Leu Gln Tyr Asp His Thr Pro Asp Lys Ala Asp Asn Pro Lys
        355                 360                 365
Pro Ser Asn Pro Pro Arg Lys Pro Glu Val His Thr Gly Gly Lys Arg
    370                 375                 380
Phe Val Lys Lys Asp Ser Thr Glu Thr Gln Thr Leu Gly Gly Ala Glu
385                 390                 395                 400
Phe Asp Leu Leu Ala Ser Asp Gly Thr Ala Val Lys Trp Thr Asp Ala
                405                 410                 415
Leu Ile Lys Ala Asn Thr Asn Lys Asn Tyr Ile Ala Gly Glu Ala Val
            420                 425                 430
Thr Gly Gln Pro Ile Lys Leu Lys Ser His Thr Asp Gly Thr Phe Glu
        435                 440                 445
Ile Lys Gly Leu Ala Tyr Ala Val Asp Ala Asn Ala Glu Gly Thr Ala
    450                 455                 460
Val Thr Tyr Lys Leu Lys Glu Thr Lys Ala Pro Glu Gly Tyr Val Ile
465                 470                 475                 480
Pro Asp Lys Glu Ile Glu Phe Thr Val Ser Gln Thr Ser Tyr Asn Thr
                485                 490                 495
Lys Pro Thr Asp Ile Thr Val Asp Ser Ala Asp Ala Thr Pro Asp Thr
            500                 505                 510
Ile Lys Asn Asn Lys Arg Pro Ser Ile Pro Asn Thr Gly
        515                 520                 525
```

<210> 5
<211> 5

<212> PRT
<213> Streptococcus Agalactiae

<400> 5

```
Ile Pro Asn Thr Gly
1               5
```

<210> 6
<211> 520
<212> PRT
<213> Streptococcus Agalactiae

<400> 6

```
Met Lys Leu Ser Lys Lys Leu Leu Phe Ser Ala Ala Val Leu Thr Met
1               5                   10                  15
Val Ala Gly Ser Thr Val Glu Pro Val Ala Gln Phe Ala Thr Gly Met
            20                  25                  30
Ser Ile Val Arg Ala Ala Glu Val Ser Gln Glu Arg Pro Ala Lys Thr
        35                  40                  45
Thr Val Asn Ile Tyr Lys Leu Gln Ala Asp Ser Tyr Lys Ser Glu Ile
    50                  55                  60
Thr Ser Asn Gly Gly Ile Glu Asn Lys Asp Gly Glu Val Ile Ser Asn
65                  70                  75                  80
Tyr Ala Lys Leu Gly Asp Asn Val Lys Gly Leu Gln Gly Val Gln Phe
                85                  90                  95
Lys Arg Tyr Lys Val Lys Thr Asp Ile Ser Val Asp Glu Leu Lys Lys
            100                 105                 110
Leu Thr Thr Val Glu Ala Ala Asp Ala Lys Val Gly Thr Ile Leu Glu
        115                 120                 125
Glu Gly Val Ser Leu Pro Gln Lys Thr Asn Ala Gln Gly Leu Val Val
    130                 135                 140
Asp Ala Leu Asp Ser Lys Ser Asn Val Arg Tyr Leu Tyr Val Glu Asp
145                 150                 155                 160
Leu Lys Asn Ser Pro Ser Asn Ile Thr Lys Ala Tyr Ala Val Pro Phe
                165                 170                 175
Val Leu Glu Leu Pro Val Ala Asn Ser Thr Gly Thr Gly Phe Leu Ser
            180                 185                 190
Glu Ile Asn Ile Tyr Pro Lys Asn Val Val Thr Asp Glu Pro Lys Thr
        195                 200                 205
Asp Lys Asp Val Lys Lys Leu Gly Gln Asp Asp Ala Gly Tyr Thr Ile
    210                 215                 220
Gly Glu Glu Phe Lys Trp Phe Leu Lys Ser Thr Ile Pro Ala Asn Leu
225                 230                 235                 240
Gly Asp Tyr Glu Lys Phe Glu Ile Thr Asp Lys Phe Ala Asp Gly Leu
                245                 250                 255
Thr Tyr Lys Ser Val Gly Lys Ile Lys Ile Gly Ser Lys Thr Leu Asn
            260                 265                 270
Arg Asp Glu His Tyr Thr Ile Asp Glu Pro Thr Val Asp Asn Gln Asn
        275                 280                 285
Thr Leu Lys Ile Thr Phe Lys Pro Glu Lys Phe Lys Glu Ile Ala Glu
    290                 295                 300
Leu Leu Lys Gly Met Thr Leu Val Lys Asn Gln Asp Ala Leu Asp Lys
305                 310                 315                 320
Ala Thr Ala Asn Thr Asp Asp Ala Ala Phe Leu Glu Ile Pro Val Ala
                325                 330                 335
```

```
Ser Thr Ile Asn Glu Lys Ala Val Leu Gly Lys Ala Ile Glu Asn Thr
            340                 345                 350
Phe Glu Leu Gln Tyr Asp His Thr Pro Asp Lys Ala Asp Asn Pro Lys
        355                 360                 365
Pro Ser Asn Pro Pro Arg Lys Pro Glu Val His Thr Gly Gly Lys Arg
    370                 375                 380
Phe Val Lys Lys Asp Ser Thr Glu Thr Gln Thr Leu Gly Gly Ala Glu
385                 390                 395                 400
Phe Asp Leu Leu Ala Ser Asp Gly Thr Ala Val Lys Trp Thr Asp Ala
                405                 410                 415
Leu Ile Lys Ala Asn Thr Asn Lys Asn Tyr Ile Ala Gly Glu Ala Val
            420                 425                 430
Thr Gly Gln Pro Ile Lys Leu Lys Ser His Thr Asp Gly Thr Phe Glu
        435                 440                 445
Ile Lys Gly Leu Ala Tyr Ala Val Asp Ala Asn Ala Glu Gly Thr Ala
    450                 455                 460
Val Thr Tyr Lys Leu Lys Glu Thr Lys Ala Pro Glu Gly Tyr Val Ile
465                 470                 475                 480
Pro Asp Lys Glu Ile Glu Phe Thr Val Ser Gln Thr Ser Tyr Asn Thr
                485                 490                 495
Lys Pro Thr Asp Ile Thr Val Asp Ser Ala Asp Ala Thr Pro Asp Thr
            500                 505                 510
Ile Lys Asn Asn Lys Arg Pro Ser
        515                 520
```

<210> 7
<211> 483
<212> PRT
<213>-Streptococcus Agalactiae

<400> 7

```
Ala Glu Val Ser Gln Glu Arg Pro Ala Lys Thr Thr Val Asn Ile Tyr
 1               5                  10                  15
Lys Leu Gln Ala Asp Ser Tyr Lys Ser Glu Ile Thr Ser Asn Gly Gly
            20                  25                  30
Ile Glu Asn Lys Asp Gly Glu Val Ile Ser Asn Tyr Ala Lys Leu Gly
        35                  40                  45
Asp Asn Val Lys Gly Leu Gln Gly Val Gln Phe Lys Arg Tyr Lys Val
    50                  55                  60
Lys Thr Asp Ile Ser Val Asp Glu Leu Lys Lys Leu Thr Thr Val Glu
65                  70                  75                  80
Ala Ala Asp Ala Lys Val Gly Thr Ile Leu Glu Glu Gly Val Ser Leu
                85                  90                  95
Pro Gln Lys Thr Asn Ala Gln Gly Leu Val Val Asp Ala Leu Asp Ser
            100                 105                 110
Lys Ser Asn Val Arg Tyr Leu Tyr Val Glu Asp Leu Lys Asn Ser Pro
        115                 120                 125
Ser Asn Ile Thr Lys Ala Tyr Ala Val Pro Phe Val Leu Glu Leu Pro
    130                 135                 140
Val Ala Asn Ser Thr Gly Thr Gly Phe Leu Ser Glu Ile Asn Ile Tyr
145                 150                 155                 160
Pro Lys Asn Val Val Thr Asp Glu Pro Lys Thr Asp Lys Asp Val Lys
                165                 170                 175
Lys Leu Gly Gln Asp Asp Ala Gly Tyr Thr Ile Gly Glu Glu Phe Lys
            180                 185                 190
Trp Phe Leu Lys Ser Thr Ile Pro Ala Asn Leu Gly Asp Tyr Glu Lys
```

```
            195                 200                 205
Phe Glu Ile Thr Asp Lys Phe Ala Asp Gly Leu Thr Tyr Lys Ser Val
        210                 215                 220
Gly Lys Ile Lys Ile Gly Ser Lys Thr Leu Asn Arg Asp Glu His Tyr
225                 230                 235                 240
Thr Ile Asp Glu Pro Thr Val Asp Asn Gln Asn Thr Leu Lys Ile Thr
                245                 250                 255
Phe Lys Pro Glu Lys Phe Lys Glu Ile Ala Glu Leu Leu Lys Gly Met
            260                 265                 270
Thr Leu Val Lys Asn Gln Asp Ala Leu Asp Lys Ala Thr Ala Asn Thr
        275                 280                 285
Asp Asp Ala Ala Phe Leu Glu Ile Pro Val Ala Ser Thr Ile Asn Glu
    290                 295                 300
Lys Ala Val Leu Gly Lys Ala Ile Glu Asn Thr Phe Glu Leu Gln Tyr
305                 310                 315                 320
Asp His Thr Pro Asp Lys Ala Asp Asn Pro Lys Pro Ser Asn Pro Pro
                325                 330                 335
Arg Lys Pro Glu Val His Thr Gly Gly Lys Arg Phe Val Lys Lys Asp
            340                 345                 350
Ser Thr Glu Thr Gln Thr Leu Gly Gly Ala Glu Phe Asp Leu Leu Ala
        355                 360                 365
Ser Asp Gly Thr Ala Val Lys Trp Thr Asp Ala Leu Ile Lys Ala Asn
    370                 375                 380
Thr Asn Lys Asn Tyr Ile Ala Gly Glu Ala Val Thr Gly Gln Pro Ile
385                 390                 395                 400
Lys Leu Lys Ser His Thr Asp Gly Thr Phe Glu Ile Lys Gly Leu Ala
                405                 410                 415
Tyr Ala Val Asp Ala Asn Ala Glu Gly Thr Ala Val Thr Tyr Lys Leu
            420                 425                 430
Lys Glu Thr Lys Ala Pro Glu Gly Tyr Val Ile Pro Asp Lys Glu Ile
        435                 440                 445
Glu Phe Thr Val Ser Gln Thr Ser Tyr Asn Thr Lys Pro Thr Asp Ile
    450                 455                 460
Thr Val Asp Ser Ala Asp Ala Thr Pro Asp Thr Ile Lys Asn Asn Lys
465                 470                 475                 480
Arg Pro Ser
```

<210> 8
<211> 271
<212> PRT
<213> Streptococcus Agalactiae

<400> 8

Ala Glu Val Ser Gln Glu Arg Pro Ala Lys Thr Thr Val Asn Ile Tyr
1               5                   10                  15
Lys Leu Gln Ala Asp Ser Tyr Lys Ser Glu Ile Thr Ser Asn Gly Gly
            20                  25                  30
Ile Glu Asn Lys Asp Gly Glu Val Ile Ser Asn Tyr Ala Lys Leu Gly
        35                  40                  45
Asp Asn Val Lys Gly Leu Gln Gly Val Gln Phe Lys Arg Tyr Lys Val
    50                  55                  60
Lys Thr Asp Ile Ser Val Asp Glu Leu Lys Lys Leu Thr Thr Val Glu
65                  70                  75                  80
Ala Ala Asp Ala Lys Val Gly Thr Ile Leu Glu Glu Gly Val Ser Leu
                85                  90                  95

Pro Gln Lys Thr Asn Ala Gln Gly Leu Val Val Asp Ala Leu Asp Ser
            100                 105                 110
Lys Ser Asn Val Arg Tyr Leu Tyr Val Glu Asp Leu Lys Asn Ser Pro
        115                 120                 125
Ser Asn Ile Thr Lys Ala Tyr Ala Val Pro Phe Val Leu Glu Leu Pro
    130                 135                 140
Val Ala Asn Ser Thr Gly Thr Gly Phe Leu Ser Glu Ile Asn Ile Tyr
145                 150                 155                 160
Pro Lys Asn Val Val Thr Asp Glu Pro Lys Thr Asp Lys Asp Val Lys
                165                 170                 175
Lys Leu Gly Gln Asp Asp Ala Gly Tyr Thr Ile Gly Glu Glu Phe Lys
            180                 185                 190
Trp Phe Leu Lys Ser Thr Ile Pro Ala Asn Leu Gly Asp Tyr Glu Lys
        195                 200                 205
Phe Glu Ile Thr Asp Lys Phe Ala Asp Gly Leu Thr Tyr Lys Ser Val
    210                 215                 220
Gly Lys Ile Lys Ile Gly Ser Lys Thr Leu Asn Arg Asp Glu His Tyr
225                 230                 235                 240
Thr Ile Asp Glu Pro Thr Val Asp Asn Gln Asn Thr Leu Lys Ile Thr
                245                 250                 255
Phe Lys Pro Glu Lys Phe Lys Glu Ile Ala Glu Leu Leu Lys Gly
            260                 265                 270

<210> 9
<211> 2118
<212> DNA
<213> Streptococcus Agalactiae

<400> 9

```
ttaagcttcc tttgattggc gtcttttcat gataactact gctccaagca taatgcttaa 60
accaataatt gtgaaaagaa ttgtaccaat accacctgtt tgtgggattg ttaccttttt 120
atttctaca cgtgtcgcat ctttttggtt gctgttagca acgtagtcaa tgttaccacc 180
tgttatgtat gacccttgat taactacaaa cttaatatta cctgccaact tagcaaatcc 240
tgctggagca agtgtttctt caaggttgta agtaccgtct gcaagacctg taacttcaaa 300
ttgaccttga tcgtttgaag tgtaggtaat ggctctagcc ttatctgtta tccactcata 360
agctgtacga gcctcaatga aggctgcatc gtaatctgct tgtttagttt tgataagttc 420
ttttgcagta attccttttt caccttttg gtctgttgca gacaacttgt tataagcagc 480
gatagcttca tctaaagcta ttttcttagc agctaaagtt ttttgacctt ctgattgatc 540
tgctttaaga gcaaggtatt tacctgctga gtttttcaca acgaattgtg caccagccaa 600
acggtcacct tgttcattag ttttgacaaa tttcttacca tgagtttcaa cttttggttc 660
agttgggttc aatggtgttg ggttatcaga atctttggta ttggtaatgg ttactttacc 720
atttctaga tttattgcac ttccgtaacc agaaacacgt tctgagatca tgtatgattt 780
gttttctaga ccagtgaatt tacccgagaa gttaccagat acttcaaatt tgataccatt 840
tccaaggtcg attgtacctt tagatgtttt tgtcaatgat actgaagcaa cagttttatc 900
tttatctttc aatgtgtaaa caacgtttac accatcaggt gcaattccgt cagaccaagt 960
tttagcaact gttacttcac cctttgaagg tgtaacagga agttcagtca agtctttacc 1020
tggtttgtta ccatacgaca atttgatatc attggattct ggattatcaa taattgcttg 1080
accattaaca gtagcactat aagtcaatgt aaattcaata tcagctgttt tagctgcttt 1140
ttccaatttg cccaatccat cagctgtgaa ttttaatgtg aaaccacggg catcaatgct 1200
aagttcatag tctgtatcct tagcaaaagt ttctgtagtt cctgaagctt taaggctaac 1260
agttgaaccc attgtcaaac catttgacat tatatctgtc caaaccaagt tttcgtattt 1320
agaacctttg tgaatttttg ttttaacttc ataaggaaca actttaccga tttcagcagt 1380
agcagttgct ttgtcacgtg cataattacc ataatttgcg ccagctgtca aaagtctatt 1440
aacatctgtc aatgctgtca aatcgtttgt tttagcaaag tttttatcaa tttctggttt 1500
ttcttcagtg ttctttggat aaacatgggc atcagcaaca acaccatctt catttaccaa 1560
tggaagagtg atgttaactg gaaccgcttt tgaagcagcc aggagggaac cattattgtt 1620

gtaagtagat tttgatttaa cttcaacaat tttaaactcg cctttcaatc ctttggtgtt 1680
gaaaacaagt ccagtatctc cctctggtgt caatccagac acggcctcat caatatttac 1740
tgttatttca ggagtaccat ctttattaat taaggctggt gttaatttgt taccttcttt 1800
tgccttaaca tattgcactt taccactttt atcttctttc aaagctaaag caaagaacgc 1860
accttcgatt tctttagatc cctcgccaaa gtaaccagca aggtcagaaa tagctccacc 1920
tttgtagtct tttccgttaa gacctgtagt tcctgggaag ttacttttgt taagatttga 1980
ttcggtttgc aaaatcttgt gcaaagtcac tgtattagtt gttgcttcat ccgcaaacgc 2040
tggtgcaact gagagcaatg acgttaaagt cagtaacaat gccgagaaca ttgcaaaata 2100
tttgttgatt cttttcat                                               2118
```

<210> 10
<211> 705
<212> PRT
<213> Streptococcus Agalactiae

<400> 10

Met Lys Arg Ile Asn Lys Tyr Phe Ala Met Phe Ser Ala Leu Leu Leu
1 5 10 15
Thr Leu Thr Ser Leu Leu Ser Val Ala Pro Ala Phe Ala Asp Glu Ala
20 25 30
Thr Thr Asn Thr Val Thr Leu His Lys Ile Leu Gln Thr Glu Ser Asn
35 40 45
Leu Asn Lys Ser Asn Phe Pro Gly Thr Thr Gly Leu Asn Gly Lys Asp
50 55 60
Tyr Lys Gly Gly Ala Ile Ser Asp Leu Ala Gly Tyr Phe Gly Glu Gly
65 70 75 80
Ser Lys Glu Ile Glu Gly Ala Phe Phe Ala Leu Ala Leu Lys Glu Asp
85 90 95
Lys Ser Gly Lys Val Gln Tyr Val Lys Ala Lys Glu Gly Asn Lys Leu
100 105 110
Thr Pro Ala Leu Ile Asn Lys Asp Gly Thr Pro Glu Ile Thr Val Asn
115 120 125
Ile Asp Glu Ala Val Ser Gly Leu Thr Pro Glu Gly Asp Thr Gly Leu
130 135 140
Val Phe Asn Thr Lys Gly Leu Lys Gly Glu Phe Lys Ile Val Glu Val
145 150 155 160
Lys Ser Lys Ser Thr Tyr Asn Asn Asn Gly Ser Leu Leu Ala Ala Ser
165 170 175
Lys Ala Val Pro Val Asn Ile Thr Leu Pro Leu Val Asn Glu Asp Gly
180 185 190
Val Val Ala Asp Ala His Val Tyr Pro Lys Asn Thr Glu Glu Lys Pro
195 200 205
Glu Ile Asp Lys Asn Phe Ala Lys Thr Asn Asp Leu Thr Ala Leu Thr
210 215 220
Asp Val Asn Arg Leu Leu Thr Ala Gly Ala Asn Tyr Gly Asn Tyr Ala
225 230 235 240
Arg Asp Lys Ala Thr Ala Thr Ala Glu Ile Gly Lys Val Val Pro Tyr
245 250 255
Glu Val Lys Thr Lys Ile His Lys Gly Ser Lys Tyr Glu Asn Leu Val
260 265 270
Trp Thr Asp Ile Met Ser Asn Gly Leu Thr Met Gly Ser Thr Val Ser
275 280 285
Leu Lys Ala Ser Gly Thr Thr Glu Thr Phe Ala Lys Asp Thr Asp Tyr
290 295 300
Glu Leu Ser Ile Asp Ala Arg Gly Phe Thr Leu Lys Phe Thr Ala Asp
305 310 315 320
Gly Leu Gly Lys Leu Glu Lys Ala Ala Lys Thr Ala Asp Ile Glu Phe

```
                        325                         330                         335
Thr Leu Thr Tyr Ser Ala Thr Val Asn Gly Gln Ala Ile Ile Asp Asn
                340                         345                         350
Pro Glu Ser Asn Asp Ile Lys Leu Ser Tyr Gly Asn Lys Pro Gly Lys
        355                         360                         365
Asp Leu Thr Glu Leu Pro Val Thr Pro Ser Lys Gly Glu Val Thr Val
    370                         375                         380
Ala Lys Thr Trp Ser Asp Gly Ile Ala Pro Asp Gly Val Asn Val Val
385                         390                         395                         400
Tyr Thr Leu Lys Asp Lys Asp Lys Thr Val Ala Ser Val Ser Leu Thr
                        405                         410                         415
Lys Thr Ser Lys Gly Thr Ile Asp Leu Gly Asn Gly Ile Lys Phe Glu
                420                         425                         430
Val Ser Gly Asn Phe Ser Gly Lys Phe Thr Gly Leu Glu Asn Lys Ser
        435                         440                         445
Tyr Met Ile Ser Glu Arg Val Ser Gly Tyr Gly Ser Ala Ile Asn Leu
    450                         455                         460
Glu Asn Gly Lys Val Thr Ile Thr Asn Thr Lys Asp Ser Asp Asn Pro
465                         470                         475                         480
Thr Pro Leu Asn Pro Thr Glu Pro Lys Val Glu Thr His Gly Lys Lys
                        485                         490                         495
Phe Val Lys Thr Asn Glu Gln Gly Asp Arg Leu Ala Gly Ala Gln Phe
                500                         505                         510
Val Val Lys Asn Ser Ala Gly Lys Tyr Leu Ala Leu Lys Ala Asp Gln
        515                         520                         525
Ser Glu Gly Gln Lys Thr Leu Ala Ala Lys Lys Ile Ala Leu Asp Glu
    530                         535                         540
Ala Ile Ala Ala Tyr Asn Lys Leu Ser Ala Thr Asp Gln Lys Gly Glu
545                         550                         555                         560
Lys Gly Ile Thr Ala Lys Glu Leu Ile Lys Thr Lys Gln Ala Asp Tyr
                        565                         570                         575
Asp Ala Ala Phe Ile Glu Ala Arg Thr Ala Tyr Glu Trp Ile Thr Asp
                580                         585                         590
Lys Ala Arg Ala Ile Thr Tyr Thr Ser Asn Asp Gln Gly Gln Phe Glu
        595                         600                         605
Val Thr Gly Leu Ala Asp Gly Thr Tyr Asn Leu Glu Glu Thr Leu Ala
    610                         615                         620
Pro Ala Gly Phe Ala Lys Leu Ala Gly Asn Ile Lys Phe Val Val Asn
625                         630                         635                         640
Gln Gly Ser Tyr Ile Thr Gly Gly Asn Ile Asp Tyr Val Ala Asn Ser
                        645                         650                         655
Asn Gln Lys Asp Ala Thr Arg Val Glu Asn Lys Lys Val Thr Ile Pro
                660                         665                         670
Gln Thr Gly Gly Ile Gly Thr Ile Leu Phe Thr Ile Ile Gly Leu Ser
        675                         680                         685
Ile Met Leu Gly Ala Val Val Ile Met Lys Arg Arg Gln Ser Lys Glu
    690                         695                         700
Ala
705
```

<210> 11
<211> 2025
<212> DNA
<213> Streptococcus Agalactiae

<400> 11

```
atgaaaaaaa tcaacaaatg tcttacaatg ttctcgacac tgctattgat cttaacgtca 60
ctattctcag ttgcaccagc gtttgcggac gacgcaacaa ctgatactgt gaccttgcac 120
aagattgtca tgccacaagc tgcatttgat aactttactg aaggtacaaa aggtaagaat 180
gatagcgatt atgttggtaa acaaattaat gaccttaaat cttattttgg ctcaaccgat 240
gctaaagaaa tcaagggtgc tttctttgtt ttcaaaaatg aaactggtac aaaattcatt 300
actgaaaatg gtaaggaagt cgatactttg gaagctaaag atgctgaagg tggtgctgtt 360
ctttcagggt taacaaaaga caatggtttt gtttttaaca ctgctaagtt aaaaggaatt 420
taccaaatcg ttgaattgaa agaaaaatca aactacgata acaacggttc tatcttggct 480
gattcaaaag cagttccagt taaaatcact ctgccattgg taaacaacca aggtgttgtt 540
aaagatgctc acatttatcc aaagaatact gaaacaaaac cacaagtaga taagaacttt 600
gcagataaag atcttgatta tactgacaac cgaaaagaca aaggtgttgt ctcagcgaca 660
gttggtgaca aaaaagaata catagttgga acaaaaattc ttaaaggctc agactataag 720
aaactggttt ggactgatag catgactaaa ggtttgacgt tcaacaacaa cgttaaagta 780
acattggatg gtgaagattt tcctgtttta aactacaaac tcgtaacaga tgaccaaggt 840
ttccgtcttg ccttgaatgc aacaggtctt gcagcagtag cagcagctgc aaaagacaaa 900
gatgttgaaa tcaagatcac ttactcagct acggtgaacg gctccactac tgttgaaatt 960
ccagaaacca atgatgttaa attggactat ggtaataacc caacggaaga aagtgaacca 1020
caagaaggta ctccagctaa ccaagaaatt aaagtcatta aagactgggc agtagatggt 1080
acaattactg atgctaatgt tgcagttaaa gctatcttta ccttgcaaga aaaacaaacg 1140
gatggtacat gggtgaacgt tgcttcacac gaagcaacaa aaccatcacg ctttgaacat 1200
actttcacag gtttggataa tgctaaaact taccgcgttg tcgaacgtgt tagcggctac 1260
actccagaat acgtatcatt taaaaatggt gttgtgacta tcaagaacaa caaaaactca 1320
aatgatccaa ctccaatcaa cccatcagaa ccaaaagtgg tgacttatgg acgtaaattt 1380
gtgaaaacaa atcaagctaa cactgaacgc ttggcaggag ctaccttcct cgttaagaaa 1440
gaaggcaaat acttggcacg taaagcaggt gcagcaactg ctgaagcaaa ggcagctgta 1500
aaaactgcta aactagcatt ggatgaagct gttaaagctt ataacgactt gactaaagaa 1560
aaacaagaag gccaagaagg taaaacagca ttggctactg ttgatcaaaa acaaaaagct 1620
tacaatgacg cttttgttaa agctaactac tcatatgaat gggttgcaga taaaaaggct 1680
gataatgttg ttaaattgat ctctaacgcc ggtggtcaat ttgaaattac tggtttggat 1740
aaaggcactt atggcttgga agaaactcaa gcaccagcag gttatgcgac attgtcaggt 1800
gatgtaaact ttgaagtaac tgccacatca tatagcaaag gggctacaac tgacatcgca 1860
tatgataaag gctctgtaaa aaaagatgcc caacaagttc aaaacaaaaa agtaaccatc 1920
ccacaaacag gtggtattgg tacaattctt ttcacaatta ttggtttaag cattatgctt 1980
ggagcagtag ttatcatgaa aaaacgtcaa tcagaggaag cttaa                 2025
```

<210> 12
<211> 674
<212> PRT
<213> Streptococcus Agalactiae

<400> 12

```
Met Lys Lys Ile Asn Lys Cys Leu Thr Met Phe Ser Thr Leu Leu Leu
 1               5                  10                  15
Ile Leu Thr Ser Leu Phe Ser Val Ala Pro Ala Phe Ala Asp Asp Ala
            20                  25                  30
Thr Thr Asp Thr Val Thr Leu His Lys Ile Val Met Pro Gln Ala Ala
        35                  40                  45
Phe Asp Asn Phe Thr Glu Gly Thr Lys Gly Lys Asn Asp Ser Asp Tyr
    50                  55                  60
Val Gly Lys Gln Ile Asn Asp Leu Lys Ser Tyr Phe Gly Ser Thr Asp
65                  70                  75                  80
Ala Lys Glu Ile Lys Gly Ala Phe Phe Val Phe Lys Asn Glu Thr Gly
                85                  90                  95
Thr Lys Phe Ile Thr Glu Asn Gly Lys Glu Val Asp Thr Leu Glu Ala
            100                 105                 110
Lys Asp Ala Glu Gly Gly Ala Val Leu Ser Gly Leu Thr Lys Asp Asn
        115                 120                 125
```

Gly Phe Val Phe Asn Thr Ala Lys Leu Lys Gly Ile Tyr Gln Ile Val
    130                 135                 140
Glu Leu Lys Glu Lys Ser Asn Tyr Asp Asn Asn Gly Ser Ile Leu Ala
145                 150                 155                 160
Asp Ser Lys Ala Val Pro Val Lys Ile Thr Leu Pro Leu Val Asn Asn
                165                 170                 175
Gln Gly Val Val Lys Asp Ala His Ile Tyr Pro Lys Asn Thr Glu Thr
            180                 185                 190
Lys Pro Gln Val Asp Lys Asn Phe Ala Asp Lys Asp Leu Asp Tyr Thr
        195                 200                 205
Asp Asn Arg Lys Asp Lys Gly Val Val Ser Ala Thr Val Gly Asp Lys
    210                 215                 220
Lys Glu Tyr Ile Val Gly Thr Lys Ile Leu Lys Gly Ser Asp Tyr Lys
225                 230                 235                 240
Lys Leu Val Trp Thr Asp Ser Met Thr Lys Gly Leu Thr Phe Asn Asn
                245                 250                 255
Asn Val Lys Val Thr Leu Asp Gly Glu Asp Phe Pro Val Leu Asn Tyr
            260                 265                 270
Lys Leu Val Thr Asp Asp Gln Gly Phe Arg Leu Ala Leu Asn Ala Thr
        275                 280                 285
Gly Leu Ala Ala Val Ala Ala Ala Ala Lys Asp Lys Asp Val Glu Ile
    290                 295                 300
Lys Ile Thr Tyr Ser Ala Thr Val Asn Gly Ser Thr Thr Val Glu Ile
305                 310                 315                 320
Pro Glu Thr Asn Asp Val Lys Leu Asp Tyr Gly Asn Asn Pro Thr Glu
                325                 330                 335
Glu Ser Glu Pro Gln Glu Gly Thr Pro Ala Asn Gln Glu Ile Lys Val
            340                 345                 350
Ile Lys Asp Trp Ala Val Asp Gly Thr Ile Thr Asp Ala Asn Val Ala
        355                 360                 365
Val Lys Ala Ile Phe Thr Leu Gln Glu Lys Gln Thr Asp Gly Thr Trp
    370                 375                 380
Val Asn Val Ala Ser His Glu Ala Thr Lys Pro Ser Arg Phe Glu His
385                 390                 395                 400
Thr Phe Thr Gly Leu Asp Asn Ala Lys Thr Tyr Arg Val Val Glu Arg
                405                 410                 415
Val Ser Gly Tyr Thr Pro Glu Tyr Val Ser Phe Lys Asn Gly Val Val
            420                 425                 430
Thr Ile Lys Asn Asn Lys Asn Ser Asn Asp Pro Thr Pro Ile Asn Pro
        435                 440                 445
Ser Glu Pro Lys Val Val Thr Tyr Gly Arg Lys Phe Val Lys Thr Asn
    450                 455                 460
Gln Ala Asn Thr Glu Arg Leu Ala Gly Ala Thr Phe Leu Val Lys Lys
465                 470                 475                 480
Glu Gly Lys Tyr Leu Ala Arg Lys Ala Gly Ala Ala Thr Ala Glu Ala
                485                 490                 495
Lys Ala Ala Val Lys Thr Ala Lys Leu Ala Leu Asp Glu Ala Val Lys
            500                 505                 510
Ala Tyr Asn Asp Leu Thr Lys Glu Lys Gln Glu Gly Gln Glu Gly Lys
        515                 520                 525
Thr Ala Leu Ala Thr Val Asp Gln Lys Gln Lys Ala Tyr Asn Asp Ala
    530                 535                 540
Phe Val Lys Ala Asn Tyr Ser Tyr Glu Trp Val Ala Asp Lys Lys Ala
545                 550                 555                 560
Asp Asn Val Val Lys Leu Ile Ser Asn Ala Gly Gly Gln Phe Glu Ile
                565                 570                 575
Thr Gly Leu Asp Lys Gly Thr Tyr Gly Leu Glu Glu Thr Gln Ala Pro

```
                580                           585                           590
Ala Gly Tyr Ala Thr Leu Ser Gly Asp Val Asn Phe Glu Val Thr Ala
            595                           600                           605
Thr Ser Tyr Ser Lys Gly Ala Thr Thr Asp Ile Ala Tyr Asp Lys Gly
    610                           615                           620
Ser Val Lys Lys Asp Ala Gln Gln Val Gln Asn Lys Lys Val Thr Ile
625                           630                           635                           640
Pro Gln Thr Gly Gly Ile Gly Thr Ile Leu Phe Thr Ile Ile Gly Leu
                    645                           650                           655
Ser Ile Met Leu Gly Ala Val Val Ile Met Lys Lys Arg Gln Ser Glu
                660                           665                           670
Glu Ala
```

<210> 13
<211> 5
<212> PRT
<213> Streptococcus Agalactiae

<400> 13

```
Ile Pro Gln Thr Gly
 1               5
```

## Description

## FIELD OF INVENTION

The invention relates to isolated or purified bacterial adhesin conformers, preferably with improved stability and/or immunogenicity. In a preferred aspect, the invention comprises an isolated bacterial adhesin conformer F, wherein the isolated bacterial adhesin is GB580.

## BACKGROUND OF THE INVENTION

Proteins are biological polymers which fold into complex three-dimensional structures. The classical hierarchy of structure of proteins has four levels including: (i) the primary structure - the sequence of amino acids that make up the protein, (ii) secondary structure - the local three-dimensional structure of the peptide backbone that can include alpha helices, beta sheets, $3_{10}$ helices and pi helices, (iii) tertiary structure - the global three-dimensional structure of the entire protein or protein sub-unit (i.e., all the atoms), and (iv) the quaternary structure - the three-dimensional relationship of subunits or proteins in a protein complex. Each protein can exist in multiple conformations (or "conformers") depending upon the local conditions and multiple conformers can co-exist in equilibrium. The simplest example of protein conformers are folded and unfolded conformations of a protein. Many proteins have multiple folded conformations. For example, alpha- and beta-tubulin are subunits that can polymerize to form microtubules. Such polymerization changes the quaternary structure of tubulin and therefore represents an alternate conformer of tubulin. In addition, certain proteins have conformations with different secondary structures such as certain amyloid proteins which convert from soluble proteins with predominantly alpha helical secondary structure to long, insoluble fibrils with predominantly beta sheet secondary structure.

When purifying proteins from a host organism, it is often difficult to separate different conformers of a protein given that the physical properties of the different conformers are very similar. When dealing with heterologous protein expression, this can be exacerbated by the fact that the heterologous organism may lack necessary chaperonins that assist folding, enzymes that post-translationally modify the protein, and other co-factors that assist with the interconversion between conformers, such as kinases that add phosphate groups to the protein to switch from an inactive to an active form or vice versa. Potential troubles include protein misfolding, instability, insolubility (formation of so-called inclusion bodies), and inability to generate certain conformers without co-expression of co-factors. Even when the heterologous organism can express the conformer of interest, other conformers may also be expressed that are difficult to separate from the conformer of interest given the similar biophysical properties.

Protein conformation is particularly relevant to production of therapeutic proteins and vaccine component proteins. In high-throughput early discovery and high-yield production of candidate therapeutic proteins or recombinant vaccine candidates, E. coli-based expression systems are now widely used. The major advantages of these systems are speed, simplicity, and low cost of the recombinant protein production plus extensive knowledge of basic cellular processes of this host. The latter allows easy manipulation of protein expression and provides the means for operative interference to improve the yield and quality of proteins to be expressed. Yet, despite the general, similarity of protein biosyntheses for all living species, *E. coli* is not a universal host that can produce large amounts of every protein derived from other species because of differences between translational and/or post-translational machineries that can affect the protein conformations produced as discussed above.

The difficulties in expression and purification of proteins in desired conformations are particularly important for proteins that are to be used as vaccine components. The antigenicity of a protein does not necessarily depend upon the three-dimensional structure of a protein such as when antigenic portions of a protein are found in a loop region which does not depend upon the overall three dimensional structure or when the relevant antigenicity lies in presentation of peptide fragments by MHC molecules. However in some instances, the antigenic properties of an antigen depend upon the three-dimensional shape which may be found in only one or a limited number of the conformations of the protein. Therefore, to maximize the antigenicity of such protein vaccine components, it is therefore necessary to identify the conformation or conformations that are the most antigenic and determine protocols for purification or isolation of preparation of the protein that are enriched in the desired conformation or conformations.

For vaccine development a particularly preferred class of antigens is represented by the adhesin class. Adhesins are a group of surface-exposed antigens that are involved in host tissues adhesion and colonization.

Applicants have previously identified adhesin island loci within the genome of Streptococcus agalactiae ("GBS"). The polypeptides encoded by these loci are useful in compositions for the treatment or prevention of GBS infection. Similar sequences have been identified in other Gram positive bacteria and can be used in immunogenic compositions for the treatment or prevention of infection of Gram positive bacteria. The identified adhesin island surface protein are usually assembled into high-molecular weight polymeric structures such as pili. GBS 80, one of the adhesin identified in GBS, demonstrated to be highly protective in immunological studies.

GBS has emerged in the last 20 years as the major cause of neonatal sepsis and meningitis. that affect 0.5-3 per 1000 live births, and an important cause of morbidity among the older age group affecting 5-8 per 100,000 of the population. Current disease management strategies rely on intrapartum antibiotics and neonatal monitoring which have reduced neonatal case mortality from >50% in the 1970's to less than 10% in the 1990's. Nevertheless, there is still considerable morbidity and mortality and the management is expensive. 15-35% of pregnant women are asymptomatic carriers and at high risk of transmitting the disease to their babies. Risk of neonatal infection is associated with low serotype specific maternal antibodies and high titers are believed to be protective. In addition, invasive GBS disease is increasingly recognized in elderly adults with underlying disease such as diabetes and cancer.

The "B" in "GBS" refers to the Lancefield classification, which is based on the antigenicity of a carbohydrate which is soluble in dilute acid and called the C carbohydrate. Lancefield identified 13 types of C carbohydrate, designated A to O, that could be serologically differentiated; the organisms that most commonly infect humans are found in groups A, B, D, and G. Within group B, strains can be divided into at least 9 serotypes (Ia, Ib, Ia/c, II, III, IV, V, VI, VII and VIII) based on the structure of their polysaccharide capsule. In the past, serotypes Ia, Ib, II, and III were equally prevalent in normal vaginal carriage and early onset sepsis in newborns. Type V GBS has emerged as an important cause of GBS infection in the USA, however, and strains of types VI and VIII have become prevalent among Japanese women.

The genome sequence of a serotype V strain 2603 V/R has been published (Tettelin et al. (2002) Proc. Natl. Acad Sci. USA, 10.1 073/pnas. 182380799) and various polypeptides for use a vaccine antigens have been identified (WO02/34771). The vaccines currently in clinical trials, however, are based on polysaccharide antigens. These suffer from serotype specificity and poor immunogenicity, and so there is a need for effective vaccines against S. agalactiae infection. WO 05/028618 describes vaccine compositions comprising combinations of GBS antigens which include GBS80.

It is an object of the invention to provide further and improved compositions for providing immunity against disease and/or infection of pathogenic bacteria, including *S. agalactiae.* In one aspect of the invention, the compositions are based on the isolation of adhesin conformers that posses improved stability, conformation and immunogenicity, and their use in therapeutic or prophylactic compositions.

## SUMMARY OF THE INVENTION

The present application both identifies a problem - that bacterial adhesins are expressed in multiple conformations which have differing antigenicities - and provides the solution by providing methods for isolation and inter-conversion of the conformers. In a broad form therefore an aspect of the invention may be said to reside in GBS80 isoforms preferably having improved stability and/or immunogenicity.

Said preferred isoform, herein referred to as "conformer F", has distinguishable structural properties and can be isolated from other associated isoforms through different chromatographic techniques. GBS 80, a representative member of the adhesin family from *S. agalactiae,* can be separated in either one of two isoforms through anion exchange chromatography. Specifically, the more stable conformer F is purified from the less stable isoform "conformer A" through its inability to be retained by a Q-Sepharose ion exchange column while is retained by hydroxyapatite. In one embodiment a purified GBS 80 conformer F is characterized in that it is easily separated as single band in a non-denaturing sodium dodecyl sulfate-polyacrylamide gel electrophoresis (SDS-PAGE). Yet in a further embodiment, an isolated GBS 80 conformer F is eluted as a single monodisperse peak by Size Exclusion Chromatography (SEC). In another embodiment, an isolated GBS 80 conformer F shows increased resistance to protease digestion over conformer A.

One aspect of the present invention provides isolated and/or separated GBS80 comprising conformer F or conformer A where preferably the GBS80 conformer is capable of generating an immune response in a subject. In preferred embodiments; the bacterial adhesin will be a homolog of GBS 80, or more preferably an ortholog or a paralog. Preferably the homology of such homolog, ortholog or paralog will be at least about 60% identity, at least about 70% identity, at least about 80% identity, at least about 85% identity, at least about 90% identity, at least about 92.5% identity, at least about 95% identity, at least about 96% identity, at least about 97% identify, at least about 98% identity, or at least about 99% identity. In various embodiments, the GBS80 may be produced recombinantly, preferably by bacterial expression such as *E. coli* expression. In some embodiments, the GBS80 in conformer F may have one or more of the following characteristics: not being retained on a Q-Sepharose column, being retained by a hydroxyapatite column, running as a single band with lower apparent molecular weight on SDS-PAGE in the absence of heat-denaturation when compared to the bacterial adhesin after heat-denaturation, being more resistant to protease digestion than GBS80 in conformer A, and eluting from a size exclusion chromatography column as a single monodisperse peak. In preferred embodiments, the GBS80 conformer will be substantially free of other conformers including by way of example, but not limitation, GBS80 in conformer F substantially free of GBS80 in conformer A, which will preferably, be less that at least about 20% conformer A, less than at least about 15% of conformer A, less than at least about 10% other conformers, at least about 5% other conformers, at least about 2% other conformers, or at least about 1 % other conformers of the protein. In other embodiments, the GBS80 may not be completely free of the other conformer including, by way of

example, GBS80 in conformer F may have between about 20% and about 1%, between about 15% and about 1%, between about 10% and about 1%, between about 5% and about 1%, or between about 2% and about 1% of GBS80 in conformer A.

In preferred embodiments, the GBS80 of the present invention will be capable of generating an immune response in a target organism such as a bird or a mammal, preferably a human subject. More preferably, the GBS80 will provide a target organism passive immunity and/or active immunity.

In some embodiments, the GBS80 compositions of the present invention may additionally include other immunogenic polypeptides from GBS 80 (including without limitation polypeptides and polysaccharides) or other pathogens.

Another aspect of the present invention provides methods of separating or isolating GBS80 of a particular conformer. A preferred embodiment of such methods includes providing a sample containing a mixture of GBS80 in two or more conformers and separating the two or more conformers using a separation technology selected from the group consisting of an anion exchange separation technology, an hydroxyapatite-based separation technology, and a friction coefficient-based separation technology. Examples of friction coefficient-based separation technologies are gel electrophoresis, size-exclusion chromatography, field-flow fractionation and velocity sedimentation centrifugation.

Another aspect of the present invention is antibodies that specifically bind to or recognize any GBS80 of the present invention. In certain embodiments, such antibodies may be polyclonal or monoclonal. In some embodiments, the antibody may be a chimeric antibody, a humanized antibody, or a fully human antibody. In some embodiments, the antibody may specifically bind to one conformer and not any other such as binding to conformer F and not conformer A. Additional embodiments are described more fully below regarding antibodies, methods of prepare, methods of screening and methods of using such antibodies.

As described more fully below, additional aspects of the present invention include methods of using the foregoing bacterial adhesins as (a) medicaments for treating or preventing infection due to *Streptococcus* bacteria; (b) diagnostics or immunodiagnostic assays for detecting the presence of *Streptococcus* bacteria or of antibodies raised against *Streptococcus* bacteria; and/or (c) reagents which can raise antibodies against *Streptococcus bacteria.*

Another aspect of the present disclosure includes methods of screening and/or testing peptides of the bacterial adhesins in a particular conformer for generation of an immune response, active immunization or passive immunization in a target organism. In some aspects the disclosure will involve contacting or administering the GBS80 composition of the present invention to the target organism and detecting antibodies in the target organism that recognize the bacterial adhesins composition. In some aspects, the target organism will be challenged with Streptococcus bacteria to determine whether the target organism has active immunity or passive immunity. Such methods of screening may be applied to any of the compositions of the present invention including, without limitation, GBS80 antibodies to GBS80 and pharmaceutical compositions for immunogenicity or antigenicity. A preferred aspect of such screening methods includes providing a bacterial adhesins and screening the polypeptide for antigenicity or immunogenicity. Where more than one bacterial adhesin is to be screened, a criterion may be applied to select one or more bacterial adhesins for further use. Such criteria may be used to select among two or more bacterial adhesins, three or more bacterial adhesins, five or more bacterial adhesins, ten or more bacterial adhesins, or twenty or more bacterial adhesins.

Another aspect of the present invention provides pharmaceutical compositions that include the GBS80 or antibodies of the present invention in a therapeutically effective amount (or an immunologically effective amount in a vaccine). In certain embodiments, the pharmaceutical compositions will be vaccines. The pharmaceutical vaccines may also have pharmaceutically acceptable carriers including adjuvants.

## DETAILED DESCRIPTION OF THE INVENTION

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, Mack Publishing Company, Easton, Pa., 19th Edition (1995); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Handbook of Surface and Colloidal Chemistry (Birdi, K.S. ed., CRC Press, 1997); Short Protocols in Molecular Biology, 4th ed. (Ausubel et al. eds., 1999, John Wiley & Sons); Molecular Biology Techniques: An Intensive Laboratory. Course, (Ream et al., eds., 1998, Academic Press); PCR (Introduction to Biotechniques Series), 2nd ed. (Newton & Graham eds., 1997, Springer Verlag); Peters and Dalrymple, Fields Virology (2d ed), Fields et al. (eds.), B.N. Raven Press, New York, NY.

As used herein, the term "conformer" refers to isolated isoforms of a bacterial adhesin, such as GBS 80, and fragments thereof which have the same or similar amino acid sequences but different immunogenicity and/or distinguishable biophysical properties as determined for example by Size Exclusion Chromatography (SEC).

As used herein "bacterial adhesins" refers to proteins belong to the group of surface-exposed bacterial proteins that are involved in host-tissue adhesion and colonization.

As used herein "gram-positive bacterial adhesins" refers to bacterial adhesins from gram positive bacteria. Preferred gram-positive bacteria are *S. pyogenes, S. agalactiae, S. pneumonaie, S. mutans, E. faecalis, E. faecium, C. difficile, L. monocytogenes,* or *C. diphtheriae.*

As used herein "bacterial adhesin GBS 80 homolog" refers to GBS 80 and all proteins related to GBS 80 by descent from a common ancestral DNA sequence encoding the ancestral protein. The term, homolog, may apply to the relationship between genes separated by an event of speciation and to the relationship between genes separated by the event of genetic duplication. One of skill in the art will readily recognize GBS 80 homologs based upon comparison of the amino acid sequences or the nucleic acid sequences encoding the proteins. Preferably that homology will be at least about 60% identity, at least about 70% identity, at least about 80% identity, at least about 85% identity, at least about 90% identity, at least about 92.5% identity, at least about 95% identity, at least about 96% identity, at least about 97% identity, at least about 98% identity, or at least about 99% identity.

As used herein "bacterial adhesin GBS 80 ortholog" refers to GBS 80 and all proteins in other bacterial species that evolved from a common ancestral gene by speciation. Such orthologs will have retained the same function in the course of evolution. One of skill in the art will readily recognize GBS 80 orthologs as the homolog in another bacterial species that has the greatest homology to GBS 80. Preferably that homology will be at least about 60% identity, at least about 70% identity, at least about 80% identity, at least about 85% identity, at least about 90% identity, at least about 92.5% identity, at least about 95% identity, at least about 96% identity, at least about 97% identity, at least about 98% identity, or at least about 99% identity.

As used herein "bacterial adhesin GBS 80 paralog" refers to GBS 80 and all proteins in the same bacterial species that evolved from a common ancestral gene by gene duplication. Such paralogs are genes related by duplication within a genome and therefore will have very similar but often distinct functional roles. One of skill in the art will readily recognize GBS 80 paralogs as the homolog in another bacterial species that has the greatest homology to GBS 80. Preferably that homology will be at least about Preferably that homology will be at least about 60% identity, at least about 70% identity, at least about 80% identity, at least about 85% identity, at least about 90% identity, at least about 92.5% identity, at least about 95% identity, at least about 96% identity, at least about 97% identity, at least about 98% identity, or at least about 99% identity. By way of example, but not limitation, GBS 59 is a bacterial adhesin GBS 80 paralog.

The definitions for bacterial adhesin GBS 80 homolog, bacterial adhesin GBS 80 ortholog, and bacterial adhesin GBS 80 paralog are representative definitions of the bacterial adhesins disclosed herein and GBS 59, GBS 104, and GBS 67 have corresponding definitions which may be used interchangeably with the GBS 80 definitions through this application. By way of example, without limitation, "bacterial adhesin GBS 80 homolog" as used in the Summary of the Invention may be replaced with "bacterial adhesin GBS 59 homolog."

## ADHESINS

As discussed above, the invention relates to GBS80 conformers, methods of separating or isolating the conformers and uses of the conformers. Adhesins are a large heterogeneous class of surface proteins involved in the adhesion and colonization of host tissues by both Gram-positive and Gram-negative bacteria. Adherence to host tissues is a key virulence determinant for pathogenic bacteria and many examples of adhesion mechanisms are known in the art.

A particularly effective adhesion apparatus is represented in several pathogens by fimbriae or pili. These are adhesive bacterial organelles which enable bacteria to target and to colonize special host tissues. They are long, threadlike surface structures made by the ordered assembly of different building elements, including adhesins. The surface exposure and the key role in pathogenesis of these proteins make them a particularly attractive target for immunogenic compositions and vaccines. Many such vaccines are known in the art. Pili, which have been long known to be important for capsulated gram negative bacteria such as Neisseria spp., have also been described in gram positive bacteria such as *Corynebacterium diphtheriae,* Actinomyces spp., *Streptococcuspneumoniae, Streptococcus agalactiae* and *Streptococcus pyogenes*. In these species, pili are formed by the covalent cross-linking of protein subunits by the action of sortase enzymes which cleave proteins containing the LPXTG sorting motif between the T and G residues and then link the cleaved protein to the ε-amino group of a conserved lysine in a pilin motif (VYPKN) in the pilin components themselves. Sortase enzymes also catalyze the covalent coupling of LPXTG proteins to the peptidoglycan cell wall. Adhesin subunits of this family are often found clustered in a genomic island.

## GBS 80

GBS 80 is a preferred bacteria adhesin of the present invention. GBS 80 refers to a putative cell wall surface anchor family protein and is one of the building subunits of a pilus structure. Nucleotide and amino acid sequences of GBS 80 sequenced from serotype V isolated strain 2603 V/R can be found in WO04041157. These sequences are also set forth below as SEQ ID NOS 1 and 2:

**SEQ ID NO. 1**

ATGAAATTATCGAAGAAGTTATTGTTTTCGGCTGCTGTTTTAACAATGGTGGCGGGGTCAACTGTTGAACCAG
TAGCTCAGTTTGCGACTGGAATGAGTATTGTAAGAGCTGCAGAAGTGTCACAAGAACGCCCAGCGAAAACAAC
AGTAAATATCTATAAATTACAAGCTGATAGTTATAAATCGGAAATTACTTCTAATGGTGGTATCGAGAATAAA
GACGGCGAAGTAATATCTAACTATGCTAAACTTGGTGACAATGTAAAAGGTTTGCAAGGTGTACAGTTTAAAC
GTTATAAAGTCAAGACGGATATTTCTGTTGATGAATTGAAAAAATTGACAACAGTTGAAGCAGCAGATGCAAA
AGTTGGAACGATTCTTGAAGAAGGTGTCAGTCTACCTCAAAAAACTAATGCTCAAGGTTTGGTCGTCGATGCT
CTGGATTCAAAAAGTAATGTGAGATACTTGTATGTAGAAGATTTAAAGAATTCACCTTCAAACATTACCAAAG
CTTATGCTGTACCGTTTGTGTTGGAATTACCAGTTGCTAACTCTACAGGTACAGGTTTCCTTTCTGAAATTAA
TATTTACCCTAAAAACGTTGTAACTGATGAACCAAAAACAGATAAAGATGTTAAAAAATTAGGTCAGGACGAT
GCAGGTTATACGATTGGTGAAGAATTCAAATGGTTCTTGAAATCTACAATCCCTGCCAATTTAGGTGACTATG
AAAAATTTGAAATTACTGATAAATTTGCAGATGGCTTGACTTATAAATCTGTTGGAAAAATCAAGATTGGTTC
GAAAACACTGAATAGAGATGAGCACTACACTATTGATGAACCAACAGTTGATAACCAAAATACATTAAAAATT
ACGTTTAAACCAGAGAAATTTAAAGAAATTGCTGAGCTACTTAAAGGAATGACCCTTGTTAAAAATCAAGATG
CTCTTGATAAAGCTACTGCAAATACAGATGATGCGGCATTTTTGGAAATTCCAGTTGCATCAACTATTAATGA
AAAAGCAGTTTTAGGAAAAGCAATTGAAAATACTTTTGAACTTCAATATGACCATACTCCTGATAAAGCTGAC
AATCCAAAACCATCTAATCCTCCAAGAAAACCAGAAGTTCATACTGGTGGGAAACGATTTGTAAAGAAAGACT
CAACAGAAACACAAACACTAGGTGGTGCTGAGTTTGATTTGTTGGCTTCTGATGGGACAGCAGTAAAATGGAC
AGATGCTCTTATTAAAGCGAATACTAATAAAAACTATATTGCTGGAGAAGCTGTTACTGGGCAACCAATCAAA
TTGAAATCACATACAGACGGTACGTTTGAGATTAAAGGTTTGGCTTATGCAGTTGATGCGAATGCAGAGGGTA
CAGCAGTAACTTACAAATTAAAAGAAACAAAAGCACCAGAAGGTTATGTAATCCCTGATAAAGAAATCGAGTT
TACAGTATCACAAACATCTTATAATACAAAACCAACTGACATCACGGTTGATAGTGCTGATGCAACACCTGAT
ACAATTAAAAACAACAAACGTCCTTCAATCCCTAATACTGGTGGTATTGGTACGGCTATCTTTGTCGCTATCG
GTGCTGCGGTGATGGCTTTTGCTGTTAAGGGGATGAAGCGTCGTACAAAAGATAAC

**SEQ ID NO: 2**

MKLSKKLLFSAAVLTMVAGSTVEPVAQFATGMSIVRAAEVSQERPAKTTVNIYKLQADSYKSEITSNGGIENK
DGEVISNYAKLGDNVKGLQGVQFKRYKVKTDISVDELKKLTTVEAADAKVGTILEEGVSLPQKTNAQGLVVDA
LDSKSNVRYLYVEDLKNSPSNITKAYAVPFVLELPVANSTGTGFLSEINIYPKNVVTDEPKTDKDVKKLGQDD
AGYTIGEEFKWFLKSTIPANLGDYEKFEITDKFADGLTYKSVGKIKIGSKTLNRDEHYTIDEPTVDNQNTLKI
TFKPEKFKEIAELLKGMTLVKNQDALDKATANTDDAAFLEIPVASTINEKAVLGKAIENTFELQYDHTPDKAD
NPKPSNPPRKPEVHTGGKRFVKKDSTETQTLGGAEFDLLASDGTAVKWTDALIKANTNKNYIAGEAVTGQPIK
LKSHTDGTFEIKGLAYAVDANAEGTAVTYKLKETKAPEGYVIPDKEIEFTVSQTSYNTKPTDITVDSADATPD
TIKNNKRPS*IPNTG*GIGTAIFVAIGAAVMAFAVKGMKRRTKDN

Aspects of the invention may include fragments of GBS80, such as those described in U.S. Prov. Ser. App. No. 60/812,145. In some instances, removal of one or more domains, such as a leader or signal sequence region, a transmembrane region, a cytoplasmic region or a cell wall anchoring motif, may facilitate cloning of the gene encoding the antigen and/or recombinant expression of the GBS protein. In addition, fragments comprising immunogenic epitopes of GBS antigens may be used in the compositions of the invention.

GBS 80 contains an N-terminal leader or signal sequence region which is indicated by the underlined sequence at the beginning of SEQ ID NO: 2 above. In one embodiment, one or more amino acids from the leader or signal sequence region of GBS 80 are removed. An example of such a GBS 80 fragment is set forth below as SEQ ID NO: 3:

**SEQ ID NO: 3**

AEVSQERPAKTTVNIYKLQADSYKSEITSNGGIENKDGEVISNYAKLGDNVKGLQGVQFKRYKVKTDISVDEL
KKLTTVEAADAKVGTILEEGVSLPQKTNAQGLVVDALDSKSNVRYLYVEDLKNSPSNITKAYAVPFVLELPVA
NSTGTGFLSEINIYPKNVVTDEPKTDKDVKKLGQDDAGYTIGEEFKWFLKSTIPANLGDYEKFEITDKFADGL
TYKSVGKIKIGSKTLNRDEHYTIDEPTVDNQNTLKITFKPEKFKEIAELLKGMTLVKNQDALDKATANTDDAA
FLEIPVASTINEKAVLGKAIENTFELQYDHTPDKADNPKPSNPPRKPEVHTGGKRFVKKDSTETQTLGGAEFD
LLASDGTAVKWTDALIKANTNKNYIAGEAVTGQPIKLKSHTDGTFEIKGLAYAVDANAEGTAVTYKLKETKAP
EGYVIPDKEIEFTVSQTSYNTKPTDITVDSADATPDTIKNNKRPSIPNTGGIGTAIFVAIGAAVMAFAVKGMK
RRTKDN

GBS 80 contains a C-terminal transmembrane region which is indicated by the underlined sequence near the end of SEQ ID NO: 2 above. In one embodiment, one or more amino acids from the transmembrane region and/or a cytoplasmic region are removed. An example of such a GBS 80 fragment is set forth below as SEQ ID NO: 4:

**SEQ ID NO: 4**

MKLSKKLLFSAAVLTMVAGSTVEPVAQFATGMSIVRAAEVSQERPAKTTVNIYKLQADSYKSEITSNGGIENK
DGEVISNYAKLGDNVKGLQGVQFKRYKVKTDISVDELKKLTTVEAADAKVGTILEEGVSLPQKTNAQGLVVDA
LDSKSNVRYLYVEDLKNSPSNITKAYAVPFVLELPVANSTGTGFLSEINIYPKNVVTDEPKTDKDVKKLGQDD
AGYTIGEEFKWFLKSTIPANLGDYEKFEITDKFADGLTYKSVGKIKIGSKTLNRDEHYTIDEPTVDNQNTLKI
TFKPEKFKEIAELLKGMTLVKNQDALDKATANTDDAAFLEIPVASTINEKAVLGKAIENTFELQYDHTPDKAD
NPKPSNPPRKPEVHTGGKRFVKKDSTETQTLGGAEFDLLASDGTAVKWTDALIKANTNKNYIAGEAVTGQPIK
LKSHTDGTFEIKGLAYAVDANAEGTAVTYKLKETKAPEGYVIPDKEIEFTVSQTSYNTKPTDITVDSADATPD
TIKNNKRPS*IPNTG*

GBS 80 contains an amino acid motif indicative of a cell wall anchor: SEQ ID NO: 5 "IPNTG" (shown in italics in SEQ ID NO: 2 above). In some recombinant host cell systems, it may be preferable to remove this motif to facilitate secretion of a recombinant GBS 80 protein from the host cell. Accordingly, in one preferred fragment of GBS 80 for use in the invention, the transmembrane and/or cytoplasmic regions and the cell wall anchor motif are removed from GBS 80. An example of such a GBS 80 fragment is set forth below as SEQ ID NO: 6.

**SEQ ID NO: 6**

MKLSKKLLFSAAVLTMVAGSTVEPVAQFATGMSIVRAAEVSQERPAKTTVNIYKLQADSYKSEITSNGGIENK
DGEVISNYAKLGDNVKGLQGVQFKRYKVKTDISVDELKKLTTVEAADAKVGTILEEGVSLPQKTNAQGLVVDA
LDSKSNVRYLYVEDLKNSPSNITKAYAVPFVLELPVANSTGTGFLSEINIYPKNVVTDEPKTDKDVKKLGQDD
AGYTIGEEFKWFLKSTIPANLGDYEKFEITDKFADGLTYKSVGKIKIGSKTLNRDEHYTIDEPTVDNQNTLKI
TFKPEKFKEIAELLKGMTLVKNQDALDKATANTDDAAFLEIPVASTINEKAVLGKAIENTFELQYDHTPDKAD
NPKPSNPPRKPEVHTGGKRFVKKDSTETQTLGGAEFDLLASDGTAVKWTDALIKANTNKNYIAGEAVTGQPIK
LKSHTDGTFEIKGLAYAVDANAEGTAVTYKLKETKAPEGYVIPDKEIEFTVSQTSYNTKPTDITVDSADATPD
TIKNNKRPS

Alternatively, in some recombinant host cell systems, it may be preferable to use the cell wall anchor motif to anchor the recombinantly expressed protein to the cell wall. The extracellular domain of the expressed protein may be cleaved during purification or the recombinant protein may be left attached to either inactivated host cells or cell membranes in the final composition.

In one embodiment, the leader or signal sequence region, the transmembrane and cytoplasmic regions and the cell wall anchor motif are removed from the GBS 80 sequence. An example of such a GBS 80 fragment is set forth below as SEQ ID NO: 7.

**SEQ ID NO: 7**

AEVSQERPAKTTVNIYKLQADSYKSEITSNGGIENKDGEVISNYAKLGDNVKGLQGVQFKRYKVKTDISVDEL
KKLTTVEAADAKVGTILEEGVSLPQKTNAQGLVVDALDSKSNVRYLYVEDLKNSPSNITKAYAVPFVLELPVA
NSTGTGFLSEINIYPKNVVTDEPKTDKDVKKLGQDDAGYTIGEEFKWFLKSTIPANLGDYEKFEITDKFADGL
TYKSVGKIKIGSKTLNRDEHYTIDEPTVDNQNTLKITFKPEKFKEIAELLKGMTLVKNQDALDKATANTDDAA
FLEIPVASTINEKAVLGKAIENTFELQYDHTPDKADNPKPSNPPRKPEVHTGGKRFVKKDSTETQTLGGAEFD
LLASDGTAVKWTDALIKANTNKNYIAGEAVTGQPIKLKSHTDGTFEIKGLAYAVDANAEGTAVTYKLKETKAP
EGYVIPDKEIEFTVSQTSYNTKPTDITVDSADATPDTIKNNKRPS

Applicants have identified a particularly immunogenic fragment of the GBS 80 protein. This immunogenic fragment is located towards the N-terminus of the protein and is underlined in the GBS 80 SEQ ID NO: 2 sequence below. The underlined fragment is set forth below as SEQ ID NO: 8.

**SEQ ID NO: 2**
MKLSKKLLFSAAVLTMVAGSTVEPVAQFATGMSIVRAAEVSQERPAKTTVNIYKLQADSYKSEITSNGGIENK
DGEVISNYAKLGDNVKGLQGVQFKRYKVKTDISVDELKKLTTVEAADAKVGTILEEGVSLPQKTNAQGLVVDA
LDSKSNVRYLYVEDLKNSPSNITKAYAVPFVLELPVANSTGTGFLSEINIYPKNVVTDEPKTDKDVKKLGQDD
AGYTIGEEFKWFLKSTIPANLGDYEKFEITDKFADGLTYKSVGKIKIGSKTLNRDEHYTIDEPTVDNQNTLKI
TFKPEKFKEIAELLKGMTLVKNQDALDKATANTDDAAFLEIPVASTINEKAVLGKAIENTFELQYDHTPDKAD
NPKPSNPPRKPEVHTGGKRFVKKDSTETQTLGGAEFDLLASDGTAVKWTDALIKANTNKNYIAGEAVTGQPIK
LKSHTDGTFEIKGLAYAVDANAEGTAVTYKLKETKAPEGYVIPDKEIEFTVSQTSYNTKPTDITVDSADATPD
TIKNNKRPS*IPNTG*GIGTAIFVAIGAAVMAFAVKGMKRRTKDN

**SEQ ID NO: 8**
AEVSQERPAKTTVNIYKLQADSYKSEITSNGGIENKDGEVISNYAKLGDNVKGLQGVQFKRYKVKTDISVDEL
KKLTTVEAADAKVGTILEEGVSLPQKTNAQGLVVDALDSKSNVRYLYVEDLKNSPSNITKAYAVPFVLELPVA
NSTGTGFLSEINIYPKNVVTDEPKTDKDVKKLGQDDAGYTIGEEFKWFLKSTIPANLGDYEKFEITDKFADGL
TYKSVGKIKIGSKTLNRDEHYTIDEPTVDNQNTLKITFKPEKFKEIAELLKG

## CONFORMER F

The inventors have discovered that antigens of the adhesin family can be isolated as two distinct main isoforms: (a) conformer F and (b) conformer A. These isoforms, while sharing the same or similar amino acid sequences, can be separated according to their differential biophysical properties using protein purification steps such as chromatographic separation. For instance, in ion-exchange chromatography conformer A is characterized in that it's adsorbed to Q-Sepharose while conformer F is eluted. A process for isolation and purification of conformer F can be carried out also by chromatography with hydroxyapatite as described for instance in example 1. The two isoforms run at different apparent MW during non-denaturing sodium dodecyl sulfate-polyacrylamide gel electrophoresis (SDS-PAGE), whereas show the same apparent MW once they have been heat-denatured. When run on a size exclusion chromatography column, larger elution volumes are required for the conformer F compared to conformer A. The two isoforms show also different stability and immunogenicity, with conformer F being the more stable and immunogenic form. In fact, over the time, a purified lot of conformer A will present additional isoforms, including conformer F. Immunization with a purified conformer F give an improved immune response when compared to an immunization with the conformer A or a isoform mix wherein conformer F is only a subfraction. Conformer F shows also increased resistance to protease digestion.

In some embodiments, the conformers have the amino acid sequence of a full-length GBS80. In other embodiments, these conformers have the amino acid sequence of GBS80 fragments which can be found in conformer F. Such fragments can be readily identified using methods known to those of skill in the art and described herein.

For example the inventors have discovered that the recombinant fragment of GBS 80 set forth as SEQ ID NO: 7 indeed can be purified as one of the two conformers described above and have shown that conformer F has improved immunogenicity over conformer A. MALDI mass spectrometry (MS) and sequencing of the amino terminus confirmed that the amino acidic sequence of the two isoforms coincide. On a non-denaturing SDS-PAGE conformer F shows a lower molecular weight compared to conformer A but, when samples are boiled, the two isoforms run at the same apparent MW. Accordingly a similar anomaly is observed when the protein preparation is applied to a gel filtration column where conformer F elutes as a monodisperse peak at a higher elution volume. This behavior is consistent with the lower apparent molecular weight observed in non denaturing SDS-PAGE. As explained in further details below, stability tests furthermore indicate that a preparation of GBS 80 recovered from the Q Sepharose adsorbed fraction elutes from a gel filtration column as a polydispersed peak over the time, indicating that additional isoforms are generated. The additional peak with the lowest elution volume corresponds to conformer F.

Over time, any residual conform A may convert to conformer F. Preferably, when the immunogenic compositions of the invention are about to be administered to a mammal, the composition is substantially free of conformer A.

## Expression systems

The bacterial adhesin conformer F may be recombinantly produced via a variety of different expression systems; for example those used with mammalian cells, baculoviri, plants, bacteria, and yeast.

### *i. Mammalian Systems*

Mammalian expression systems are known in the art. A mammalian promoter is any DNA sequence capable of binding mammalian RNA polymerase and initiating the downstream (3') transcription of a coding sequence (e.g.,

structural gene) into mRNA. A promoter will have a transcription initiating region, which is usually placed proximal to the 5' end of the coding sequence, and a TATA box, usually located 25- 30 base pairs (bp) upstream of the transcription initiation site. The TATA box is thought to direct RNA polymerase 11 to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation (Sambrook et. (1989) Expression of Cloned Genes in Mammalian Cells. In Molecular Cloning: A Laboratory Manual, 2nd ed.).

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallothionein gene, also provide useful promoter sequences, Expression may be either constitutive or regulated (inducible), depending on the promoter can be induced with glucocorticoid in hormone-responsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter (Maniatis et al. (1987) Science 236:1237; Alberts et al. (1989) Molecular Biology of the Cell, 2nd ed.). Enhancer elements derived from viruses may be particularly useful, because they usually have a broader host range. Examples include the SV40 early gene enhancer (Dijkema et al (1985) EMBO J. 4:7611) and the enhancer/promoters derived from the long terminal repeat (LTR) of the Rous Sarcoma Virus (Gorman et al. (1982b) Proc. Natl. Acad. Sci. 79:6777) and from human cytomegalovirus (Boshart et al. (1985) Cell 41:5211). Additionally, some enhancers are regulatable and become active only in the presence of an inducer, such as a hormone or metal ion (Sassone-Corsi and Borelli (1986) Trends Genet. 2:215; Maniatis et al. (1987) Science 236:1237).

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by in vitro incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells, Preferably, there are processing sites encoded between the leader fragment and the foreign gene that can be cleaved either in vivo or in vitro. The leader sequence fragment usually encodes a signal peptide comprised of hydrophobic amino acids which direct the secretion of the protein from the cell. The adenovirus tripartite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation (Bimstiel et al. (1985) Cell 41:349; Proudfoot and Whitelaw (1988) Termination and 3' end processing of eukaryotic RNA. In Transcription and splicing (ed. B.D. Hames and D.M, Glover); Proudfoot (1989) Trends Biochem. Sci. 14:1051). These sequences direct the transcription of an mRNA which can be translated into the polypeptide encoded by the DNA. Examples of transcription terminater/polyadenylation signals include those derived from SV40 (Sambrook et al (1989) Expression of cloned genes in cultured mammalian cells. In Molecular Cloning: A Laboratory Manual).

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (e.g., plasmids) capable of stable maintenance in a host, such as mammalian cells or bacteria. Mammalian replication systems include those derived from animal viruses, which require trans-acting factors to replicate. For example, plasmids containing the replication systems of papovaviri, such as SV40 (Gluzman (1981) Cell 23:1751) or polyomavirus, replicate to extremely high copy number in the presence of the appropriate viral T antigen. Additional examples of mammalian replicons include those derived from bovine papillomavirus and Epstein- Barf virus. Additionally, the replicon may have two replication systems, thus allowing it to be maintained, for example, in mammalian cells for expression and in a prokaryotic host for cloning and amplification. Examples of such mammalian-bacteria shuttle vectors include pMT2 (Kaufman et al. (1989) Mol. Cell. Biol. 9:946) and pHEBO (Shimizu et al. (1986) Mol. Cell. Biol. 6:10741). The transformation procedure used depends upon the host to be transformed. Methods for introduction of heterologous polynucleotides into mammalian cells are known in the art and include dextran-mediated transfection, calcium phosphate precipitation, protoplast fusion, electroporation, encapsulation of the polynucleotide(s) in 11posomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e. g., Hep G2), and a number of other cell lines.

*ii. Baculovirus Systems*

A polynucleotide encoding the conformer F can also be inserted into a suitable insect expression vector operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media, After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, inter alia, Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (e.g., plasmids) capable of stable maintenance in a host, such as a bacterium, The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT (Luckow and Summers, Virology (1989) 17:31).

The plasmid usually also contains the polyhedrin polyadenylation signal (Miller et al. (1988) Ann. Rev. Microbiol., 42:177) and a prokaryotic ampicillin-resistance (amp) gene and origin of replication for selection and propagation in E. coli.

Baculovirus transfer vectors usually contain a baculovirus promoter. A baculovirus promoter is any DNA sequence capable of binding a baculovirus RNA polymerase and initiating the downstream (5' to 3') transcription of a coding sequence (e.g., structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein (Friesen et al., (1986) The Regulation of Baculovirus Gene Expression, in: The Molecular Biology of Baculoviruses (ed. Walter Doerfler); EPO Publ. Nos. 127 839 and 155 476) and the gene encoding the p10 protein (Vlak et al, (1988), J. Gen. Virol. 69:765).

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell post-translational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human $\alpha$-interferon (Maeda et al., (1985), Nature 315:592); human gastrin-releasing peptide (Lebacq-Verheyden et al., (1988), Molec. Cell. Biol. 8:3129); human IL-2 (Smith et al., (1985) Proc. Nat'l Acad. Sci. USA, 82:8404); mouse IL-3 (Miyajima et al., (1987) Gene 58:273); and human glucocerebrosidase (Martin et al. (1988) DNA, 7:99), can also be used to provide for secretion in insects.

A recombinant polypeptide or polyprotein may be expressed intracellularly or, if it is expressed with the proper regulatory sequences, it can be secreted. Good intracellular expression of non-fused foreign proteins usually requires heterologous genes that ideally have a short leader sequence containing suitable translation initiation signals preceding

an ATG start signal. If desired, methionine at the N-terminus may be cleaved from the mature protein by in vitro incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co- transformed with the heterologous DNA of the transfer vector and the genomic DNA of wild type baculovirus -- usually by co-transfection. The promoter and transcription termination sequence of the construct will usually comprise a 2-5 kb section of the baculovirus genome. Methods for introducing heterologous DNA into the desired site in the baculovirus virus are known in the art, (See Summers and Smith supra; Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene (Miller et al., (1989), Bioessays 4:91). The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%); thus, the majority of the virus produced after cotransfection is still wildtype virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 μm in size, are highly refractile, giving them a bright shiny appearance that is readily visualized under the light microscope. Cells infected with recombinant viruses lack occlusion bodies. To distinguish recombinant virus from wildtype virus, the transfection supernatant is plaqued onto a monolayer of insect cells by techniques known to those skilled in the 'art. Namely, the plaques are screened under the light microscope for the presence (indicative of wild-type virus) or absence (indicative of recombinant virus) of occlusion bodies *(*Current Protocols in Microbiology, Vol. 2 (Ausubel et al. eds) at 16.8 (Supp. 10, 1990); Summers and Smith, supra; Miller et al. (1989)).

Recombinant baculovirus expression vectors have been developed for infection into several insect cells. For example, recombinant baculoviruses have been developed for, inter alia: *Aedes aegypti, Autographa californica, Bombyx mori, Drosophila melanogaster, Spodoptera frugiperda,* and *Trichoplusia ni* (WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. See, e.g., Summers and Smith supra.

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g., HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g., proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

*iii. Plant Systems*

There are many plant cell culture and whole plant genetic expression systems known in the art. Exemplary plant cellular genetic expression systems include those described in patents, such as: US 5,693,506; US 5, 659,122; and US 5,608,143. Additional examples of genetic expression in plant cell culture have been described by Zenk, Phytochemistry 30:3861- 3863 (1991). Descriptions of plant protein signal peptides may be found in addition to the references described above in Vaulcombe et al., Mol. Gen. Genet. 209:33-40 (1987); Chandler et al., Plant Molecular Biology 3: 407-418 (1984); Rogers, J. Biol. Chem. 260:3731-3738 (1985); Rothstein et al., Gene 55:353-356 (1987); Whittier et

al., Nucleic Acids Research 15:2515-2535 (1987); Wirsel et al., Molecular Microbiology 3:3-14 (1989); and Yu et al., Gene 122:247-253 (1992). A description of the regulation of plant gene expression by the phytohormone, gibberellic acid and secreted enzymes induced by gibberellic acid can be found in R.L. Jones and J. MacMillin, Gibberellins: in: Advanced Plant Physiology, Malcolm B. Wilkins, ed., 1984 Pitman Publishing Limited, London, pp. 21-52. References that describe other metabolically-regulated genes: Sheen, Plant Cell, 2:1027-1038 (1990); Maas et al., EMBO J. 9: 3447-3452 (1990); Benkel and Hickey, Proc. Natl. Acad. Sci. 84:1337-1339(1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, Plant Mol. Biol. Reptr, 11 (2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules which encode the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eukaryotic cell it is desirable to determine whether any portion of the cloned gene contains sequences which will be processed out as introns by the host's splicosome machinery. If so, site-directed mutagenesis of the "intron" region may be conducted to prevent losing a portion of the genetic message as a false intron code (Reed and Maniatis, Cell 41:95-105, 1985).

The vector can be microinjected directly into plant cells by use of micropipettes to mechanically transfer the recombinant DNA (Crossway, Mol. Gen. Genet, 202:179-185, 1985). The genetic material may also be transferred into the plant cell by using polyethylene glycol (Krens, et al., Nature, 296, 72-74, 1982). Another method of introduction of nucleic acid segments is high velocity ballistic penetration by small particles with the nucleic acid either within the matrix of small beads or particles, or on the surface (Klein, et al., Nature, 327, 70-73, 1987 and Knudsen and Muller, 1991, Planta, 185:330-336) teaching particle bombardment of barley endosperm to create transgenic barley. Yet another method of introduction would be fusion of protoplasts with other entities, either minicells, cells, lysosomes or other fusible lipid-surfaced bodies, Fraley, et al., Proc. Natl. Acad. Sci. USA, 79, 1859-1863, 1982.

The vector may also be introduced into the plant cells by electroporation (Fromm et al., Proc. Natl Acad. Sci. USA 82:5824, 1985). In this technique, plant protoplasts are electroporated in the presence of plasmids containing the gene construct. Electrical impulses of high field strength reversibly permeablize membranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium,*

*Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura.*

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

*iv. Bacterial Systems*

Bacterial expression techniques are known in the art. A bacterial promoter is any DNA sequence capable of binding bacterial RNA polymerase and initiating the downstream (3') transcription of a coding sequence (e.g., a structural gene) into mRNA, A promoter will have a transcription initiation region which is usually placed proximal to the 5' end of the coding sequence. This transcription initiation region usually includes an RNA polymerase binding site and a transcription initiation site. A bacterial promoter may also have a second domain called an operator that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in *Escherichia coli* (Raibaud et al. (1984) Annu. Rev. Genet. 18:173). Regulated expression may therefore either be positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (lac) (Chang et al. (1977) Nature 198:1056), and maltose. Additional examples include promoter sequences derived from biosynthetic enzymes such as tryptophan (trp) (Goeddel et al. (1980) Nuc. Acids Res. 8:4057; Yelverton et al. (1981) Nucl. Acids Res. 9:731; US patent 4,738, 921; EP-A-0036776 and EP-A- 0121775); and the β-lactamase (bla) promoter system (Weissmann (1981) "The cloning of interferon and other mistakes." In Interferon 3 (ed. 1. Gresser)). The bacteriophage lambda PL (Shimatake et al. (1981) Nature 292:128) and T5 (US patent 4,689,406) promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter (US patent 4,551,4331). For example, the tac promoter is a hybrid trp-lac promoter comprised of both trp promoter and lac operon sequences that is regulated by the lac repressor (Amann et al. (1983) Gene 25:167; de Boer et al. (1983) Proc. Natl. Acad. Sci. 80:21). Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system (Studier et al. (1986) J. Mol. Biol. 189:113; Tabor et al. (1985) Proc Natl. Acad. Sci. 82:1074). In addition, a hybrid promoter can also be comprised of a bacteriophage promoter and an E. coli operator region (EPO-A-0 267 851).

In addition to a functioning promoter sequence, an efficient ribosome binding site is also useful for the expression of foreign genes in prokaryotes. In *E. coli,* the ribosome binding site is called the Shine-Dalgarno (SD) sequence and includes an initiation codon (ATG) and a sequence 3-9 nucleotides in length located 3-11 nucleotides upstream of the initiation codon (Shine et al. (1975) Nature 254:34). The SD sequence is thought to promote binding of mRNA to the

ribosome by the pairing of bases between the SD sequence and the 3' end of E. coli 16S rRNA (Steitz et al. (1979) "Genetic signals and nucleotide sequences in messenger RNA." In Biological Regulation and Development: Gene Expression (ed. R.F. Goldberger)). To express eukaryotic genes and prokaryotic genes with weak ribosome-binding site (Sambrook et al. (1989) "Expression of cloned genes in Escherichia coli." In Molecular Cloning: A Laboratory Manual).

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by in vitro incubation with cyanogen bromide or by either in vivo on in vitro incubation with a bacterial methionine N- terminal peptidase (EPO-A-0 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene (Nagai et al. (1984) Nature 309:8101). Fusion proteins can also be made with sequences from the lacZ (Jia et al. (1987) Gene 60:197), trpE (Allen et al. (1987) J. Biotechnol. 5:93; Makoff et al. (1989) J. Gen. Microbiol. 135:11), and Chey (EP-A-0 324 647) genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (e.g., ubiquitin specific processing-protease) to cleave the ubiquitin from the foreign protein. Through this method, native foreign protein can be isolated (Miller et al. (1989) Biotechnology 7:698).

Alternatively, foreign proteins can also be secreted from the cell by creating chimeric DNA molecules that encode a fusion protein comprised of a signal peptide sequence fragment that provides for secretion of the foreign protein in bacteria (US patent 4,336,336). The signal sequence fragment usually encodes a signal peptide comprised of hydrophobic amino acids which direct the secretion of the protein from the cell. The protein is either secreted into the growth media (gram-positive bacteria) of into the periplasmic space, located between the inner and outer membrane of the cell (gram-negative bacteria). Preferably there are processing sites, which can be cleaved either in vivo or in vitro encoded between the signal peptide fragment and the foreign gene.

DNA encoding suitable signal sequences can be derived from genes for secreted bacterial proteins, such as the E. coli outer membrane protein gene (ompA) (Masui et al. (1983), in: Experimental Manipulation of Gene Expression; Ghrayeb et al. (1984) EMBO J. 3:2437) and the E. coli alkaline phosphatase signal sequence (phoA) (Oka et al. (1985) Proc. Natl. Acad. Sci. 82:7212). As an additional example, the signal sequence of the alpha-amylase gene from various Bacillus strains can be used to secrete heterologous proteins from B. subtilis (Palva et al. (1982) Proc. Natl. Acad. Sci. USA 79:5582; EP-A-0 244 042).

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the trp gene in E, coli as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (e.g., plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome, For example, integrating vectors constructed with DNA from various Bacillus strains integrate into the Bacillus chromosome (EP-A- 0 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline (Davies et al. (1978) Annu. Rev. Microbiol. 32:469). Selectable markers may

also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon, or developed into an integrating vector, as described above.

Expression and transformation vectors, either extra-chromosomal replicons or integrating vectors, have been developed for transformation into many bacteria. For example, expression vectors have been developed for, inter alia, the following bacteria: Bacillus subtilis (Palva et al. (1982) Proc. Natl. Acad. Sci. USA 79:5582; EP-A-0 036 259 and EP-A-0 063 953; WO 84/04541), Escherichia coli (Shimatake et al. (1981) Nature 292:128; Amann et al. (1985) Gene 40: 183; Studier et al. (1986) J. Mol. Biol. 189:113; EP-A-0 036 776,EPA-0 136 829 and EP-A-0 136 907), Streptococcus cremoris (Powell et al. (1988) Appl. Environ. Microbiol. 54:655), Streptococcus lividans (Powell et at (1988) Appl. Environ. Microbiol. 54:655), and Streptomyces lividans (US patent 4,745,056).

Methods of introducing exogenous DNA into bacterial hosts are well- known in the art, and usually include either the transformation of bacteria treated with CaC12 or other agents, such as divalent cations and DMSO. DNA can also be introduced into bacterial cells by electroporation. Transformation procedures usually vary with the bacterial species to be transformed. See e.g., (Masson et al. (1989) FEMS Microbiol. Lett. 60:273; Palva et al. (1982) Proc. Natl. Acad. Sci. USA 79:5582; EP-A-0 036 259 and EP-A-0 063 953; WO 84/04541, Bacillus) (Miller et al. (1988) Proc. Natl. Acad. Sci. 85:856; Wang et al. (1990) J. Bacteriol. 172:949, Campylobacter), (Cohen et al. (1973) Proc. Natl. Acad. Sci. 69:2110; Dower et al. (1988) Nucleic Acids Res. 16:6127; Kushner (1978) "An improved method for transformation of Escherichia coli with ColEl-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia), (Chassy et al. (1987) FEMS Microbiol. Lett. 44:173, Lactobacillus), (Fiedler et al. (1988) Anal. Biochem 170:38, Pseudomonas), (Augustin et al. (1990) FEMS Microbiol. Lett. 66:203, Staphylococcus), (Barany et al. (1980) J. Bacteriol. 144:698; Harlander (1987) "Transformation of Streptococcus lactis by electroporation, in: Streptococcal Genetics (ed. J. Ferretti and R. Curtiss 111); Perry et al. (1981) Infect. Immun. 32:1295; Powell et al: (1988) Appl. Environ. Microbiol. 54:655; Somkuti et al. (1987) Proc. 4th Evr. Cong. Biotechnology 1:412, Streptococcus).

*v. Yeast Expression*

Yeast expression systems are also known to one of ordinary skill in the art. A yeast promoter is any DNA sequence capable of binding yeast RNA polymerase and initiating the downstream 3') transcription of a coding sequence (e.g., structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS, but may be enhanced with one or more UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EP-A-0 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO-A-0 329 203). The yeast PH05 gene, encoding acid phosphatase, also provides useful promoter sequences (Myanohara et al. (1983) Proc. Natl. Acad. Sci. USA 80: 1).

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (US Patent Nos. 4,876,197 and 4,880, 734). Other examples of hybrid promoters include promoters which consist of the regulatory sequences of either the AD112, GAL4, GALIO, OR PH05 genes, combined with the transcriptional activation region of a glycolytic enzyme gene such as GAP or PyK (EP-A-0 164 556). Furthermore, a yeast promoter can include naturally occurring promoters of non-yeast origin that have the ability to bind yeast RNA polymerase and initiate transcription. Examples of such promoters include, inter alia, (Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes in the Yeast Saccharomyces cerevisiae," in: Plasmids of Medical, Environmental and Commercial Importance (eds. K.N. Timmis and A. Puhler); Mercerau-Puigalon et al. (1980) Gene 11:163; Panthier et al. (1980) Curr. Genet. 2:109).

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by in vitro incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See e.g., EP-A-0 196 056. Another example is a ubiquitin fusion protein. Such a fusion protein is made with the ubiquitin region that preferably retains a site for a processing enzyme (e.g., ubiquitin specific processing protease) to cleave the ubiquitin from the foreign protein. Through this method, therefore, native foreign protein can be isolated (e.g., WO88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either in vivo or in vitro. The leader sequence fragment usually encodes a signal peptide comprised of hydrophobic amino acids which direct the secretion of the protein from the cell.

DNA encoding suitable signal sequences can be derived from genes for secreted yeast proteins, such as the yeast invertase gene (EP-A-0 012 873; JPO. 62,096,086) and the A-factor gene (US patent 4,588,684). Alternatively, leaders of non-yeast origin, such as an interferon leader, exist that also provide for secretion in yeast (EP-A-0 060 057).

A preferred class of secretion leader sequences is that which employs a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (US Patents 4,546,083 and 4,870,008; EP-A-0 324 274). Additional leaders employing an alpha- factor leader fragment that provides for secretion include hybrid alpha-factor leaders made with a presequence of a first yeast, but a pro-region from a second yeast alpha factor. (e.g., see W 0 89/02463.) Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (e.g., plasmids) capable of stable maintenance in a host, such as yeast or bacteria. The replicon may have two replication systems, thus allowing it to be maintained, for example, in yeast for expression and in a prokaryotic host for cloning and amplification, Examples of such yeastbacteria shuttle vectors include YEp24 (Botstein et al. (1979) Gene 8:17-24), pCl/l (Brake et al. (1984) PNAS USA 81:4642-4646), and YRp17 (Stinchcomb et al. (1982) J. Mol. Biol. 158:157). In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Either a high or low copy number vector may be selected, depending upon the effect of the vector and the foreign protein on the host. See e.g., Brake et al., supra.

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome (Orr-Weaver et al. (1983) Methods in Enzymol. 101:228-245). An integrating vector may be directed to a specific locus in yeast by selecting the appropriate homologous sequence for inclusion in the vector. See Orr-Weaver et al., supra. One or more expression construct may integrate, possibly affecting levels of recombinant protein produced (Rine et al. (1983) Proc. Natl. Acad. Sci. USA 80:6750). The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as ADE2, HIS4, LEU2, TRPI, and ALG7, and the G418 resistance gene, which confer resistance in yeast cells to tunicamycin and G418, respectively. In addition, a suitable selectable marker may also provide yeast with the ability to grow in the presence of toxic compounds, such as metal. For example, the presence of CUP1; allows yeast to grow in the presence of copper ions (Butt et al. (1987) Microbiol, Rev. 51:351), Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, inter alia, the following yeasts: *Candida albicans* (Kurtz, et al. (1986) Mol. Cell. Biol. 6:142), *Candida maltosa* (Kunze, et al. (1985) J. Basic Microbiol. 25:141), *Hansenula polymorpha* (Gleeson, et al. (1986) J. Gen. Microbiol. 132:3459; Roggenkamp et al. (1986) Mol. Gen. Genet. 202:302), *Kluyveromyces fragilis* (Das, et al. (1984) J. Bacteriol. 158:1165), *Kluyveromyces lactis* (De Louvencourt et al. (1983) J. Bacteriol. 154:737; Van den Berg et al. (1990) Biol Technology 8:135), *Pichia guillerimondii* (Kunze et al. (1985) J. Basic Microbiol. 25:141), *Pichia pastoris* (Cregg, et al. (1985) Mol. Cell. Biol. 5: 3376; US Patent Nos. 4,837,148 and 4,929, 555), *Saccharomyces cerevisiae* (Hinnen et al. (1978) Proc. Natl. Acad. Sci. USA 75:1929; Ito et al. (1983) J. Bacteriol. 153:163), *Schizosaccharomyces pombe* (Beach and Nurse (1981) Nature 300:706), and *Yarrowia lipolytica* (Davidow, et al. (1985) Curr. Genet. 10:39; Gaillardin, et al. (1985) Curr. Genet. 10:49).

Methods of introducing exogenous DNA into yeast hosts are well- known in the art, and usually include either the transformation of spheroplasts or of intact yeast cells treated with alkali cations. Transformation procedures usually vary with the yeast species to be transformed. See e.g., (Kurtz et al. (1986) Mol. Cell. Biol. 6:142; Kunze et al. (1985) J. Basic Microbiol. 25:141; Candida); (Gleeson et al. (1986) J. Gen. Microbiol. 132:3459; Roggenkamp et al. (1986) Mol. Gen. Genet. 202:302; Hansenula); (Das et al. (1984) J. Bacteriol. 158:1165; De Louvencourt et al. (1983) J. Bacteriol. 154:1165; Van den Berg et al. (1990) BiolTechnology 8:135; Kluyveromyces); (Cregg et al. (1985) Mol. Cell. Biol. 5: 3376; Kunze et al, (1985) J. Basic Microbiol. 25:141; US Patent Nos. 4,837,148 and 4,929,555; Pichia); (Hinnen et al. (1978) Proc. Natl. Acad. Sci. USA 75;1929; Ito et al. (1983) J. Bacteriol. 153:163; Saccharomyces); (Beach and Nurse (1981) Nature 300:706; Schizosaccharomyces); and (Davidow et al. (1985) Curr. Genet. 10:39; Gaillardin et al. (1985) Curr. Genet. 10:49; Yarrowia).

PURIFICATION OF THE CONFORMERS

The conformers of the present invention are preferably purified to at least about 80% purity, to at least about 90% purity, to at least about 95% purity, or greater than 95% purity with respect to contaminating macromolecules, particularly other proteins and nucleic acids, and free of infectious and pyrogenic agents. The conformers of the present invention may also be purified to a pharmaceutically pure state, which is greater than at least about 99.5% pure or preferably greater than at least about 99.9% pure. In certain embodiments, the purified or isolated conformers are substantially free of other conformers of the protein. Preferably, the purified or isolated conformer will have less than at least about 20% of other conformers, less than at least about 15% of other conformers, less than at least about 10% other conformers, less than at least about 5% other conformers, less than at least about 2% other conformers, or less than at least about 1% other conformers of the protein. In certain embodiments, it may not be necessary or desirable to remove all of the other conformers in which case the purified or isolated conformer may have between about 20% and about 1% other conformers, between about 15% and about 1% other conformers, between about 10% and about 1% other conformers, between about 5% and about 1% other conformers, or between about 2% and about 1% other conformers.

The bacterial adhesin protein or polypeptides thereof may be purified by any fractionation and/or purification methods available. See, e.g., Robert K. Scopes, "Protein Purification. Principles and Practice," (4th ed. 2000, Springer Verlag). In general, ammonium sulfate precipitation and acid or chaotrope extraction may be used for fractionation of samples. Exemplary purification steps may include hydroxyapatite, size exclusion, FPLC and reverse-phase high performance liquid chromatography. Suitable chromatographic media include derivatized dextrans, agarose, cellulose, polyacrylamide, specialty silicas, and the like. PEI, DEAE, QAE and Q derivatives are preferred examples of anion exchange. Exemplary chromatographic media include those media derivatized with phenyl, butyl, or octyl groups, such as Phenyl-Sepharose FF (Pharmacia), Toyopearl butyl 650 (Toso Haas, Montgomeryville, Pa.), Octyl-Sepharose (Pharmacia) and the like; or polyacrylic resins, such as Amberchrom CG 71 (Toso Haas) and the like. Suitable solid supports include glass beads, silica-based resins, cellulosic resins, agarose beads, cross-linked agarose beads, polystyrene beads, cross-linked polyacrylamide resins and the like that are insoluble under the conditions in which they are to be used. These supports may be modified with reactive groups that allow attachment of proteins by amino groups, carboxyl groups, sulfhydryl groups, hydroxyl groups and/or carbohydrate moieties.

Examples of coupling chemistries include cyanogen bromide activation, N-hydroxysuccinimide activation, epoxide activation, sulfhydryl activation, hydrazide activation, and carboxyl and amino derivatives for carbodiimide coupling chemistries. These and other solid media are well known and widely used in the art, and are available from commercial suppliers. Selection of a particular method for polypeptide isolation and purification is a matter of routine design and is determined in part by the properties of the chosen support. See, for example, Affinity Chromatography: Principles & Methods 18-1022-29 (2002), available from Amersham Biosciences, and Doonan, Protein Purification Protocols (The Humana Press 1996).

Additional variations in isolation and purification of the bacterial adhesin proteins or polypeptides thereof can be devised by those of skill in the art. For example, antibodies directed to the bacterial adhesin proteins can be used to

isolate large quantities of protein by immunoaffinity purification.

The polypeptides of the present invention can also be isolated by exploitation of particular properties. For example, immobilized metal ion adsorption (IMAC) chromatography can be used to purify histidine-rich proteins, including those comprising polyhistidine tags. Briefly, a gel is first charged with divalent metal ions to form a chelate (Sulkowski, Trends in Biochem. 3:1 (1985)). Histidine-rich proteins will be adsorbed to this matrix with differing affinities, depending upon the metal ion used, and will be eluted by competitive elution, lowering the pH, or use of strong chelating agents. Other methods of purification include purification of glycosylated proteins by lectin affinity chromatography and ion exchange chromatography (M. Deutscher, (ed.), Meth. Enzymol. 182:529 (1990)). Within additional embodiments of the invention, a fusion of the polypeptide of interest and an affinity tag (e.g., maltose-binding protein, an immunoglobulin domain) may be constructed to facilitate purification.

Bacterial adhesin proteins or especially polypeptides thereof may also be prepared through chemical synthesis. Bacterial adhesin proteins or polypeptides thereof may be monomers or multimers; glycosylated or non-glycosylated; PEGylated or non-PEGylated; and may or may not include an initial methionine amino acid residue.

*Separation of the conformers*

The bacterial adhesin conformers may be purified or isolated by any purification or separation technology that method that can differentiate the conformers based upon their differential biophysical characteristics. Preferred examples of such technologies are technologies that differentiate based upon differential frictional coefficients, differential charge distributions, differential affinity for particular cations or anions such as calcium or phosphate as found in hydroxyapatite, or differential presentation of surface antigens.

Preferred examples of technologies that can separate the bacterial adhesin conformers based upon differences in their frictional coefficients are size-exclusion chromatography, velocity sedimentation centrifugation, fast flow fractionation, and gel electrophoresis. The frictional coefficient of a molecule is based upon the mass and the shape of the molecule. A general theory developed to understand the transport of molecules in aqueous solution is called hydrodynamics. Stoke's law describes the relation between the friction coefficient $f_o$ and the viscosity $\eta$ of the medium:

$$f_o = 6\pi\eta R$$

Where R is the Stoke's radius of the molecule. For spherical macromolecules, the Stokes radius of the molecule is the radius of the spherical macromolecule plus its solvation shell. For macromolecules that are non-spherical, the Stokes radius is the radius of a spherical molecule that would have an equivalent friction coefficient. Non-spherical molecules will always have a higher frictional coefficient than that of a spherical molecule of the same molecular weight and solvation. Thus a molecule's deviation from a perfect spherical shape can be represented as $f/f_o$ where f is the actual frictional coefficient of the molecule and $f_o$ is the frictional coefficient of a spherical molecule with the same molecular weight and salvation. For spherical molecules, $f/f_o = 1$ and for non-spherical molecules, $f/f_o > 1$. Thus, conformers with different shapes and therefore different frictional coefficients may be separated based upon their different frictional coefficients as demonstrated in Example 2 below.

A preferred method of separation of bacterial adhesin conformers based upon their differential frictional coefficients is size-exclusion or gel filtration chromatography. One of skill in the art may readily select appropriate resins and buffers. Examples of suitable size-exclusion resins include, but are not limited to, Superdex 75, Superose 12, and Sephycryl 100. Any buffer conditions may be selected based upon conditions that suitably stabilize the bacterial adhesin conformer as long as the resin tolerates the buffer conditions. For general principles of size-exclusion chromatography including initial exploratory conditions, optimization, and scale-up, see "Gel Filtration: Principles and Methods," 18-1022-18 (2002), available from Amersham Biosciences.

Field flow fractionation (FFF) is another method that may be used to separate or purify the conformers based upon differential frictional coefficients. By way of example, but not limitation, sedimentation FFF may be used where the fractionation channel is spooled inside a centrifuge bowl. The spinning of the channel generates differential acceleration forces at right angles to flow. Retention time in sedimentation FFF depends on particles' dimension and density. Another example is flow FFF which has a broad range of applications. It can separate almost all macromolecules, colloid systems and particulate dispersions. In flow FFF, two crossed flow streams are superimposed on the same channel. The channel walls in flow FFF are permeable. The pore size of the membrane determines the lower size limit for the separation. The driving force in flow FFF is the viscous force exerted on a particle by the crossflow stream and separation is based on size alone, with retention times proportional to particles' diameter and shape.

Preparative gel electrophoresis may also be used to separate or purify the conformers based upon their differential frictional coefficients. Preferably the gel electrophoresis will be native, but denaturing conditions such as SDS may also be used given that the conformer F is resistant to denaturation by SDS as demonstrated in Example 2 below. Any suitable gel may be used, though agarose or acrylamide are preferred. Following electrophoresis, proteins may be recovered by passive diffusion or electroelution. In order to maintain the integrity of proteins during electrophoresis, it is important to keep the apparatus cool and minimize the effects of denaturation and proteolysis.

Another preferred method of separation or isolation of the conformers of bacterial adhesins is anion-exchange separation technology. Any of a large number of anion-exchange resins known in the art can be employed, including, for example, monoQ, Sepharose-Q, macro-prepQ, AG1-X2, HiQ, as well as DEAE-based resins. Elution can be achieved with aqueous solutions of salt including, without limitation, potassium chloride or sodium chloride at concentrations ranging from 0.01 M to 2.0 M over a wide range of pH. Alternatively elution may be achieved by alternate means such as pH gradients. For ion-exchange separation techniques, see generally "Ion Exchange Chromatography: Principles and Methods," 18-1114-21 (2002) available from Amersham Biosciences.

Yet another preferred separation technology for differentiating the conformers of bacterial adhesins is hydroxyapatite. Hydroxyapatite is a calcium phosphate based resin, as such it can function as both a cation-exchange resin and an anion-exchange resin. A wide range of cation-exchange resins may be used including by way of example sulfate based resins, carboxylate based resins, and phosphate based resins.

In addition, certain proteins behave differently on hydroxyapatite than on other cation- or anion-exchange resins owing to a higher affinity for either the calcium or the phosphate. By way of example, DNA binding proteins often bind more strongly to hydroxyapatite than to other cation-exchange resins owing to the DNA binding proteins having binding pockets for the phosphate backbone of DNA. Therefore in addition to hydroxyapatite, phosphate based resins such as phospho-cellulose may be used in the separation or isolation of bacterial adhesin conformers. Similarly immobilized divalent metal affinity separation technologies may be used where proteins have affinity for divalent metal ions such as calcium or magnesium.

Another example of a purification technology that can separate the bacterial conformers is antibody affinity, preferably monoclonal antibodies. As is demonstrated in the Examples below, the conformers of bacterial adhesins have different immunogenicities. The different immunogenicities is likely due in part to the conformers having different antigens exposed on their surface, which could include different accessibility of loops or different three dimensional surface structure. Thus antibodies can be isolated that are specific to one or a limited number of conformers. By way of example, antibodies may be generated by immunizing an animal with conformer F of a bacterial adhesin. Then polyclonal antibodies specific to conformer F can be purified by isolating antibodies from the sera of the animal and flowing the antibodies across conformer A that has been immobilized on a solid support. Antibodies that only recognize conformer F and not conformer A will not bind and therefore can be separated from the solid support. Alternatively, monoclonal antibody producing hybridoma could be generated from the animal and the monoclonal antibodies could be screened for their ability to bind to conformer F and not conformer A. Such antibodies that are specific to one conformer can be used to separate or isolate the conformer. Antibody affinity purification technologies are well known and readily available.

<u>SCREENING</u>

Another aspect of the present disclosure includes screening of the bacterial adhesin conformer F. Such screening may be performed for a wide range of purposes including by way of example selecting the more immunogenic conformers to maximize the immune response in the vaccine recipient, screening multi-component vaccine candidates for immune response to all of the components, screening immunogenic conformers for no or only limited side effects, and any other characteristic one of skill in the art may desire, non-limiting examples of which may be found throughout the specification.

The immunogenicity of the conformer F may be assayed by any method known to one skilled in the art. Typically, the presence (or absence), titers, affinities, avidities, etc. of antibodies generated in vivo are tested by standard methods, such as, but not limited to, ELISA assays, by which the immunogenicity or antigenicity are tested on immunoglobulin present in the serum of an organism (or patient). Additional methods, such as generating T-cell hybridomas and measuring activation in the presence of antigen presenting cells ("APCs") and antigen (Surman S et al., 2001 Proc. Natl. Acad. Sci. USA 98: 4587-92, below), examining labeled or unlabeled MHC presented peptides by chromatography, electrophoresis, and/or mass spectroscopy, T-cell activation assays, such as, but not limited to, T cell proliferation assays (Adorini L et al., 1988. J. Exp. Med. 168: 2091; So T. et al., 1996. Immunol. Let. 49: 91-97) and IL-2 production by proliferative response assays of CTLL-2 cells (Gillis S et al., 1978. J. Immunol. 120: 2027; So T. et al., 1996. Immunol. Let. 49: 91-97), and many others may be applied to determine more specific aspects of an immune response, or the lack thereof, such as, for example, the identity of the immunogenic T cell epitope of the antigen.

As non-limiting, specific examples, in vitro T cell assays may be carried out whereby the polypeptide, protein, or protein complex can be processed and presented in the groove of MHC molecules by appropriate antigen-presenting cells (APCs) to syngeneic T cells. T cell responses may be measured by simple proliferation measurements or by

measuring release of specific cytokine by activated cells; APCs may be irradiated or otherwise treated to prevent proliferation to facilitate interpretation of the results of such assays. In order to determine the immunogenicity of an epitope in the context of different MHC allotypes, in vivo assays using syngeneic APCs and T-cells of a range of allotypes may be carried out to test for T cell epitopes in a range of individuals or patients.

Alternatively, transgenic animals expressing MHC molecules from human (or any other species of interest) maybe used to assay for T cell epitopes; in a preferred embodiment this assay is carried out in transgenic animals in which the endogenous MHC repertoire has been knocked out and, better yet, in which one or more other accessory molecules of the endogenous MHC/T cell receptor complex have also been replaced with human molecules (or molecules of any other species of interest), such as, for example, the CD4 molecule.

Furthermore, to detect anti-protein/antigen/immunogenic polypeptide antibodies directly in vivo, for example in clinical and animal studies, ELISA assays, such as, for example solid phase indirect ELISA assays, may be used to detect binding of antibodies. In one specific embodiment, microtiter plates are incubated with the immunogenic polypeptide of interest at an appropriate concentration and in a suitable buffer. After washes with an appropriate washing solution, such as, for example PBS (pH 7.4), PBS containing 1% BSA and 0.05% Tween 20, or any other such solution as may be appropriate, serum samples are diluted, for example in PBS/BSA, and equal volumes of the samples are added in duplicate to the wells. The plates are incubated, and after additional washes, for example with PBS, anti-immunoglobulin antibodies coupled/conjugated to a reporter, such as a radioactive isotope or alkaline phosphatase, are added to each well at an appropriate concentration, and incubated. The wells are then washed again, and for example, in the case of use of alkaline phosphatase as a reporter, the enzyme reaction is carried our using a colorometric substrate, such as p-nitrophenyl phosphate in diethanolamine buffer (pH 9.8), absorbance of which can be read at 405 nm, for example, in an automatic ELISA reader (e.g. Multiskan PLUS; Labsystems).

As an additional non-limiting example, to detect antibodies in the serum of patients and animals, immunoblotting can also be applied. In one specific embodiment, an appropriate amount of the immunogenic polypeptide of interest per samples/lane is run on gels (e.g. polyacrylamide), under reducing and/or nonreducing conditions, and the polypeptide is transferred to a membrane, such as, for example, PVDF membranes; any other method to separate proteins by size can be used followed by transfer of the polypeptide to a membrane. The membranes are blocked, for example, using a solution of 5% (w/v) milk powder in PBS. In another embodiment, purified immunogenic polypeptide may be applied to the membrane. The blots are then incubated with serum samples at varying dilutions in the blocking solution (before and after injection regimen) and control anti-antigen, so far as such samples are available. The blots will be washed four times with an appropriate washing solution, and further incubated with reporter-conjugated anti-immunoglobulin at a appropriate/specified dilutions for appropriate/specified periods of time under appropriate/specified conditions. The blots are washed again with an appropriate washing solution, and the immunoreactive protein bands are visualized, for example, in the case of use of horseradish peroxidase-conjugated anti-immunoglobulin, using enhanced chemiluminescence reagents marketed by Amersham (Bucks, United Kingdom).

To test for a neutralizing effect of antibodies generated in vivo (patients or animals), a relevant biological activity of the pathogen of interest can, for example, be determined by using the bioassays, such, as for example, cell proliferation assays or host adhesion, in varying concentrations of serum of individuals or animals exposed/immunized with the immunogenic polypeptide of interest. Exponentially growing cells of the pathogen are washed and resuspended to a consistent and appropriate concentration in growth medium in a series of serial dilutions, and added in aiiquots to each well. For neutralization, a dilution series of serum before and after in vivo exposure (immunization) is added to the wells. The plates are incubated for an appropriate period of time (depending on the pathogen). The growth rate of the pathogen in each well is determined.

A preferred method of screening for immunogenicity is by the Active Maternal Immunization Assay. As discussed in Example 1, this assay may be used to measure serum titers of the female mice during the immunization schedule as well as the survival time of the pups after challenge. The skilled artisan can use the other methods of screening to determine antigenicity or immunogenicity of the immunogenic polypeptides of the present invention set forth in this specification and in the art for screening immunogenic polypeptides.

Methods of screening for antigenicity or immunogenicity may be used to select immunogenic polypeptides of interest from groups of two or more, three or more, five or more, ten or more, or fifty or more immunogenic polypeptides of the present invention based upon a criterion. One of skill in the art may apply any desired criterion in selecting the immunogenic polypeptide of interest. The criterion will depend upon the intended use of the immunogenic polypeptide of interest. By way of example, but not limitation, the criterion may be as simple as selecting the polypeptide with the highest antigenicity or immunogenicity. More complicated criterion may also be used such as selecting the polypeptide with the highest antigenicity or immunogenicity that produces no undesirable side effects upon immunization or selecting a multicomponent vaccine that includes the immunogenic polypeptide that has the highest antigenicity or immunogenicity against a panel of pathogens. Determination of the criterion is a simple matter of experimental design based upon the intended use and therefore one of skill in the art would have no difficulty in selecting appropriate criteria for any situation.

COMBINATIONS

The purified conformer F can be administered as a vaccine at appropriate levels, either by itself or in combination with other antigens such as non-adhesin proteins or polysaccharides

Other GBS Antigens

Another aspect of the present invention includes combination of the GBS80 conformer F, with other GBS antigens. Preferably, the combination of GBS antigens consists of three, four, five, six, seven, eight, nine, or ten GBS antigens. Still more preferably, the combination of GBS antigens consists of three, four, or five GBS antigens. Such combinations may include full length and/or antigenic fragments of the respective antigens and include combinations where the polypeptides and antigens are physically linked to one another and combinations where the polypeptides and antigens are not physically linked but are included in the same composition.

Preferably, the combinations of the invention provide for improved immunogenicity over the immunogenicity of the conformer when administered alone. Improved immunogenicity may be measured, for example, by the Active Maternal Immunization Assay. This assay may be used to measure serum titers of the female mice during the immunization schedule as well as the survival time of the pups after challenge. Preferably, immunization with the immunogenic compositions of the invention yield an increase of at least 2 percentage points (preferably at least 3, 4 or 5 percentage points) in the percent survival of the challenged pups as compared to the percent survival from maternal immunization with a single antigen of the composition when administered alone. Preferably, the increase is at least 6, 7, 8, 9, 10, 11, 12, 13, 14, 15, 16, 17, 18, 19, 20, 21, 22, 23, 24, 25, 26, 27, 28, 29 or 30 percentage points. Preferably, the GBS combinations of the invention comprising GBS 80 demonstrate an increase in the percent survival as compared to the percent survival from immunization with a non-GBS 80 antigen alone.

In one embodiment, the combination may consist of two to thirteen GBS antigens selected from an antigen group consisting of GBS 91, GBS 293, GBS 104, GBS 67, GBS 184, GBS 276, GBS 322, GBS 305, GBS 330, GBS 338, GBS 361, GBS 404, GBS 690, and GBS 691. Preferably, the combination includes GBS 80 conformer F in combination with one or more of GBS 104, GBS 59, GBS 67 and GBS 322. Polynucleotide and amino acid sequences for each of these GBS antigens and immunogenic fragments thereof are described in W004041157.

According to one embodiment of the invention, combinations of antigens or fusion proteins containing a portion or portions of the antigens will include GBS 80 or a portion thereof in combination with from one to 10 antigens, preferably one to 10 or less antigens. Examples of GBS antigens may be found in U.S. Serial Number 10/415,182, filed April 28, 2003, the International Applications (WO04/041157 and WO05/028618), and WO04/099242.

GBS polysaccharides

The compositions of the invention may be further improved by including GBS polysaccharides. Preferably, the GBS80 conformer F and the saccharide each contribute to the immunological response in a recipient. The combination is particularly advantageous where the saccharide and GBS80 conformer F provide protection from different GBS serotypes.

The combined antigens may be present as a simple combination where separate saccharide antigen and conformer are administered together, or they may be present as a conjugated combination, where the saccharide and polypeptide antigens are covalently linked to each other.

Thus the invention provides an immunogenic composition comprising (i) one or more GBS 80 conformer F and (ii) one or more GBS saccharide antigens. The polypeptide and the polysaccharide may advantageously be covalently linked to each other to form a conjugate.

In a further embodiment adhesins of the invention can be conjugated with one or more polysaccharides, such as those derived from GBS serotypes Ia, Ib, II, III, IV, V, VI, VII, and VIII.

Between them, the combined polypeptide and saccharide antigens preferably cover (or provide protection from) two or more GBS serotypes (*e.g.* 2, 3, 4, 5, 6, 7, 8 or more serotypes). The serotypes of the polypeptide and saccharide antigens may or may not overlap. For example, the polypeptide might protect against serogroup II or V, while the saccharide protects against either serogroups Ia, Ib, or III. Preferred combinations protect against the following groups of serotypes: (1) serotypes Ia and Ib, (2) serotypes Ia and II, (3) serotypes Ia and III, (4) serotypes Ia and IV, (5) serotypes Ia and V, (6) serotypes Ia and VI, (7) serotypes Ia and VII, (8) serotypes Ia and VIII, (9) serotypes Ib and II, (10) serotypes Ib and III, (11) serotypes Ib and IV, (12) serotypes Ib and V, (13) serotypes Ib and VI, (14) serotypes Ib and VII, (15) serotypes Ib and VIII, 16) serotypes II and m, (17) serotypes II and IV, (18) serotypes II and V; (19) serotypes II and VI, (20) serotypes II and III, (21) serotypes II and VII, (22) serotypes III and IV, (23) serotypes III and V, (24) serotypes III and VI, (25) serotypes III and VII, (26) serotypes III and VIII, (27) serotypes IV and V, (28) serotypes IV and VI, (29) serotypes IV and VII, (30) serotypes IV and VIII, (31) serotypes V and VI, (32) serotypes V and VII, (33) serotypes V

and VIII, (34) serotypes VI and VII, (35) serotypes VI and VIII, and (36) serotypes VII and VIII.

Still more preferably, the combinations protect against the following groups of serotypes: (1) serotypes Ia and II, (2) serotypes Ia and V, (3) serotypes Ib and II, (4) serotypes Ib and V, (5) serotypes III and II, and (6) serotypes III and V. Most preferably, the combinations protect against serotypes III and V. Protection against serotypes II and V is preferably provided by polypeptide antigens.

Protection against serotypes Ia, Ib and/or III may be polypeptide or saccharide antigens.

In one embodiment, the conformer F immunogenic composition comprises a GBS saccharide antigen and at least two GBS polypeptide antigens or fragments thereof, wherein said GBS saccharide antigen comprises a saccharide selected from GBS serotype Ia, Ib, and III, and wherein said GBS polypeptide antigens comprise a combination of at least two polypeptide or a fragment thereof selected from the antigen group consisting of GBS 80 (gi:2253618), GBS 67 (gi22537555), SAN1518 (Spb1, gi:77408651), GBS 104 and GBS 322 (the foregoing antigens are described in U.S. Patent App. No. 11/192,046 ). Preferably, the combination includes GBS 80 or a fragment thereof.

## Further antigens

The compositions of the invention may further comprise one or more additional antigens, including additional bacterial, viral or parasitic antigens.

In another embodiment, the GB680 conformer F's of the invention are combined with one or more additional, antigens suitable for use in a vaccine designed to protect elderly or immunocomprised individuals. For example, the conformer F may be combined with an antigen derived from the group consisting of *Enterococcus faecalis, Staphylococcus aureus, Staphylococcus epidermis, Pseudomonas aeruginosa; Legionella pneumophila, Listeia monocytogenes, Neisseria meningitides,* influenza, and Parainfluenza virus ('PIV').

Where a saccharide or carbohydrate antigen is used, it is preferably conjugated to a carrier protein in order to enhance immunogenicity (*e.g.* Ramsay et al. (2001) Lancet 357(9251): 195-196; Lindberg (1999) Vaccine 17 Suppl 2: S28-36; Buttery & Moxon (2000) J R Coll Physicians Lond 34:163-168; Ahmad & Chapnick (1999) Infect Dis Clin North Am 13: 113 133, vii; Goldblatt (1998) J. Med. Microbiol. 47:563-567; EP-0 477 508; US 5,306,492; WO98/42721; Conjugate Vaccines (eds. Cruse et al.) ISBN 3805549326, particularly vol. 10:48-114; Hermanson (1996) Bioconjugate Techniques ISBN: 0123423368 or 012342335X). Preferred carrier proteins are bacterial toxins or toxoids, such as diphtheria or tetanus toxoids. The CRM97 diphtheria toxoid is particularly preferred (Research Disclosure, 453077 (Jan 2002)). Other carrier polypeptides include the *N.meningitidis* outer membrane protein (EP-A-0372501), synthetic peptides (EP-A-0378881; EP-A-0427347), heat shock proteins (WO93/17712; WO94/03208), pertussis proteins (WO98/58668; EP-A-0471177), protein D from *H.influenzae* (WO00/56360), cytokines (WO91/01146), lymphokines, hormones, growth factors, toxin A or B from *C.difficile* (WO00/61761), iron uptake proteins (WO01/72337), *etc.* Where a mixture comprises capsular saccharides from both serogroups A and C, it may be preferred that the ratio (w/w) of MenA saccharide:MenC saccharine is greater than 1 (*e.g.* 2:1, 3:1, 4:1, 5:1, 10:1 or higher). Different saccharides can be conjugated to the same or different type of carrier protein. Any suitable conjugation reaction can be used, with any suitable linker where necessary.

Toxic protein antigens may be detoxified where necessary *e.g.* detoxification of pertussis toxin by chemical and/or genetic means.

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Antigens in the composition will typically be present at a concentration of at least 1 μg/ml each. In general, the concentration of any given antigen will be sufficient to elicit an immune response against that antigen.

As an alternative to using protein antigens in the composition of the invention, nucleic acid encoding the antigen may be used (*e.g.* Robinson & Torres (1997) Seminars in Immunology 9:271-283; Donnelly et al. (1997) Annu Rev Immunol 15:617-648; Scott-Taylor & Dalgleish (2000) Expert Opin Investig Drugs 9:471-480; Apostolopoulos & Plebanski (2000) Curr Opin Mol. Ther 2:441-447; Ilan (1999) Curr Opin Mol. Ther 1:116-120; Dubensky et al. (2000) Mol. Med 6: 723-732; Robinson & Pertmer (2000) Adv Virus Res 55: 1-74; Donnelly et al. (2000) Am J Respir Crit Care Med 162(4 Pt 2):S190-193; Davis (1999) Mt. Sinai J. Med. 66:84-90). Protein components of the compositions of the invention may thus be replaced by nucleic acid (preferably DNA *e.g.* in the form ofa plasmid) that encodes the protein.

## VACCINES

Vaccines according to the invention may either be prophylactic (i.e., to prevent infection) or therapeutic (i.e., to treat disease after infection).

Such vaccines comprise GBS80 conformer F, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the bacterial adhesin conformer F may be conjugated to a bacterial toxoid, such as a toxoid from such pathogens as diphtheria, tetanus, cholera, H. pylori, etc.

Compositions such as vaccines and pharmaceutical compositions of the invention may advantageously include an adjuvant, which can function to enhance the immune responses (humoral and/or cellular) elicited in a patient who receives the composition.

Adjuvants that can be used with the invention include, but are not limited to:

- A mineral containing composition, including calcium salts and aluminum salts (or mixtures thereof). Calcium salts include calcium phosphate (e.g. the "CAP" particles disclosed in US 6,355,271 ). Aluminum salts include hydroxides, phosphates, sulfates, etc., with the salts taking any suitable form (e.g. gel, crystalline, amorphous, etc.). Adsorption to these salts is preferred. The mineral containing compositions may also be formulated as a particle of metal salt (WO00/23105). Aluminum salt adjuvants are described in more detail below.

- Cytokine inducing agents (see in more detail below).

- Saponins (chapter 22 of Vaccine Design: The Subunit and Adjuvant Approach (eds. Powell & Newman) Plenum Press 1995 (ISBN 0-306-44867-X)), which are a heterologous group of sterol glycosides and triterpenoid glycosides that are found in the bark, leaves, stems, roots and even flowers of a wide range of plant species. Saponin from the bark of the Quillaia saponaria Molina tree have been widely studied as adjuvants. Saponin can also be commercially obtained from Smilax ornata (sarsaparilla), Gypsophilla paniculata (brides veil), and Saponaria officianalis (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in US 5,057,540 ). It is possible to use fraction A of Quil A together with at least one other adjuvant (WO05/02620). Saponin formulations may also comprise a sterol, such as cholesterol (WO96/33739). Combinations of saponins and cholesterols can be used to form unique particles called immunostimulating complexes (ISCOMs) (chapter 23 of Vaccine Design: The Subunit and Adjuvant Approach (eds. Powell & Newman) Plenum Press 1995 (ISBN 0-306-44867-X)). ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in EP-A-0109942, US 4,578,269, WO96/11711, and US 6,352,697 . Optionally, the ISCOMS may be devoid of additional detergent (WO00/07621; US 6,506,386). It is possible to use a mixture of at least two ISCOM complexes, each complex comprising essentially one saponin fraction, where the complexes are ISCOM complexes or ISCOM matrix complexes (WO04/04762). A review of the development of saponin based adjuvants can be found in Barr et al. (1998) Advanced Drug Delivery Reviews 32:247-271 and Sjolanderet et al (1998) Advanced Drug Delivery Reviews 32:321-338.

- Fatty adjuvants (see in more detail below).

- Bacterial ADP-ribosylating toxins (e.g. the *E. coli* heat labile enterotoxin "LT", cholera toxin "CT", or pertussis toxin "PT") and detoxified derivatives thereof, such as the mutant toxins known as LT-K63 and LT R72 (Pizza et al. (2000) Int J Med Microbiol 290:455-461). The use of detoxified ADP-ribosylating toxins as mucosal adjuvants is described in WO95/17211 and as parenteral adjuvants in WO98/42375.

- Bioadhesives and mucoadhesives, such as esterified hyaluronic acid microspheres (Singh et al. (2001) J Cont Release 70:267-276) or chitosan and its derivatives (WO99/27960).

- Microparticles (i.e. a particle of ~100nm to ~150μm in diameter, more preferably ~200nm to ~30pm in diameter, or -500nm to ~10μm in diameter) formed from materials that are biodegradable and non toxic (e.g. a poly(α-hydroxy acid), a polyhydroxybutyric acid, a polyorthoester, a polyanhydride, a polycaprolactone, etc.), with poly(lactide co glycolide) being preferred, optionally treated to have a negatively-charged surface (e.g. with SDS) or a positively-charged surface (e.g. with a cationic detergent, such as CTAB).

- Liposomes (Chapters 13 & 14 of Vaccine Design: The Subunit and Adjuvant Approach (eds. Powell & Newman) Plenum Press 1995 (ISBN 0-306-44867-X)). Examples of liposome formulations suitable for use as adjuvants are

described in US 6,090,406, US 5,916,588, and EP-A-0626169.

- Oil in water emulsions (see in more detail below).

- Polyoxyethylene ethers and polyoxyethylene esters (WO99/52549). Such formulations • further include polyoxyethylene sorbitan ester surfactants in combination with an octoxynol (WO01/21207) as well as polyoxyethylene alkyl ethers or ester surfactants in combination with at least one additional non-ionic surfactant such as an octoxynol (WO01/21152). Preferred polyoxyethylene ethers are selected from the following group: polyoxyethylene-9-lauryl ether (laureth 9), polyoxyethylene-9-steoryl ether, polyoxytheylene-8-steoryl ether, polyoxyethylene-4-lauryl ether, polyoxyethylene-35-lauryl ether, and polyoxyethylene-23-lauryl ether.

- Muramyl peptides, such as N-acetylmuramyl-L-threonyl-D-isoglutamine ("thr-MDP"), N acetyl-normuramyl-L-alanyl-D-isoglutamine (nor-MDP), N-acetylglucsaminyl-N-acetylmuramyl-L-Al-D-isoglu-L-Ala-dipaimitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").

- An outer membrane protein proteosome preparation prepared from a first Gram-negative bacterium in combination with a liposaccharide preparation derived from a second Gram negative bacterium, wherein the outer membrane protein proteosome and liposaccharide preparations form a stable non-covalent adjuvant complex. Such complexes include "IVX-908", a complex comprised of Neisseria meningitidis outer membrane and lipopolysaccharides. They have been used as adjuvants for influenza vaccines (WO02/72012).

- Methyl inosine 5'-monophosphate ("MIMP") (Signorelli and Hadden (2003) Int Immunopharmacol 3(8):1177-86).

- A polyhydroxlated pyrrolizidine compound (WO04/64715), such as one having formula:

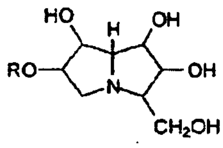

  where R is selected from the group comprising hydrogen, straight or branched, unsubstituted or substituted, saturated or unsaturated acyl, alkyl (e.g. cycloalkyl), alkenyl, alkynyl and aryl groups, or a pharmaceutically acceptable salt or derivative thereof. Examples include, but are not limited to: casuarine, casuarine-6-α-D-glucopyranose, 3 epi casuarine, 7 epi casuarine, 3,7 diepi casuarine, etc.

- A gamma inulin (Cooper (1995) Pharm Biotechnol 6:559-80) or derivative thereof, such as algammulin.

- A CD1d ligand, such as a α glycosylceramide e.g. α-galactosylceramide.

These and other adjuvant active substances are discussed in more detail in Vaccine Design: The Subunit and Adjuvant Approach (eds. Powell & Newman) Plenum Press 1995 (ISBN 0-306-44867-X) & Vaccine Adjuvants: Preparation Methods and Research Protocols (Volume 42 of Methods in Molecular Methods series). ISBN: 1-59259-083-7. Ed. O'Hagan.

Compositions may include two or more of said adjuvants. For example, they may advantageously include both an oil in water emulsion and a cytokine inducing agent, as this combination improves the cytokine responses elicited by influenza vaccines, such as the interferon γ response, with the improvement being much greater than seen when either the emulsion or the agent is used on its own.

Antigens and adjuvants in a composition will typically be in admixture.

Where a vaccine includes an adjuvant, it may be prepared extemporaneously, at the time of delivery. Thus the invention provides kits including the antigen and adjuvant components ready for mixing. The kit allows the adjuvant and the antigen to be kept separately until the time of use. The components are physically separate from each other within the kit, and this separation can be achieved in various ways. For instance, the two components may be in two separate containers, such as vials. The contents of the two vials can then be mixed e.g. by removing the contents of one vial and adding them to the other vial, or by separately removing the contents of both vials and mixing them in a third container. In a preferred arrangement, one of the kit components is in a syringe and the other is in a container such as a vial. The

syringe can be used (e.g. with a needle) to insert its contents into the second container for mixing, and the mixture can then be withdrawn into the syringe. The mixed contents of the syringe can then be administered to a patient, typically through a new sterile needle. Packing one component in a syringe eliminates the need for using a separate syringe for patient administration. In another preferred arrangement, the two kit components are held together but separately in the same syringe e.g. a dual chamber syringe, such as those disclosed in WO05/89837, US 6,692,468, WO00/07647, WO99/17820, US 5,971,953, 4,060,082, EP-A-0520618, and WO98/01174. When the syringe is actuated (e.g. during administration to a patient) then the contents of the two chambers are mixed. This arrangement avoids the need for a separate mixing step at the time of use.

*Oil in water emulsion adjuvants*

Oil in water emulsions have been found to be particularly suitable for use in adjuvanting influenza virus vaccines. Various such emulsions are known, and they typically include at least one oil and at least one surfactant, with the oil(s) and surfactant(s) being biodegradable (metabolisable) and biocompatible. The oil droplets in the emulsion are generally less than 5μm in diameter, and may even have a sub micron diameter, with these small sizes being achieved with a microfluidiser to provide stable emulsions. Droplets with a size less than 220 nm are preferred as they can be subjected to filter sterilization.

The invention can be used with oils such as those from an animal (such as fish) or vegetable source. Sources for vegetable oils include nuts, seeds and grains. Peanut oil, soybean oil, coconut oil, and olive oil, the most commonly available, exemplify the nut oils. Jojoba oil can be used e.g. obtained from the jojoba bean. Seed oils include safflower oil, cottonseed oil, sunflower seed oil, sesame seed oil and the like. In the grain group, corn oil is the most readily available, but the oil of other cereal grains such as wheat, oats, rye, rice, teff, triticale and the like may also be used. 6-10 carbon fatty acid esters of glycerol and 1,2-propanediol, while not occurring naturally in seed oils, may be prepared by hydrolysis, separation and esterification of the appropriate materials starting from the nut and seed oils. Fats and oils from mammalian milk are metabolizable and may therefore be used in the practice of this invention. The procedures for separation, purification, saponification and other means necessary for obtaining pure oils from animal sources are well known in the art. Most fish contain metabolizable oils which may be readily recovered. For example, cod liver oil, shark liver oils, and whale oil such as spermaceti exemplify several of the fish oils which may be used herein. A number of branched chain oils are synthesized biochemically in 5-carbon isoprene units and are generally referred to as terpenoids. Shark liver oil contains a branched, unsaturated terpenoids known as squalene, 2,6,10,15,19,23-hexamethyl-2,6,10,14,18,22-tetracosahexaene, which is particularly preferred herein. Squalane, the saturated analog to squalene, is also a preferred oil. Fish oils, including squalene and squalane, are readily available from commercial sources or may be obtained by methods known in the art. Other preferred oils are the tocopherols (see below). Mixtures of oils can be used.

Surfactants can be classified by their 'HLB' (hydrophile/lipophile balance). Preferred surfactants of the invention have a HLB of at least 10, preferably at least 15, and more preferably at least 16. The invention can be used with surfactants including, but not limited to: the polyoxyethylene sorbitan esters surfactants (commonly referred to as the Tweens), especially polysorbate 20 and polysorbate 80; copolymers of ethylene oxide (EO), propylene oxide (PO), and/or butylene oxide (BO), sold under the DOWFAX™ tradename, such as linear EO/PO block copolymers; octoxynols, which can vary in the number of repeating ethoxy (oxy-1,2-ethanediyl) groups, with octoxynol-9 (Triton X 100, or t octylphenoxypolyethoxyethanol) being of particular interest; (octylphenoxy) polyethoxyethanol (IGEPAL CA-630/NP-40); phospholipids such as phosphatidylcholine (lecithin); polyoxyethylene fatty ethers derived from lauryl, cetyl, stearyl and oleyl alcohols (known as Brij surfactants), such as triethyleneglycol monolauryl ether (Brij 30); and sorbitan esters (commonly known as the SPANs), such as sorbitan trioleate (Span 85) and sorbitan monolaurate. Preferred surfactants for including in the emulsion are Tween 80 (polyoxyethylene sorbitan monooleate), Span 85 (sorbitan trioleate), lecithin and Triton X 100. Mixtures of surfactants can be used e.g. Tween 80/Span 85 mixtures.

Specific oil in water emulsion adjuvants useful with the invention include, but are not limited to:

- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' (WO90/14837; Podda and Del Giudice (2003) Expert Rev Vaccines 2:197-203; Podda (2001) Vacccine 19:2673-2680), as described in more detail in Chapter 10 of Vaccine Design: The Subunit and Adjuvant Approach (eds. Powell & Newman) Plenum Press 1995 (ISBN 0-306-44867-X) and chapter 12 of Vaccine Adjuvants: Preparation Methods and Research Protocols (Volume 42 of Methods in Molecular Methods series). ISBN: 1-59259-083-7. Ed. O'Hagan. The MF59 emulsion advantageously includes citrate ions e.g. 10mM sodium citrate buffer.

- An emulsion of squalene, a tocopherol, and Tween 80. The emulsion may include phosphate buffered saline. It may also include Span 85 (e.g. at 1%) and/or lecithin. These emulsions may have from 2 to 10% squalene, from 2 to

10% tocopherol and from 0.3 to 3% Tween 80, and the weight ratio of squalene:tocopherol is preferably <1 as this provides a more stable emulsion. One such emulsion can be made by dissolving Tween 80 in PBS to give a 2% solution, then mixing 90ml of this solution with a mixture of (5g of DL α tocopherol and 5ml squalene), then microfluidising the mixture. The resulting emulsion may have submicron oil droplets e.g. with an average diameter of between 100 and 250nm, preferably about 180nm.

- An emulsion of squalene, a tocopherol, and a Triton detergent (e.g. Triton X-100).

- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl MDP in the "SAF 1" adjuvant (Allison and Byars (1992) Res Immunol 143:519-25) (0.05-1% Thr MDP, 5% squalane, 2.5% Pluronic L121 and 0.2% polysorbate 80). It can also be used without the Thr MDP, as in the "AF" adjuvant (Hariharan et al. (1995) Cancer Res 55:3486-9) (5% squalane, 1.25% Pluronic L121 and 0.2% polysorbate 80). Microfluidisation is preferred.

- An emulsion having from 0.5 50% of an oil, 0.1 10% of a phospholipid, and 0.05 5% of a non ionic surfactant. As described in reference WO95/11700 preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.

- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in US 6,080,725, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.

- An emulsion in which a saponin (e.g. QuilA or QS21) and a sterol (e.g. a cholesterol) are associated as helical micelles (WO05/097181).

The emulsions may be mixed with antigen extemporaneously, at the time of delivery. Thus the adjuvant and antigen may be kept separately in a packaged or distributed vaccine, ready for final formulation at the time of use. The antigen will generally be in an aqueous form, such that the vaccine is finally prepared by mixing two liquids. The volume ratio of the two liquids for mixing can vary (e.g. between 5:1 and 1:5) but is generally about 1:1.

After the antigen and adjuvant have been mixed, the antigen will generally remain in aqueous solution but may distribute itself around the oil/water interface. In general, little if any antigen will enter the oil phase of the emulsion.

Where a composition includes a tocopherol, any of the α, β, γ, δ, ε or ζ tocopherols can be used, but α tocopherols are preferred. The tocopherol can take several forms e.g. different salts and/or isomers. Salts include organic salts, such as succinate, acetate, nicotinate, etc. D α tocopherol and DL α tocopherol can both be used. Tocopherols are advantageously included in vaccines for use in elderly patients (e.g. aged 60 years or older) because vitamin E has been reported to have a positive effect on the immune response in this patient group (Han et al. (2005) Impact of Vitamin E on Immune Function and Infectious Diseases in the Aged at Nutrition, Immune functions and Health EuroConference, Paris, 9-10 June 2005). They also have antioxidant properties that may help to stabilize the emulsions (US 6,630,161). A preferred α tocopherol is DL α tocopherol, and the preferred salt of this tocopherol is the succinate. The succinate salt has been found to cooperate with TNF related ligands in vivo. Moreover, α tocopherol succinate is known to be compatible with vaccines (for example, influenza vaccines) and to be a useful preservative as an alternative to mercurial compounds (WO02/097072).

*Cytokine-inducing agents*

Cytokine inducing agents for inclusion in compositions of the invention are able, when administered to a patient, to elicit the immune system to release cytokines, including interferons and interleukins. Cytokine responses are known to be involved in the early and decisive stages of host defense against pathogen infection (Hayden et al. (1998) J Clin Invest 101 (3):643-9). Preferred agents can elicit the release of one or more of: interferon γ; interleukin 1; interleukin 2; interleukin 12; TNF α; TNF β; and GM CSF. Preferred agents elicit the release of cytokines associated with a Th1-type immune response e.g. interferon γ, TNF α, interleukin 2. Stimulation of both interferon γ and interleukin 2 is preferred.

As a result of receiving a composition of the invention, therefore, a patient will have T cells that, when stimulated with an antigen, will release the desired cytokine(s) in an antigen specific manner. For example, T cells purified form their blood will release γ interferon when exposed in vitro to the stimulated antigen. Methods for measuring such responses

in peripheral blood mononuclear cells (PBMC) are known in the art, and include ELISA, ELISPOT, flow cytometry and real time PCR. For example, Tassignon et al. (2005) J Immunol Meth 305:188-98 reports a study in which antigen specific T cell-mediated immune responses against tetanus toxoid, specifically γ interferon responses, were monitored, and found that ELISPOT was the most sensitive method to discriminate antigen specific TT-induced responses from spontaneous responses, but that intracytoplasmic cytokine detection by flow cytometry was the most efficient method to detect re stimulating effects.

Suitable cytokine inducing agents include, but are not limited to:

- An immunostimulatory oligonucleotide, such as one containing a CpG motif (a dinucleotide sequence containing an unmethylated cytosine linked by a phosphate bond to a guanosine), or a double stranded RNA, or an oligonucleotide containing a palindromic sequence, or an oligonucleotide containing a poly(dG) sequence.

- 3 O deacylated monophosphoryl lipid A ('33dMPL', also known as 'MPL™') (Myers et al. (1990) pages 145-156 of Cellular and molecular aspects of endotoxin reactions; Ulrich (2000) Chapter 16 (pages 273-282) of Vaccine Adjuvants: Preparation Methods and Research Protocols (Volume 42 of Methods in Molecular Methods series). ISBN: 1-59259-083-7. Ed. O'Hagan; Johnson et al. (1999) J Med Chem 42:4640-9; Baldrick et al. (2002) Regulatory Toxicol Pharmacol 35:398-413).

- An imidazoquinoline compound, such as Imiquimod ("R 837") (US 4,680,338; US 4,988,815), Resiquimod ("R 848") (WO92/15582), and their analogs; and salts thereof (e.g. the hydrochloride salts). Further details about immunostimulatory imidazoquinolines can be found in Stanley (2002) Clin Exp Dermatol 27:571-577, Wu et al. (2004) Antiviral Res. 64(2):79-83, Vasilakos et al. (2000) Cell Immunol. 204(1):64-74, US 4,689,338, US 4,929,624, US 5,238,944, US 5,266,575, US 5,268,376, US 5,346,905, US 5,352,784, US 5,389,640, US 5,395,937, US 5,482,936, US 5,494,916, US 5,525,612, US 6,083,505, US 6,440,992, US 6,627,640, US 6,656,938, US 6,660,735, US 6,660,747, US 6,664,260, US 6,664,264, US 6,664,265, US 6,667,312, US 6,670,372, US 6,677,347, US 6,677,348, US 6,677,349, US 6,683,088, US 6,703,402, US 6,743,920, US 6,800,624, US 6,809,203, US 6,888,000, US 6,924,293, and Jones (2003) Curr Opin Investig Drugs 4:214-218.

- A thiosemicarbazone compound, such as those disclosed in WO2004/060308. Methods of formulating, manufacturing, and screening for active compounds are also described in WO2004/060308. The thiosemicarbazones are particularly effective in the stimulation of human peripheral blood mononuclear cells for the production of cytokines, such as TNF-α.

- A tryptanthrin compound, such as those disclosed in WO2004/064759. Methods of formulating, manufacturing, and screening for active compounds are also described in WO2004/064759. The thiosemicarbazones are particularly effective in the stimulation of human peripheral blood mononuclear cells for the production of cytokines, such as TNF-α.

- A nucleoside analog, such as: (a) Isatorabine (ANA-245; 7-thia-8-oxoguanosine):

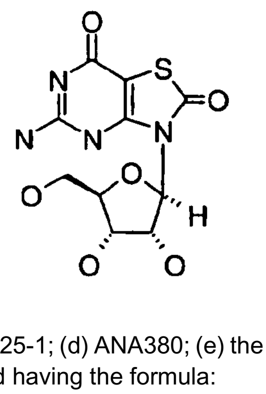

and prodrugs thereof; (b) ANA975; (c) ANA-025-1; (d) ANA380; (e) the compounds disclosed in US 6,924,271, US 2005/0070556, and US 5,658,731; (f) a compound having the formula:

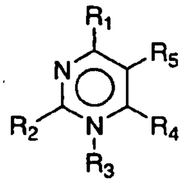

wherein:

R1 and R2 are each independently H, halo, -NRaRb, -OH, C1-6 alkoxy, substituted C1-6 alkoxy, heterocyclyl, substituted heterocyclyl, C6-10 aryl, substituted C6-10 aryl, C1-6 alkyl, or substituted C1-6 alkyl;

R3 is absent, H, C1-6 alkyl, substituted C1-6 alkyl, C6-10 aryl, substituted C6-10 aryl, heterocyclyl, or substituted heterocyclyl;

R4 and R5 are each independently H, halo, heterocyclyl, substituted heterocyclyl, C(O)-Rd, C1-6 alkyl, substituted C1-6 alkyl, or bound together to form a 5 membered ring as in R4-5:

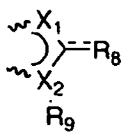

$R_{4\text{-}5}$

the binding being achieved at the bonds indicated by a

X1 and X2 are each independently N, C, O, or S;

R8 is H, halo, -OH, C1-6 alkyl, C2-6 alkenyl, C2-6 alkynyl, -OH, -NRaRb, -(CH2)n-O-Rc, -O-(C1-6 alkyl), -S(O)pRe, or -C(O)-Rd;

R9 is H, C1-6 alkyl, substituted C1-6 alkyl, heterocyclyl, substituted heterocyclyl or R9a, wherein R9a is:

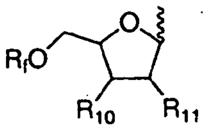

R9a

the binding being achieved at the bond indicated by a

R10 and R11 are each independently H, halo, C1-6 alkoxy, substituted C1-6 alkoxy, -NRaRb, or -OH;

each Ra and Rb is independently H, C1-6 alkyl, substituted C1-6 alkyl, -C(O)Rd, C6-10 aryl;

each Rc is independently H, phosphate, diphosphate, triphosphate, C1-6 alkyl, or substituted C1-6 alkyl;

each Rd is independently H, halo, C1-6 alkyl, substituted C1-6 alkyl, C1-6 alkoxy, substituted C1-6 alkoxy, -NH2, -NH(C1-6 alkyl), -NH(substituted C1-6 alkyl), -N(C1-6 alkyl)2,-N(substituted C1-6 alkyl)2, C6-10 aryl, or heterocyclyl;

each Re is independently H, C1-6 alkyl, substituted C1-6 alkyl, C6-10 aryl, substituted C6-10 aryl, heterocyclyl, or substituted heterocyclyl;

each Rf is independently H, C1-6 alkyl, substituted C1-6 alkyl, -C(O)Rd, phosphate, diphosphate, or triphosphate;

each n is independently 0, 1, 2, or 3;

each p is independently 0, 1, or 2; or

or (g) a pharmaceutically acceptable salt of any of (a) to (f), a tautomer of any of (a) to (f), or a pharmaceutically acceptable salt of the tautomer.

- Loxoribine (7-allyl-8-oxoguanosine) (US 5,011,828).

- Compounds disclosed in WO2004/87153, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds (US 6,605,617; WO02/18383), Hydrapthalamide compounds, Benzophenone compounds, Isoxazole compounds, Sterol compounds, Quinazilinone compounds, Pyrrole compounds (WO20041018455), Anthraquinone compounds, Quinoxaline compounds, Triazine compounds, Pyrazalopyrimidine compounds, and Benzazole compounds (WO03/082272).

- Compounds disclosed in PCT/US2005/022769.

- An aminoalkyl glucosaminide phosphate derivative, such as RC 529 (Johnson et al. (1999) Bioorg Med Chem Lett 9:2273-2278; Evans et al. (2003) Expert Rev Vaccines 2:219-229).

- A phosphazene, such as poly(di(carboxylatophenoxy)phosphazene) ("PCPP") as described, for example, in Andrianov et al. (1998) Biomaterials 19:109-115 and Payne et al. (1998) Adv Drug Delivery Review 31:185-196.

- Small molecule immunopotentiators (SMIPs) such as:

N2-methyl-1-(2-methylpropyl)-1H-imidazo(4,5-c)quinoline-2,4-diamine

N2,N2-dimethyl-1-(2-methylpropyl)-1H-imidazo(4,5-c)quinoline-2,4-diamine

N2-ethyl-N2-methyl-1-(2-methylpropyl)-1H-imidazo(4,5-c)quinoline-2,4-diamine

N2-methyl-1-(2-methylpropyl)-N2-propyl-1H-imidazo(4,5-c)quinoline-2,4-diamine

1-(2-methylpropyl)-N2-propyl-1H-imidazo(4,5-c)quinoline-2,4-diamine

N2-butyl-1-(2-methylpropyl)-1H-imidazo(4,5-c)quinoline-2,4-diamine

N2-butyl-N2-methyl-1-(2-methylpropyl)-1H-imidazo(4,5-c)quinoline-2,4-diamine

N2-methyl-1-(2-methylpropyl)-N2-pentyl-1H-imidazo(4,5-c)quinoline-2,4-diamine

N2-methyl-1-(2-methylpropyl)-N2-prop-2-enyl-1H-imidazo(4,5-c)quinoline-2,4-diamine

1-(2-methylpropyl)-2-((phenylmethyl)thio)-1H-imidazo(4,5-c)quinolin-4-amine

1-(2-methylpropyl)-2-(propylthio)-1H-imidazo(4,5-c)quinolin-4-amine

2-((4-amino-1-(2-methylpropyl)-1H-imidazo(4,5-c)quinolin-2-yl)(methyl)amino)ethanol

2-((4-amino-1-(2-methylpropyl)-1H-imidazo(4,5-c)quinolin-2-yl)(methyl)amino)ethyl acetate

4-amino-1-(2-methylpropyl)-1,3-dihydro-2H-imidazo(4,5-c)quinolin-2-one

N2-butyl-1-(2-methylpropyl)-N4,N4-bis(phenylmethyl)-1H-imidazo(4,5-c)quinoline-2,4-diamine

N2-butyl-N2-methyl-1-(2-methylpropyl)-N4,N4-bis(phenylmethyl)-1H-imidazo(4,5-c)quinoline-2,4-diamine

N2-methyl-1-(2-methylpropyl)-N4,N4-bis(phenylmethyl)-1H-imidazo(4,5-c)quinoline-2,4-diamine

N2,N2-dimethyl-1-(2-methylpropyl)-N4,N4-bis(phenylmethyl)-1H-imidazo(4,5-c)quinoline-2,4-diamine

1-{4-amino-2-(methyl(propyl)amino)-1H-imidazo(4,5-c)quinolin-1-yl}-2-methylpropan-2-ol

1-(4-amino-2-(propylamino)-1H-imidazo(4,5-c)quinolin-1-yl)-2-methylpropan-2-ol

N4,N4-dibenzyl-1-(2-methoxy-2-methylpropyl)-N2-propyl-1H-imidazo(4,5-c)quinoline-2,4-diamine.

The cytokine inducing agents for use in the present invention may be modulators and/or agonists of Toll-Like Receptors (TLR). For example, they may be agonists of one or more of the human TLR1, TLR2, TLR3, TLR4, TLR7, TLR8, and/or TLR9 proteins. Preferred agents are agonists of TLR7 (e.g. imidazoquinolines) and/or TLR9 (e.g. CpG oligonucleotides). These agents are useful for activating innate immunity pathways.

The cytokine inducing agent can be added to the composition at various stages during its production. For example, it may be within an antigen composition, and this mixture can then be added to an oil in water emulsion. As an alternative, it may be within an oil in water emulsion, in which case the agent can either be added to the emulsion components before emulsification, or it can be added to the emulsion after emulsification. Similarly, the agent may be coacervated within the emulsion droplets. The location and distribution of the cytokine inducing agent within the final composition will depend on its hydrophilic/lipophilic properties e.g. the agent can be located in the aqueous phase, in the oil phase, and/or at the oil water interface.

The cytokine inducing agent can be conjugated to a separate agent, such as an antigen (e.g. CRM197). A general review of conjugation techniques for small molecules is provided in Thompson et al. (2003) Methods in Molecular Medicine 94:255-266. As an alternative, the adjuvants may be non-covalently associated with additional agents, such as by way of hydrophobic or ionic interactions.

Two preferred cytokine inducing agents are (a) immunostimulatory oligonucleotides and (b) 3dMPL.

Immunostimulatory oligonucleotides can include nucleotide modifications/analogs such as phosphorothioate modifications and can be double-stranded or (except for RNA) single-stranded. Kandimalla et al. (2003) Nucleic Acids Research 31:2393-2400, WO02/26757, and WO99/62923 disclose possible analog substitutions e.g. replacement of guanosine with 2'-deoxy-7-deazaguanosine. The adjuvant effect of CpG oligonucleotides is further discussed in Krieg (2003) Nature Medicine 9:831-835, McCluskie et al. (2002) FEMS Immunology and Medical Microbiology 32:179-185, WO98/401 US 6,207,646, US 6,239,116, and US 6,429,199. A CpG sequence may be directed to TLR9, such as the motif GTCGTT or TTCGTT (Kandimalla et al. (2003) Biochemical Society Transactions 31 (part 3):654-658). The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in Blackwell et al. (2003) J Immunol 170:4061-4068, Krieg (2002) Trends Immunol 23:64-65, and WO01/95935 Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, Kandimalla et al. (2003) Biochemical Society Transactions 31 (part 3):654-658, Kandimalla et al. (2003) BBRC 306:948-953, Bhagat et al. (2003) BBRC 300:853-861, and WO03/035836. A useful CpG adjuvant is CpG7909, also known as ProMune™ (Coley Pharmaceutical Group, Inc.).

As an alternative, or in addition, to using CpG sequences, TpG sequences can be used (WO01/22972). These oligonucleotides may be free from unmethylated CpG motifs.

The immunostimulatory oligonucleotide may be pyrimidine rich. For example, it may comprise more than one consecutive thymidine nucleotide (e.g. TTTT, as disclosed in WO01/22972), and/or it may have a nucleotide composition with >25% thymidine (e.g. >35%, >40%, >50%, >60%, >80%, etc.). For example, it may comprise more than one consecutive cytosine nucleotide (e.g. CCCC, as disclosed in WO2004/87153), and/or it may have a nucleotide composition with >25% cytosine (e.g. >35%, >40%, >50%, >60%, >80%, etc.). These oligonucleotides may be free from unmethylated CpG motifs.

Immunostimulatory oligonucleotides will typically comprise at least 20 nucleotides. They may comprise fewer than 100 nucleotides.

3dMPL (also know as 3 de-O-acylated monophosphoryl lipid A or 3 O desacyl 4' monophosphoryl lipid A) is an adjuvant in which position 3 of the reducing end glucosamine in monophosphoryl lipid A has been de-acylated. 3dMPL has been prepared from a heptoseless mutant of Salmonella minnesota, and is chemically similar to lipid A but lacks an acid-labile phosphoryl group and a base-labile acyl group. It activates cells of the monocyte/macrophage lineage and stimulates release of several cytokines, including IL 1, IL-12, TNF a and GM-CSF (see also Thompson et al. (2005) J

Leukoc Biol 78: 'The low-toxicity versions of LPS, MPL® adjuvant and RC529, are efficient adjuvants for CD4+ T cells'). Preparation of 3dMPL was originally described in GB A 2220211.

3dMPL can take the form of a mixture of related molecules, varying by their acylation (e.g. having 3, 4, 5 or 6 acyl chains, which may be of different lengths). The two glucosamine (also known as 2 deoxy-2-amino glucose) monosaccharides are N acylated at their 2 position carbons (i.e. at positions 2 and 2'), and there is also O acylation at the 3' position. The group attached to carbon 2 has formula -NH-CO-CH2-CR1R1'. The group attached to carbon 2' has formula-NH-CO-CH2-CR2R2'. The group attached to carbon 3' has formula -O-CO-CH2-CR3R3'. A representative structure is:

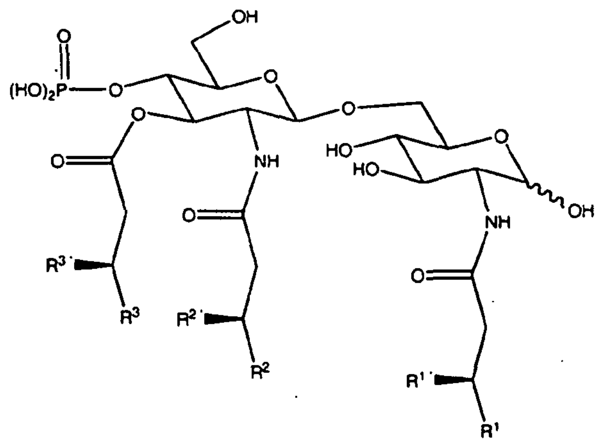

Groups R1, R2 and R3 are each independently -(CH2)n-CH3. The value of n is preferably between 8 and 16, more preferably between 9 and 12, and is most preferably 10.

Groups R1', R2' and R3' can each independently be: (a) -H; (b) -OH; or (c) -0 CO R4,where R4 is either -H or-(CH2)m-CH3, wherein the value of m is preferably between 8 and 16, and is more preferably 10, 12 or 14. At the 2 position, m is preferably 14. At the 2' position, m is preferably 10. At the 3' position, m is preferably 12. Groups R1', R2' and R3' are thus preferably - O acyl groups from dodecanoic acid, tetradecanoic acid or hexadecanoic acid.

When all of R1', R2' and R3' are -H then the 3dMPL has only 3 acyl chains (one on each of positions 2, 2' and 3'). When only two of R1', R2' and R3' are -H then the 3dMPL can have 4 acyl chains. When only one of R1', R2' and R3' is -H then the 3dMPL can have 5 acyl chains. When none of R1', R2' and R3' is -H then the 3dMPL can have 6 acyl chains. The 3dMPL adjuvant used according to the invention can be a mixture of these forms, with from 3 to 6 acyl chains, but it is preferred to include 3dMPL with 6 acyl chains in the mixture, and in particular to ensure that the hexaacyl chain form makes up at least 10% by weight of the total 3dMPL e.g.>20%, >30%, >40%, >50% or more. 3dMPL with 6 acyl chains has been found to be the most adjuvant active form.

Thus the most preferred form of 3dMPL for inclusion in compositions of the invention is:

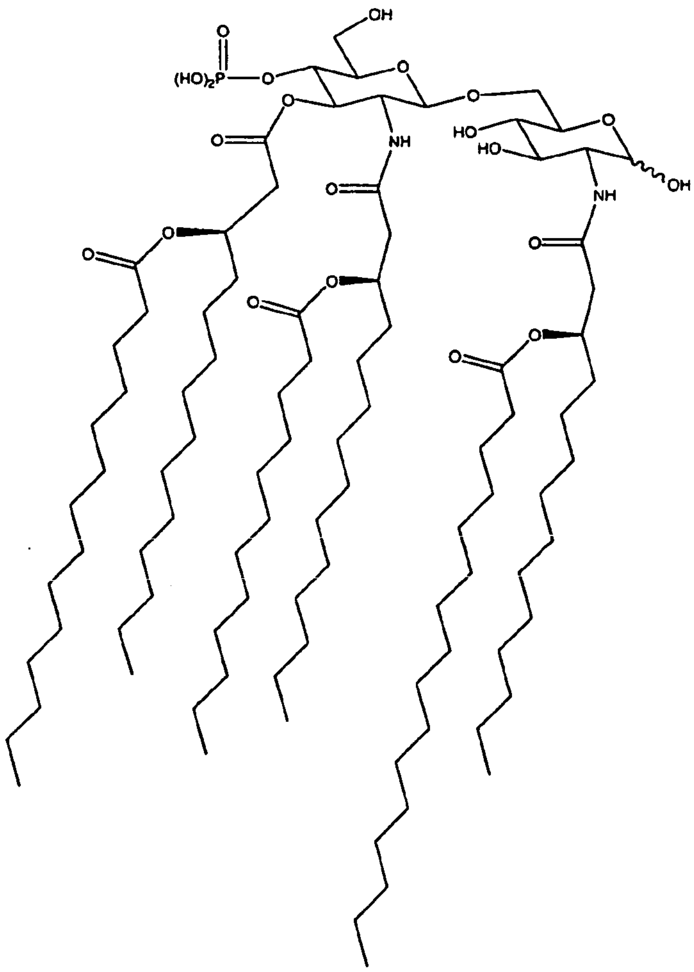

Where 3dMPL is used in the form of a mixture then references to amounts or concentrations of 3dMPL in compositions of the invention refer to the combined 3dMPL species in the mixture.

In aqueous conditions, 3dMPL can form micellar aggregates or particles with different sizes e.g. with a diameter <150nm or >500nm. Either or both of these can be used with the invention, and the better particles can be selected by routine assay. Smaller particles (e.g. small enough to give a clear aqueous suspension of 3dMPL) are preferred for use according to the invention because of their superior activity (WO 94/21292). Preferred particles have a mean diameter less than 220nm, more preferably less than 200nm or less than 150nm or less than 120nm, and can even have a mean diameter less than 100nm. In most cases, however, the mean diameter will not be lower than 50nm. These particles are small enough to be suitable for filter sterilization. Particle diameter can be assessed by the routine technique of dynamic light scattering, which reveals a mean particle diameter. Where a particle is said to have a diameter of x nm, there will generally be a distribution of particles about this mean, but at least 50% by number (e.g. >60%, >70%, >80%, >90%, or more) of the particles will have a diameter within the range x+25%.

3dMPL can advantageously be used in combination with an oil in water emulsion. Substantially all of the 3dMPL may be located in the aqueous phase of the emulsion.

The 3dMPL can be used on its own, or in combination with one or more further compounds. For example, it is known to use 3dMPL in combination with the QS21 saponin (WO94/00153) (including in an oil in water emulsion (WO95/17210)), with an immunostimulatory oligonucleotide, with both QS21 and an immunostimulatory oligonucleotide, with aluminum phosphate (WO96/26741), with aluminum hydroxide (WO93/19780 or with both aluminum phosphate and aluminum hydroxide.

*Fatty adjuvants*

Fatty adjuvants that can be used with the invention include the oil in water emulsions described above, and also include, for example:

- A compound of formula I, II or III, or a salt thereof:

I

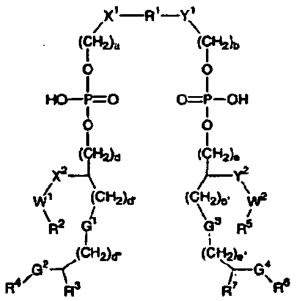

II

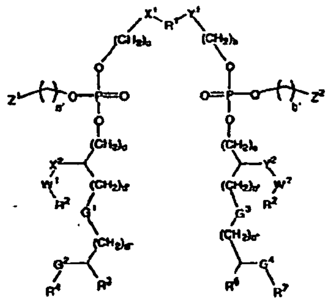

III

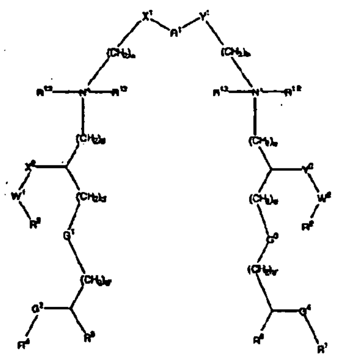

as defined in WO03/011223, such as 'ER 803058', 'ER 803732', 'ER 804053', ER 804058', 'ER 804059', 'ER 804442', 'ER 804680', 'ER 804764', ER 803022 or 'ER 804057' e.g.:

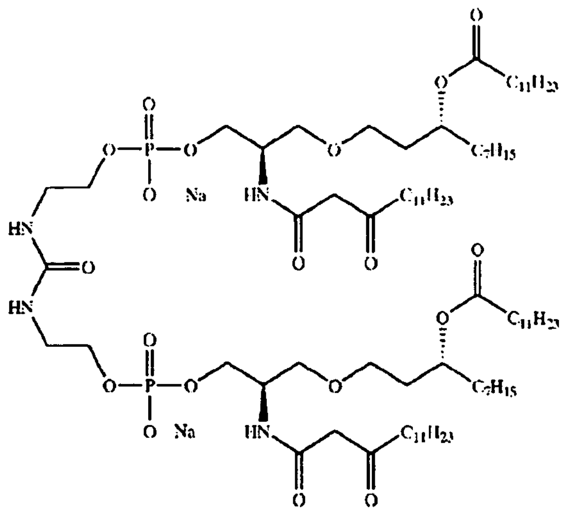

ER804057

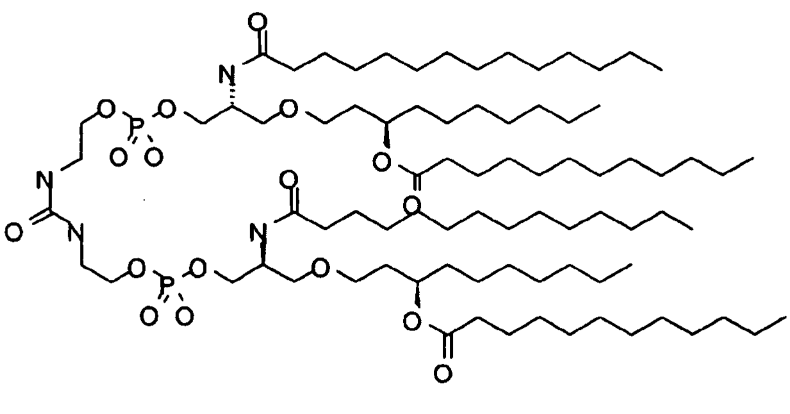

ER 803022:

- Derivatives of lipid A from Escherichia coli such as OM-174 (described in Meraldi et al. (2003) Vaccine 21:2485-2491 and Pajak et al. (2003) Vaccine 21:836-842).

- A formulation of a cationic lipid and a (usually neutral) co-lipid, such as aminopropyl-dimethyl-myristoleyloxy-propanaminium bromide-diphytanoylphosphatidyl-ethanolamine ("Vaxfectin™") or aminopropyl-dimethyl-bis-dodecyloxy-propanaminium bromide-dioleoylphosphatidyl-ethanolamine ("GAP-DLRIE:DOPE"). Formulations containing (+)-N-(3-aminopropyl)-N,N-dimethyl-2,3-bis(syn-9-tetradeceneyloxy)-1-propanaminium salts are preferred (US 6,586,409).

- 3 O deacylated monophosphoryl lipid A (see above).

- Compounds containing lipids linked to a phosphate-containing acyclic backbone, such as the TLR4 antagonist E5564 (Wong et al. (2003) J Clin Pharmacol 43(7):735-42; US 2005/0215517):

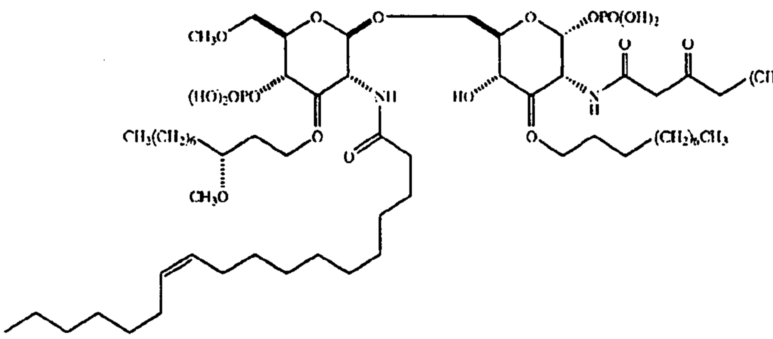

*Aluminum salt adjuvants*

The adjuvants known as aluminum hydroxide and aluminum phosphate may be used. These names are conventional, but are used for convenience only, as neither is a precise description of the actual chemical compound which is present (e.g. see chapter 9 of Vaccine Design: The Subunit and Adjuvant Approach (eds. Powell & Newman) Plenum Press 1995 (ISBN 0-306-44867-X)). The invention can use any of the "hydroxide" or "phosphate" adjuvants that are in general use as adjuvants.

The adjuvants known as "aluminium hydroxide" are typically aluminium oxyhydroxide salts, which are usually at least partially crystalline. Aluminium oxyhydroxide, which can be represented by the formula AlO(OH), can be distinguished from other aluminium compounds, such as aluminium hydroxide $Al(OH)_3$, by infrared (IR) spectroscopy, in particular by the presence of an adsorption band at $1070cm^{-1}$ and a strong shoulder at $3090\text{-}3100cm^{-1}$ (chapter 9 of Vaccine Design: The Subunit and Adjuvant Approach (eds. Powell & Newman) Plenum Press 1995 (ISBN 0-306-44867-X)). The degree of crystallinity of an aluminium hydroxide adjuvant is reflected by the width of the diffraction band at half height (WHH), with poorly crystalline particles showing greater line broadening due to smaller crystallite sizes. The surface area increases as WHH increases, and adjuvants with higher WHH values have been seen to have greater capacity for antigen adsorption. A fibrous morphology (e.g. as seen in transmission electron micrographs) is typical for aluminium hydroxide adjuvants. The pI of aluminium hydroxide adjuvants is typically about 11 i.e. the adjuvant itself has a positive surface charge at physiological pH. Adsorptive capacities of between 1.8-2.6 mg protein per mg $Al^{+++}$ at pH 7.4 have been reported for aluminium hydroxide adjuvants.

The adjuvants known as "aluminium phosphate" are typically aluminium hydroxyphosphates, often also containing a small amount of sulfate (i.e. aluminium hydroxyphosphate sulfate). They may be obtained by precipitation, and the reaction conditions and concentrations during precipitation influence the degree of substitution of phosphate for hydroxyl in the salt. Hydroxyphosphates generally have a $PO_4/Al$ molar ratio between 0.3 and 1.2. Hydroxyphosphates can be distinguished from strict $AlPO_4$ by the presence of hydroxyl groups. For example, an IR spectrum band at $3164cm^{-1}$ (e.g. when heated to 200°C) indicates the presence of structural hydroxyls (ch.9 of Vaccine Design: The Subunit and Adjuvant Approach (eds. Powell & Newman) Plenum Press 1995 (ISBN 0-306-44867-X)).

The $PO_4/Al^{3+}$ molar ratio of an aluminium phosphate adjuvant will generally be between 0.3 and 1.2, preferably between 0.8 and 1.2, and more preferably 0.95+0.1. The aluminium phosphate will generally be amorphous, particularly for hydroxyphosphate salts. A typical adjuvant is amorphous aluminium hydroxyphosphate with $PO_4/Al$ molar ratio

between 0.84 and 0.92, included at 0.6mg $Al^{3+}$/ml. The aluminium phosphate will generally be particulate (e.g. plate like morphology as seen in transmission electron micrographs). Typical diameters of the particles are in the range 0.5-20μm (e.g. about 5 10μm) after any antigen adsorption. Adsorptive capacities of between 0.7-1.5 mg protein per mg $Al^{+++}$ at pH 7.4 have been reported for aluminium phosphate adjuvants.

The point of zero charge (PZC) of aluminium phosphate is inversely related to the degree of substitution of phosphate for hydroxyl, and this degree of substitution can vary depending on reaction conditions and concentration of reactants used for preparing the salt by precipitation. PZC is also altered by changing the concentration of free phosphate ions in solution (more phosphate = more acidic PZC) or by adding a buffer such as a histidine buffer (makes PZC more basic). Aluminium phosphates used according to the invention will generally have a PZC of between 4.0 and 7.0, more preferably between 5.0 and 6.5 e.g. about 5.7.

Suspensions of aluminium salts used to prepare compositions of the invention may contain a buffer (e.g. a phosphate or a histidine or a Tris buffer), but this is not always necessary. The suspensions are preferably sterile and pyrogen free. A suspension may include free aqueous phosphate ions e.g. present at a concentration between 1.0 and 20 mM, preferably between 5 and 15 mM, and more preferably about 10 mM. The suspensions may also comprise sodium chloride.

The invention can use a mixture of both an aluminium hydroxide and an aluminium phosphate (WO01/22992). In this case there may be more aluminium phosphate than hydroxide e.g. a weight ratio of at least 2:1 e.g. >5:1, >6:1, >7:1, >8:1, >9:1, etc.

The concentration of $Al^{+++}$ in a composition for administration to a patient is preferably less than 10mg/ml e.g. <5 mg/ml, <4 mg/ml, <3 mg/ml, <2 mg/ml, <1 mg/ml, etc. A preferred range is between 0.3 and 1mg/ml.

As well as including one or more aluminium salt adjuvants, the adjuvant component may include one or more further adjuvant or immunostimulating agents. Such additional components include, but are not limited to: a 3-O-deacylated monophosphoryl lipid A adjuvant ('3d MPL'); and/or an oil in water emulsion. 3d MPL has also been referred to as 3 de-O-acylated monophosphoryl lipid A or as 3 O desacyl 4'monophosphoryl lipid A. The name indicates that position 3 of the reducing end glucosamine in monophosphoryl lipid A is de-acylated. It has been prepared from a heptoseless mutant of S.minnesota, and is chemically similar to lipid A but lacks an acid-labile phosphoryl group and a base-labile acyl group. It activates cells of the monocyte/macrophage lineage and stimulates release of several cytokines, including IL-1, IL-12, TNF α and GM-CSF. Preparation of 3d MPL was originally described in reference 150, and the product has been manufactured and sold by Corixa Corporation under the name MPL™. Further details can be found in Myers *et al.* (1990) pages 145-156 of Cellular and molecular aspects of endotoxin reactions, Ulrich (2000) Chapter 16 (pages 273-282) of Vaccine Adjuvants: Preparation Methods and Research Protocols (Volume 42 of Methods in Molecular Methods series). ISBN: 1-59259-083-7. Ed. O'Hagan, Johnson et al. (1999) J Med Chem 42:4640-9, and Baldrick et al. (2002) Regulatory Toxicol Pharmacol 35:398-413.

Vaccines produced by the invention may be administered to patients at substantially the same time as (e.g. during the same medical consultation or visit to a healthcare professional) other vaccines e.g. at substantially the same time as a measles vaccine, a mumps vaccine, a rubella vaccine, a MMR vaccine, a varicella vaccine, a MMRV vaccine, a diphtheria vaccine, a tetanus vaccine, a pertussis vaccine, a DTP vaccine, a conjugated H.influenzae type b vaccine, an inactivated poliovirus vaccine, a hepatitis B virus vaccine, a pneumococcal conjugate vaccine, etc. Administration at substantially the same time as a pneumococcal vaccine is particularly useful in elderly patients.

The composition may include an antibiotic.

Immunogenic compositions used as vaccines comprise an immunologically effective amount of the immunogenic polypeptide or immunogenic polypeptides (i.e., bacterial adhesin conformer F), as well as any other of the above-mentioned components, as needed. By "immunologically effective amount," it is meant that the administration of that amount to an individual, either in a single dose or as part of a series, is effective for treatment or prevention. This amount varies depending upon the health and physical condition of the individual to be treated, the taxonomic group of individual to be treated (e.g., nonhuman primate, primate, etc.), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, e.g., by injection, either subcutaneously, intramuscularly, or transdermally/transcutaneously (e.g., WO98/20734). Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents, As an alternative to protein-based vaccines, DNA vaccination may be employed (e.g., Robinson & Torres (1997) Seminars in Immunology 9:271-283; Donnelly et al. (1997) Annu Rev Immunol 15:617-648; see later herein).

ANTIBODIES

As used herein, the term "antibody" refers to a polypeptide or group of polypeptides composed of at least one antibody combining site. An "antibody combining site" is the three-dimensional binding space with an internal surface shape and charge distribution complementary to the features of an epitope of an antigen, which allows a binding of the antibody with the antigen. Antibody includes, for example, vertebrate antibodies, hybrid antibodies, chimeric antibodies, humanized antibodies, altered antibodies, univalent antibodies, Fab proteins, and single domain antibodies.

Antibodies against the proteins of the invention are useful for affinity chromatography, immunoassays, and distinguishing/identifying bacterial proteins.

Antibodies to the conformers of the invention, both polyclonal and monoclonal, may be prepared by conventional methods. In general, the protein is first used to immunize a suitable animal, preferably a mouse, rat, rabbit or goat. Rabbits and goats are preferred for the preparation of polyclonal sera due to the volume of serum obtainable, and the availability of labeled anti-rabbit and anti-goat antibodies. Immunization is generally performed by mixing or emulsifying the protein in saline, preferably in an adjuvant such as Freund's complete adjuvant, and injecting the mixture or emulsion parenterally (generally subcutaneously or intramuscularly). A dose of 50-200 μg/injection is typically sufficient. Immunization is generally boosted 2-6 weeks later with one or more injections of the protein in saline, preferably using Freund's incomplete adjuvant. One may alternatively generate antibodies by in vitro immunization using methods known in the art, which for the purposes of this invention is considered equivalent to in vivo immunization. Polyclonal antisera is obtained by bleeding the immunized animal into a glass or plastic container, incubating the blood at 25° C for one hour, followed by incubating at 40° C for 2-18 hours. The serum is recovered by centrifugation (e.g., 1,000 g for 10 minutes). About 20-50 ml per bleed may be obtained from rabbits.

Monoclonal antibodies are prepared using the standard method of Kohler & Milstein (Nature (1975) 256:495-96), or a modification thereof. Typically, a mouse or rat is immunized as described above. However, rather than bleeding the animal to extract serum, the spleen (and optionally several large lymph nodes) is removed and dissociated into single cells. If desired, the spleen cells may be screened (after removal of nonspecifically adherent cells) by applying a cell suspension to a plate or well coated with the protein antigen, B-cells expressing membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (e.g., hypoxanthine, aminopterin, thymidine medium, "HAT"). The resulting hybridomas are plated by limiting dilution, and are assayed for the production of antibodies which bind specifically to the immunizing antigen (and which do not bind to unrelated antigens). The selected MAb- secreting hybridomas are then cultured either in vitro (e.g. , in tissue culture bottles or hollow fiber reactors), or in vivo (as ascites in mice).

If desired, the antibodies (whether polyclonal or monoclonal) may be labeled using conventional techniques. Suitable labels include fluorophores, chromophores, radioactive atom s (particularly $^{32}P$ and $^{125}I$), electron-dense reagents, enzymes, and ligands having specific binding partners. Enzymes are typically detected by their activity. For example, horseradish peroxidase is usually detected by its ability to convert 3,3',5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, $^{125}I$ may serve as a radioactive label or as an electron- dense reagent. HRP may serve as enzyme or as antigen for a MAb. Further, one may combine various labels for desired effect. For example, MAbs and avidin also require labels in the practice of this invention: thus, one might label a MAb with biotin, and detect its presence with avidin labeled with $^{125}I$, or with an anti-biotin MAb labeled with HRP. Other permutations and possibilities will be readily apparent to those of ordinary skill in the art, and are considered as equivalents within the scope of the invention.

Pharmaceutical Compositions

Pharmaceutical compositions can comprise either polypeptides or antibodies of the invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However,

the effective amount for a given situation can be determined by routine experimentation and is within the judgment of the clinician.

Preferred dosages for protein based pharmaceuticals including vaccines will be between 5 and 500 $\mu$5 of the immunogenic polypeptides of the present invention.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

<u>DELIVERY METHODS</u>

Once formulated, the compositions of the invention can be administered directly to the subject. The subjects to be treated can be animals; in particular, human subjects can be treated. _

Once formulated, the compositions of the invention can be administered (1) directly to the subject or (2) delivered ex vivo, to cells derived from the subject. The subjects to be treated can be mammals or birds. Also, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal or transcutaneous applications (e. g., see WO98/20734), needles, and gene guns or hyposprays. Dosage treatment may be a single dose schedule or a multiple dose schedule.

Methods for the ex vivo delivery and reimplantation of transformed cells into a subject are known in the aft and described in e.g., WO93/14778. Examples of cells useful in ex vivo applications include, for example, stem cells, particularly hematopoietic, lymph cells, macrophages, dendritic cells, or tumor cells.

<u>Polypeptide pharmaceutical compositions</u>

In addition to the pharmaceutically acceptable carriers and salts described above, the following additional agents can be used with the polypeptide compositions.

*<u>i. Polypeptides</u>*

One example are polypeptides which include, without limitation: asioloorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII

*<u>ii. Hormones, Vitamins, etc.</u>*

Other groups that can be included are, for example: hormones, steroids, androgens, estrogens, thyroid hormone, or vitamins, folic acid.

*iii. Polyalkylenes, Polysaccharides, etc.*

Also, polyalkylene glycol can be included with the desired polypeptides. In a preferred embodiment, the polyalkylene glycol is polyethlylene glycol. In addition, mono-, di-, or polysaccharides can be included. In a preferred embodiment of this aspect, the polysaccharide is dextran or DEAE-dextran. Also, chitosan and poly(lactide-co-glycolide)

*iv. Lipids, and Liposomes*

The desired polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) Biochim. Biophys. Acta. 1097:1-17; Straubinger (1983) Meth. Enzymol. 101:512-527.

Liposomal preparations for use in the present invention include cationic (positively charged), anionic (negatively charged) and neutral preparations. Cationic liposomes have been shown to mediate intracellular delivery of plasmid DNA (Feigner (1987) Proc. Natl. Acad. Sci. USA 84:7413-7416); mRNA (Malone (1989) Proc. Natl. Acad. Sci. USA 86: 6077- 6081); and purified transcription factors (Debs (1990) J. Biol. Chem. 265:10189-10192), in functional form.

Cationic liposomes are readily available. For example, N(1-2, 3- dioleyloxy)propyl)-N,N,N-triethylammonium (DOTMA) liposomes are available under the trademark Lipofectin, from GIBCO BRL, Grand Island, NY. (See, also, Feigner supra). Other commercially available liposomes include transfectace (DDAB/DOPE) and DOTAP/DOPE (Boerhinger). Other cationic liposomes can be prepared from readily available materials using techniques well known in the art. See, e.g., Szoka (1978) Proc. Natl. Acad. Sci. USA 75:4194-4198; WO90/11092 for a description of the synthesis of DOTAP (1 2 -bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See e.g., Straubinger (1983) Meth. Immunol. 101:512-527; Szoka (1978) Proc. Nall. Acad. Sci. USA 75:4194-4198; Papahadjopoulos (1975) Biochim. Biophys. Acta 394:483; Wilson (1979) Cell 17:77); Deamer & Bangham (1976) Biochim. Biophys. Acta 443:629; Ostro (1977) Biochem. Biophys. Res. Commun. 76:836; Fraley (1979) Proc. Natl. Acad. Sci. USA 76:3348); Enoch & Strittmatter (1979) Proc. Natl. Acad. Sci. USA 76:145; Fraley (1980) J. Biol. Chem. (1980) 255:10431; Szoka & Papahadjopoulos (1978)Proc. Natl. Acad. Sci. USA 75:145; and Schaefer- Ridder (1982) Science 215:166.

*v. Lipoproteins*

In addition, lipoproteins can be included with the polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.

Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.

A lipoprotein can comprise more than one apoprotein. For example, naturally occurring chylomicrons comprises of A, B, C, and E, over time these Lipoproteins lose A and acquire C and E apoproteins. VLDL comprises A, B, C, and E apoproteins, LDL comprises apoprotein B; and HDL comprises apoproteins A, C, and E. The amino acid of these apoproteins are known and are described in, for example, Breslow (1985) Annu Rev. Biochem 54:699; Law (1986) Adv. Exp Med. Biol. 151:162; Chen (1986) J Biol Chem 261:12918; Kane (1980) Proc Natl Acad Sci USA 77:2465; and Utermann (1984) Hum Genet 65:232.

Lipoproteins contain a variety of lipids including, triglycerides, cholesterol (free and esters), and phospholipids. The composition of the lipids varies in naturally occurring lipoproteins. For example, chylomicrons comprise mainly triglycerides. A more detailed description of the lipid content of naturally occurring lipoproteins can be found, for example, in Meth. Enzymol. 128 (1986). The composition of the lipids are chosen to aid in conformation of the apoprotein for

receptor binding activity. The composition of lipids can also be chosen to facilitate hydrophobic interaction and association with the polynucleotide binding molecule.

Naturally occurring lipoproteins can be isolated from serum by ultracentrifugation, for instance. Such methods are described in Meth. Enzymol. (supra); Pitas (1980) J. Biochem. 255:5454-5460 and Mahey (1979) J Clin. Invest 64: 743-750. Lipoproteins can also be produced by in vitro or recombinant methods by expression of the apoprotein genes in a desired host cell. See, for example, Atkinson (1986) Annu Rev Biophys Chem 15:403 and Radding (1958)Biochim BiophysActa 30:443. Lipoproteinscan also be purchased from commercial suppliers, such as Biomedical Technologies, Inc., Stoughton, Massachusetts, USA. Further description of lipoproteins can be found in Zuckermann et. al. WO98/06437..

*vi. Polycationic Agents*

Polycationic agents can be included, with or without lipoprotein, in a composition with the desired polynucleotide/ polypeptide to be delivered.

Polycationic agents, typically, exhibit a net positive charge at physiological relevant pH and are capable of neutralizing the electrical charge of nucleic acids to facilitate delivery to a desired location. These agents have both *in vitro, ex vivo,* and *in vivo* applications. Polycationic agents can be used to deliver nucleic acids to a living subject either intramuscularly, subcutaneously, etc. The following are examples of useful polypeptides as polycationic agents: polylysine, polyarginine, polyornithine, and protamine. Other examples include hisTones, protamines, human serum albumin, DNA binding proteins, non- histone chromosomal proteins, coat proteins from DNA viruses, such as (X174, transcriptional factors also contain domains that bind DNA and therefore may be useful as nucleic acid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that-bind DNA sequences.

Organic polycationic agents include: spermine, spermidine, and putrescine.

The dimensions and of the physical properties of a polycationic agent can be extrapolated from the list above, to construct other polypeptide polycationic agents or to produce synthetic polycationic agents.

Synthetic polycationic agents which are useful include, for example, DEAE- dextran, polybrene. Lipofectin™, and lipofectAMINE™ are monomers that form polycationic complexes when combined with polynucleotides/polypeptides.

IMMUNODIAGNOSTIC ASSAYS

Another aspect of the present invention includes GBS80 conformers of the present invention used in immunoassays to detect antibody levels (or, conversely, anti-bacterial adhesin conformer F antibodies can be used to detect conformer levels). Immunoassays based on well defined, recombinant antigens can be developed to replace invasive diagnostics methods. Antibodies within biological samples, including for example, blood or serum samples, can be detected. Design of the immunoassays is subject to a great deal of variation, and a variety of these are known in the art. Protocols for the immunoassay may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme labeled and mediated immunoassays, such as ELISA assays.

Kits suitable for immunodiagnosis and containing the appropriate labeled reagents are constructed by packaging the appropriate materials, including the compositions of the invention, in suitable containers, along with the remaining reagents and materials (for example, suitable buffers, salt solutions, etc.) required for the conduct of the assay, as well as suitable set of assay instructions.

GENERAL

The term "comprising" encompasses "including" as well as "consisting" e.g. a composition "comprising" X may consist exclusively of X or may include something additional e.g. X + Y. The term "about" in relation to a numerical value x means, for example, x+10%. The word "substantially" does not exclude "completely" e.g. a composition which is "substantially free" from Y may be completely free from Y. Where necessary, the word "substantially" may be omitted from the definition of the invention.

Sequences included to facilitate cloning or purification, etc., do not necessarily contribute to the invention and may be omitted or removed.

## BRIEF DESCRIPTION OF DRAWINGS AND TABLES

FIGURE 1 shows a SDS-PAGE of GBS. 80 purified isoforms.
FIGURE 2 shows an analytical gel filtration on a Superdex 200 10/30 of the same samples with PBS as buffer and a flow of 0.5 ml/min.
FIGURE 3 shows an analytical Gel Filtration of lot 3 and lot F at different times and pH.
FIGURE 4A shows an analytical Gel Filtration of 5 different GBS 80 lots
FIGURE 4B shows the molecular weights of 5 different GBS 80 lots as determined with MALDI-TOF spectrometry.
FIGURE 5 shows an SDS-PAGE after digestion with different proteases, with and without detergent denaturation.
FIGURE 6 shows a table summarizing the Active maternal immunization results.

## EXAMPLE 1: Purification of GBS 80 isoforms

*Batch production of GBS 80 in recombinant E.coli* Batch fermentation of recombinant *E. coli* expressing GBS 80 was carried out using a five-liter Applikon bench-top bioreactor (Applikon Dependable Instruments B.V., the Netherlands). The fermentor was inoculated with full-grown seed cultures that were grown at 25°C for 16 hours in two rotating Erlenmeyer flasks containing 500 ml of yeast extract medium (45 g of yeast extract per liter; 1.5 g of NaCl per liter; 1.10 g of glucose per liter; pH 7.0). For the main fermentation, a complex medium was used. The medium contained (per liter) 45 g of yeast extract, 5 g of NaCl, 10 g of glycerol, pH 7.0. The fermentation was run at 25°C. The pH of the culture was maintained automatically at 7.1 $\pm$ 0.1 by using sodium hydroxide or phosphoric acid as titrants. Fully aerobic conditions (dissolved oxygen tension 40%) were maintained throughout by injecting air and oxygen, both at a rate of 0.5 standard liter of air per liter of broth per min (= 0.5 vvm), into the region of the impeller that was rotating at about 800 rpm. Cells were grown up to 3 OD and were then induced with 0.25 mM IPTG for 3 hours before harvesting. At the time of induction 1mM $MgSO_4$, 1mM $CaCl_2$ and 5 g/L glycerol were also added.

*Purification procedure for conformer* A. Cells from fermentation were resuspended in 60 ml of 25 mM Tris/HCl 25 (pH 7.0) containing 10 mM EDTA, 2 mM PMSF and 100 Kunitz units of DNAse A, and lysed by a double pass through French press at 18000 psi. Unbroken cells and insoluble material were removed by centrifugation at 50,000 x g for 30 min. The supernatant had the pH adjusted to 7.0 with NaOH 0.1 M, was sterile filtered through a 0.22 mm filter (Millipore), diluted with MilliQ™ water until 300 ml in order to obtain a conductivity of about 2.5 mS/cm and subjected to ion exchange chromatography on a Q-Sepharose FF column (Amersham Biosciences). The entire 300 ml lysate was loaded on an 80-ml column volume Q-Sepharose FF column that had been previously equilibrated with 25 mM Tris/HCl (pH 7.0). The column was subsequently washed with six column volumes of the same buffer, and proteins were eluted with a linear gradient of 0-500 mM NaCl in the same buffer. The eluted sample was analysed for GBS 80 conformer A protein by 12% SDS-PAGE stained with Coomassie brilliant blue R-250, and the fractions of interest were pooled. The pool GBS 80 conformer A from Q-Sepharose FF (60 ml) was subsequently applied on a 75-ml Chelating Sepharose FF column (Amersham Biosciences) that had been charged with CuSO4 and equilibrated with Na-phosphate 20 mM, NaCl 1 M, pH 7.2 (buffer A). The column was washed with four column volumes of buffer A, and proteins eluted with a linear gradient of 0-100% buffer B (buffer B: Na-Phosphate 20 mM, NH4Cl 1M, pH 7.2). The eluted sample was analysed for GBS 80 conformer A protein by 12% SDS-PAGE stained with Coomassie brilliant blue R-250, and the fractions of interest were pooled. The pooled GBS 80 conformer A from Chelating Sepharose FF (140 ml) was then protein-concentrated to 15 ml under nitrogen pressure on Amicon concentration cell, filter 30 YM (Millipore) cutoff 30 KDa, and applied in three runs, each loading 5 ml of protein solution, on a Superdex 75 HiLoad 26/60 column (Amersham Biosciences) that had been equilibrated with phosphate buffered saline, pH 7.2 (PBS). The eluted sample was analysed for GBS 80 conformer A protein by 12% SDS-PAGE stained with Coomassie brilliant blue R-250, and the fractions of interest were pooled. The purity of the pooled GBS 80 conformer A protein was then estimated by 12% SDS-PAGE and analytical gel filtration, and identity confirmed by N-terminal amino acid analysis (491 cLC Protein Sequencer, Applied Biosystems), mass spectrometry and western blot.

*Purification procedure for conformer F.* Cells fermentation were resuspended in 100 ml of 25 mM Tris/HCl 25 (pH 7.2) containing 2 mM PMSF and 100 Kunitz units of DNAse A, and lysed by a double pass through French press at 18000 psi. Unbroken cells and insoluble material were removed by centrifugation at 50,000 x g for 30 min. The supernatant had the pH adjusted to 7.2 with NaOH 0.1 M, was sterile filtered through a 0.22 mm filter (Millipore), diluted with MilliQ™ water until 300 ml in order to obtain a conductivity of about 2.1 mS/cm and subjected to subtractive ion exchange chromatography on a Q-Sepharose FF column (Amersham Biosciences). The entire 300 ml lysate was loaded on an 80-ml column volume Q-Sepharose FF column that had been previously equilibrated with Tris/HCl 20 mM pH 7.7. To the pooled flow-through containing GBS 80 conformer F was added sodium phosphate buffer to a final concentration of 10 mM and the pH adjusted to 6.8 with NaOH 0.1 M. The pooled flow-through was subsequently applied on a 70-ml

Hydroxyapatite Bio-Gel HT column (Bio-Rad) equilibrated with sodium phosphate 10 mM pH 6.8. The column was washed with four column volumes of the same equilibration buffer, and proteins eluted with a linear gradient 10-500 mM sodium phosphate pH 6.8. The eluted sample was analysed for GBS 80 conformer F protein by 12% SDS-PAGE stained with Coomassie brilliant blue R-250, and the fractions of interest were pooled. The pooled GBS conformer 80 F from Hydroxyapatite Bio-Gel HT (130 ml) was then protein-concentrated to 12 ml under nitrogen pressure on Amicon concentration cell, filter 30 YM (Millipore) cutoff 30 KDa, and applied in three runs, each loading 4 ml of protein solution, on a Superdex 75 HiLoad 26/60 column (Amersham Biosciences) that had been equilibrated with phosphate buffered saline, pH 7.2 (PBS). The eluted sample was analysed for GBS 80 conformer F protein by 12% SDS-PAGE stained with Coomassie brilliant blue R-250, and the fractions of interest were pooled. The purity of the pooled GBS 80 conformer F protein was then estimated by 12% SDS-PAGE and analytical gel filtration, and identity confirmed by N-terminal amino acid analysis (491 cLC Protein Sequencer, Applied Biosystems), mass spectrometry and western blot.

EXAMPLE 2: SDS-PAGE and Analytical Gel Filtration

*Separation by SDS-PAGE*. Samples of three different GBS 80 lots were loaded on a dodecyl sulfate-polyacrylamide gel electrophoresis (SDS-PAGE) with and without previous heat denaturation (5 minutes at 99°C). The three lots are as follows:

- Lot 3: GBS 80 purified according to the "conformer A" protocol above;
- Lot F: GBS 80 recovered in the flow through of the purification from lot 3;
- Lot G-HA: GBS 80 purified according to the "conformer F" protocol above.

Figure 1 show the results of this experiment. Two main bands, corresponding to conformers A and conformer F are visible. Conformer F shows a lower apparent molecular weight compared to conformer A. As expected, lot 3 appears enriched in conformer A, whereas lot F and lot F-HA are enriched in conformer F. When the samples are boiled the two isoforms are undistinguishable and have the same electrophoretic mobility of conformer A. This demonstrates that conformer F is more stable than conformer A, which is likely denatured by the SDS where conformer F requires boiling to denature.

*Size Exclusion Chromatography.* Accordingly, a similar anomaly was also observed when the same protein preparations were applied to a size exclusion chromatography (SEC) column. Briefly, samples were applied in a final volume of 100 μl to a Superdex 200 HR10/30 gel filtration column (Amersham Biosciences) equilibrated with column buffer. The column was connected to an ÄKTApurifier system (Amersham Biosciences). Peak quantification of the elution profile obtained at 280 nm was completed according to the supplier's instructions. Figure 2 shows chromatograms of samples from the same lots. Two main peaks of UV-adsorbing material are clearly distinguishable with distinct elution volumes of approximately 12.3 and 13.3 ml.

MALDI mass spectrometry (MS) of samples from 5 different GBS 80 lots (3 enriched in conformer F and three in conformer A) revealed that the molecular weights of the different isoforms are consistent with the theoretical MW of 52,872 Da calculated for the full length expressed fragment, showing that the two isoforms have the same or very similar sequences (see figures 4a and 4b).

EXAMPLE 3: Stability over time and pH

Stability tests were performed to assess the increased stability of GBS 80 conformer F with respect to time and pH. In figure 3, chromatograms of the processed samples are reported.

The left panel shows that a GBS 80 preparation enriched in conformer A (lot 3) is less stable over the time as it undergoes to a peak redistribution. Chromatograms of a GBS 80 prep enriched in conformer F (lot F, right panel) are in contrast quite stable over the time even at different pH.

It can be noted that the absorbance peak corresponding to conformer A diminishes with time as the preparation elutes as a polydisperse peak. Conformer A converts to conformer F and to other conformers with a higher apparent MW (most probably associated to oligomer formation).

EXAMPLE 4: Protease digestion

Conformer A and conformer F show different digestion sensitivity to proteases. Figure 5 shows the results of digestion of both conformers with three different proteases (proteinase K, trypsin and AspN) both with and without prior treatment with detergent as follows.

Purified recombinant conformers A and F were heat-denatured 5 min at 95°C after addition of 0.1% final of "RapiGest" SF (Waters, Manchester, UK). Proteases were added to ratios substrate/enzyme 50/1 (wt/wt) to denatured

or non-denatured recombinant proteins. Reactions were allowed to proceed 2 hours at 37° C, and were stopped by addition of 0.2% formic acid final. Two μg of digestion products were denatured in sample loading buffer (0.06 M Tris-HCl pH 6.8, 10% (v/v) glycerol, 2% (wt/v) SDS, 100 mM DTT, 10 μg/ml bromophenol blue) and loaded on a 12% acrylamide SDS-PAGE. Gels were stained with Coomassie Bleu.

As shown in figure 5, conformer F is more resistant to digestion. Notably, a band with an apparent molecular weight of about 50 kDa was not digested, even after denaturation with "RapiGest" SF. This band (annotated with an asterisk) corresponds to the C-terminal part of the protein as defined by tryptic peptide mass finger print (result not shown). Moreover, the peptides released by these digestions are peptides belonging to the N-terminal part of the protein, as evidenced by mass spectrometry analyses (results not shown).

EXAMPLE 6: Active maternal immunization results

Both purified conformers were used to immunize groups of adult female mice which, at the end of the immunization schedule, were mated. The derived offspring were then challenged with a dose of GBS calculated to kill 80-90% of the pups. As shown in table 1 immunization with conformer F gives a higher protection level.

As used herein, an Active Maternal Immunization assay refers to an in vivo protection assay where female mice are immunized with the test antigen composition. The female mice are then bred and their pups are challenged with a lethal dose of GBS. Serum titers of the female mice during the immunization schedule are measured as well as the survival time of the pups after challenge.

Specifically, the Active Maternal Immunization assays referred to herein used groups of four CD-1 female mice (Charles River Laboratories, Calco Italy). These mice were immunized intraperitoneally with each purified conformer in Freund's adjuvant at days 1, 21 and 35, prior to breeding. 6-8 weeks old mice received 20 mg protein/dose. The immune response of the dams was monitored by using serum samples taken on day 0 and 49. The female mice were bred 2-7 days after the last immunization (at approximately t= 36 - 37), and typically had a gestation period of 21 days. Within 48 hours of birth, the pups were challenged via I.P. with GBS in a dose approximately equal to an amount which would be sufficient to kill 70 - 90 % of unimmunized pups (as determined by empirical data gathered from PBS control groups). The GBS challenge dose is preferably administered in 50ml of THB medium. Preferably, the pup challenge takes place at 56 to 61 days after the first immunization. The challenge inocula were prepared starting from frozen cultures diluted to the appropriate concentration with THB prior to use. Survival of pups was monitored for 5 days after challenge.

As shown in Figure 6 immunization with conformer F gives a higher protection level.

## Claims

1. An isolated bacterial adhesin in conformer F, wherein the bacterial adhesin is capable of generating an immune response in a subject, wherein the bacterial adhesin in conformer F is not retained by a Q-sepharose column, and wherein the bacterial adhesin is GBS80.

2. The isolated bacterial adhesin of claim 1, wherein the bacterial adhesin is produced recombinantly.

3. The isolated bacterial adhesin of claim 1 or claim 2, wherein the bacterial adhesin:

   a) is retained by a hydroxyapatite column;
   b) runs as a single band with lower apparent molecular weight on SDS-PAGE in the absence of heat-denaturation when compared to the bacterial adhesin after heat-denaturation;
   c) is more resistant to protease digestion than the bacterial adhesin in conformer A, wherein the bacterial adhesin in conformer A is retained by a Q-sepharose column; or
   d) elutes from a size exclusion chromatography column as a single monodisperse peak.

4. An antibody which binds to a bacterial adhesin in conformer F according to any one of claims 1-3, but not to the bacterial adhesin in conformer A, wherein the bacterial adhesin in conformer A is retained by a Q-sepharose column.

5. The antibody of claim 4, wherein said antibody is a monoclonal antibody, a chimeric antibody, a humanized antibody, or a fully human antibody.

6. A composition comprising the antibody of claim 4 or claim 5.

7. A composition comprising a bacterial adhesin according to any one of claims 1-3 and comprising less than 20% of the bacterial adhesin in conformer A, wherein the bacterial adhesin in conformer A is retained by a Q-sepharose column.

8. A composition comprising at least 1 or more parts of GBS80 in conformer F to 1 part of GBS80 in conformer A,

wherein the GBS80 in conformer F is capable of generating an immune response in a subject, wherein GBS80 in conformer F is not retained by a Q-sepharose column, and wherein GBS80 in conformer A is retained by a Q-sepharose column.

**9.** A composition according to any one of claims 6-8, being an immunogenic composition, a vaccine composition or a diagnostic composition.

**10.** A composition according to any one of claims 6-8 for use as a pharmaceutical.

**11.** A composition according to any one of claims 6-8 for the treatment or prevention of GBS infection.

**12.** A method of separating a GBS80 in conformer F from a GBS80 in conformer A comprising:

a) providing a sample containing a mixture of the GBS80 in conformer F and the GBS80 in conformer A;
b) separating the GBS80 in conformer F from the GBS80 in conformer A using a separation technology selected from the group consisting of an anion exchange separation technology, an hydroxyapatite-based separation technology, and a friction coefficient-based separation technology,

wherein GBS80 in conformer F is not retained by a Q-sepharose column, and wherein GBS80 in conformer A is retained by a Q-sepharose column.

**13.** The method of claim 12 wherein the friction coefficient-based separation technology is selected from the group comprising gel electrophoresis, size-exclusion chromatography, field-flow fractionation and velocity sedimentation centrifugation.

**14.** A method for isolating the GBS80 conformer F comprising applying a sample containing a mixture of conformers onto an ion exchange chromatography, recovering the flow-through and isolating the conformer F with an hydroxyapatite chromatographic step, wherein GBS80 in conformer F is not retained by a Q-sepharose column.

**Patentansprüche**

**1.** Isoliertes bakterielles Adhäsin in Konformer F, wobei das bakterielle Adhäsin eine Immunreaktion in einem Individuum hervorrufen kann, wobei das bakterielle Adhäsin in Konformer F nicht durch eine Q-Sepharosesäule zurückgehalten wird, und wobei das bakterielle Adhäsin GBS80 ist.

**2.** Isoliertes bakterielles Adhäsin nach Anspruch 1, wobei das bakterielle Adhäsin rekombinant produziert wird.

**3.** Isoliertes bakterielles Adhäsin nach Anspruch 1 oder Anspruch 2, wobei das bakterielle Adhäsin:

a) durch eine Hydroxyapatitsäule zurückgehalten wird;
b) als einzelne Bande mit einem niedrigeren apparenten Molekulargewicht auf SDS-PAGE in der Abwesenheit von Hitzedenaturierung im Vergleich zu dem bakteriellen Adhäsin nach Hitzedenaturierung läuft;
c) resistenter ist gegenüber einer Protease-Spaltung als das bakterielle Adhäsin in Konformer A, wobei das bakterielle Adhäsin in Konformer A durch eine Q-Sepharosesäule zurückgehalten wird; oder
d) von einer Größenausschluss-Chromatographiesäule als einzelner monodisperser Peak eluiert.

**4.** Antikörper, der an ein bakterielles Adhäsin in Konformer F nach einem der Ansprüche 1 bis 3 bindet, aber nicht an das bakterielle Adhäsin in Konformer A, wobei das bakterielle Adhäsin in Konformer A durch eine Q-Sepharosesäule zurückgehalten wird.

**5.** Antikörper nach Anspruch 4, wobei der Antikörper ein monoklonaler Antikörper, ein chimärer Antikörper, ein humanisierter Antikörper oder ein vollständiger humaner Antikörper ist.

**6.** Zusammensetzung, umfassend den Antikörper nach Anspruch 4 oder Anspruch 5.

**7.** Zusammensetzung, umfassend ein bakterielles Adhäsin nach einem der Ansprüche 1 bis 3 und weniger als 20% bakterielles Adhäsin in Konformer A, wobei das bakterielle Adhäsin in Konformer A durch eine Q-Sepharosesäule

zurückgehalten wird.

8. Zusammensetzung, umfassend mindestens 1 oder mehrere Teile GBS80 in Konformer F zu 1 Teil GBS80 in Konformer A, wobei das GBS80 in Konformer F eine Immunreaktion in einem Individuum hervorrufen kann, wobei das GBS80 in Konformer F nicht durch eine Q-Sepharosesäule zurückgehalten wird, und wobei das GBS80 in Konformer A durch eine Q-Sepharosesäule zurückgehalten wird.

9. Zusammensetzung nach einem der Ansprüche 6 bis 8, bei der es sich um eine immunogene Zusammensetzung, eine Impfstoff-Zusammensetzung oder eine diagnostische Zusammensetzung handelt.

10. Zusammensetzung nach einem der Ansprüche 6 bis 8 zur Verwendung als Pharmazeutikum.

11. Zusammensetzung nach einem der Ansprüche 6 bis 8 zur Behandlung oder Vorbeugung einer GBS-Infektion.

12. Verfahren zur Trennung eines GBS80 in Konformer F von einem GBS80 in Konformer A, umfassend:

a) Bereitstellen einer Probe, die ein Gemisch aus GBS80 in Konformer F und GBS80 in Konformer A enthält;
b) Trennen des GBS80 in Konformer F von dem GBS80 in Konformer A mit einer Trenntechnik, ausgewählt aus der Gruppe, bestehend aus einer Anionenaustausch-Trenntechnik, einer Trenntechnik auf Hydroxyapatit-Basis und einer Trenntechnik auf der Basis des Reibungskoeffizienten,

wobei GBS80 in Konformer F nicht durch eine Q-Sepharosesäule zurückgehalten wird, und wobei das GBS80 in Konformer A durch eine Q-Sepharosesäule zurückgehalten wird.

13. Verfahren nach Anspruch 12, wobei die Trenntechnik auf der Basis des Reibungskoeffizienten ausgewählt ist aus der Gruppe, bestehend aus Gelelektrophorese, Größenausschlusschromatographie, Feldflussfraktionierung und Geschwindigkeits-Sedimentationszentrifugation.

14. Verfahren zur Isolation des GBS80-Konformers F, umfassend das Auftragen einer Probe, die ein Gemisch von Konformeren enthält, auf eine Ionenaustauschchromatographie, Gewinnen des Durchflusses und Isolieren des Konformers F mit einem Hydroxyapatit-Chromatographieschritt, wobei das GBS80 im Konformer F nicht durch eine Q-Sepharosesäule zurückgehalten wird.

## Revendications

1. Adhésine bactérienne isolée sous forme du conformère F, **caractérisée en ce que** l'adhésine bactérienne est capable de générer une réponse immunitaire chez un sujet, **en ce que** l'adhésine bactérienne sous forme du conformère F n'est pas retenue par une colonne Q-sépharose, et **en ce que** l'adhésine bactérienne est GBS80.

2. Adhésine bactérienne isolée selon la revendication 1, **caractérisée en ce que** l'adhésine bactérienne est produite par voie recombinante.

3. Adhésine bactérienne isolée selon la revendication 1 ou la revendication 2, **caractérisée en ce que** l'adhésine bactérienne :

(a) est retenue par une colonne d'hydroxyapatite ;
(b) migre sous forme d'une bande unique ayant un poids moléculaire apparent en SDS-PAGE en l'absence de dénaturation par la chaleur plus faible en comparaison avec l'adhésine bactérienne après dénaturation par la chaleur ;
(c) est plus résistante à la digestion par les protéases que l'adhésine bactérienne sous forme du conformère A, l'adhésine bactérienne sous forme du conformère A étant retenue par une colonne Q-sépharose ; ou
(d) est éluée d'une colonne de chromatographie d'exclusion stérique sous forme d'un pic monodisperse unique.

4. Anticorps qui se lie à une adhésine bactérienne sous forme du conformère F selon l'une quelconque des revendications 1 à 3, mais pas à l'adhésine bactérienne sous forme du conformère A, l'adhésine bactérienne sous forme du conformère A étant retenue par une colonne Q-sépharose.

**5.** Anticorps selon la revendication 4, **caractérisé en ce que** ledit anticorps est un anticorps monoclonal, un anticorps chimère, un anticorps humanisé ou un anticorps totalement humain.

**6.** Composition comprenant l'anticorps selon la revendication 4 ou la revendication 5.

**7.** Composition comprenant une adhésine bactérienne selon l'une quelconque des revendications 1 à 3 et comprenant moins de 20 % de l'adhésine bactérienne sous forme du conformère A, l'adhésine bactérienne sous forme du conformère A étant retenue par une colonne Q-sépharose.

**8.** Composition comprenant au moins 1 partie de GBS80 ou plus sous forme du conformère F pour 1 partie de GBS80 sous forme du conformère A, **caractérisée en ce que** GBS80 sous forme du conformère F est capable de générer une réponse immunitaire sur un sujet, **en ce que** GBS80 sous forme du conformère F n'est pas retenu par une colonne Q-sépharose, et **en ce que** GBS80 sous forme du conformère A est retenu par une colonne Q-sépharose.

**9.** Composition selon l'une quelconque des revendications 6 à 8, étant une composition immunogène, une composition de vaccin ou une composition de diagnostic.

**10.** Composition selon l'une quelconque des revendications 6 à 8, destinée à être utilisée comme produit pharmaceutique.

**11.** Composition selon l'une quelconque des revendications 6 à 8, destinée au traitement ou à la prévention d'une infection par GBS.

**12.** Méthode de séparation d'un GBS80 sous forme du conformère F d'un GBS80 sous forme du conformère A, comprenant :

a) l'obtention d'un échantillon contenant un mélange de GBS80 sous forme du conformère F et de GBS80 sous forme du conformère A ;
b) la séparation de GBS80 sous forme du conformère F de GBS80 sous forme du conformère A en utilisant une technologie de séparation choisie dans le groupe constitué d'une technologie de séparation par échange d'anions, d'une technologie de séparation à base d'hydroxyapatite, et d'une technologie de séparation à base du coefficient de friction,

GBS80 sous forme du conformère F n'étant pas retenu par une colonne Q-sépharose et GBS80 sous forme du conformère A étant retenu par une colonne Q-sépharose.

**13.** Méthode selon la revendication 12, **caractérisée en ce que** la technologie de séparation à base du coefficient de friction est choisie dans le groupe constitué d'électrophorèse sur gel, de chromatographie d'exclusion stérique, de fractionnement par couplage flux-force et de centrifugation de sédimentation à haute vitesse.

**14.** Méthode d'isolement du conformère F de GBS80 comprenant l'application d'un échantillon contenant un mélange de conformères sur une chromatographie d'échange d'ions, la récupération du flux traversant et l'isolement du conformère F avec une étape chromatographique sur hydroxyapatite, GBS80 sous forme du conformère F n'étant pas retenu par une colonne Q-sépharose.

**Figure 1**: SDS-PAGE of GBS 80 purified isoforms

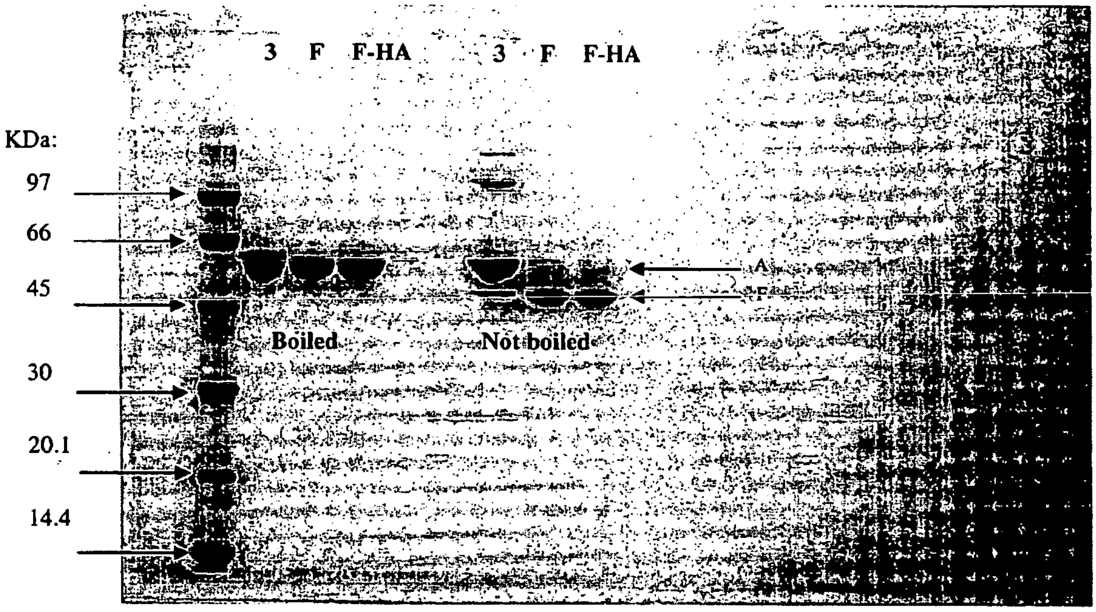

**Figure 2**: Analytical Gel filtration on a Superdex 200 10/30 of the same samples with PBS as buffer, and a flow of 0.5 ml/min.

**Lot F**

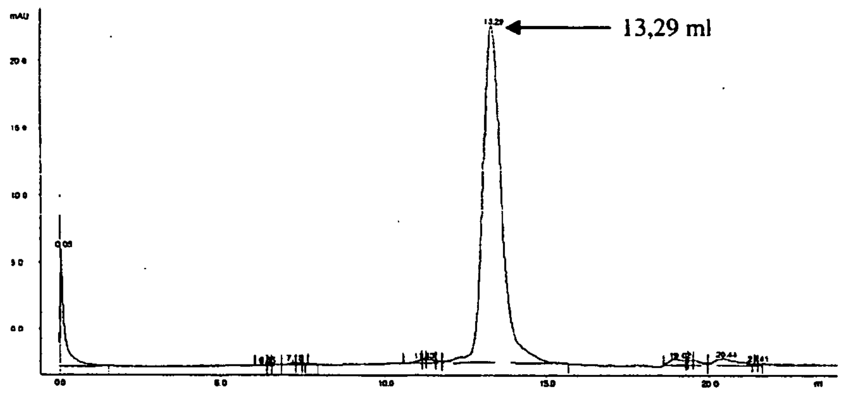


**Lot 3**

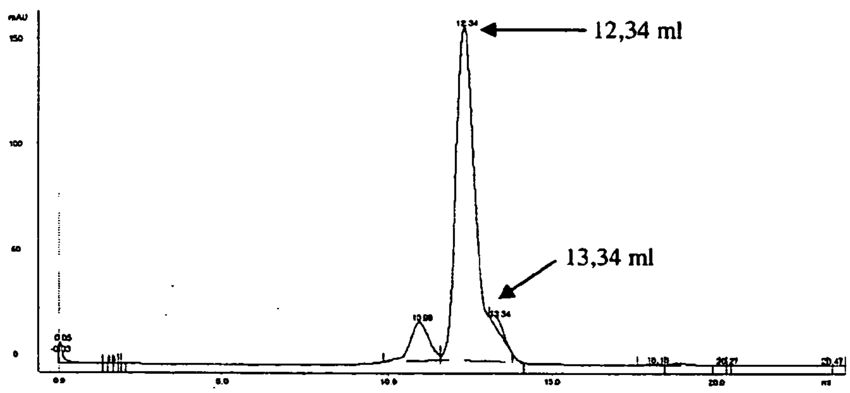

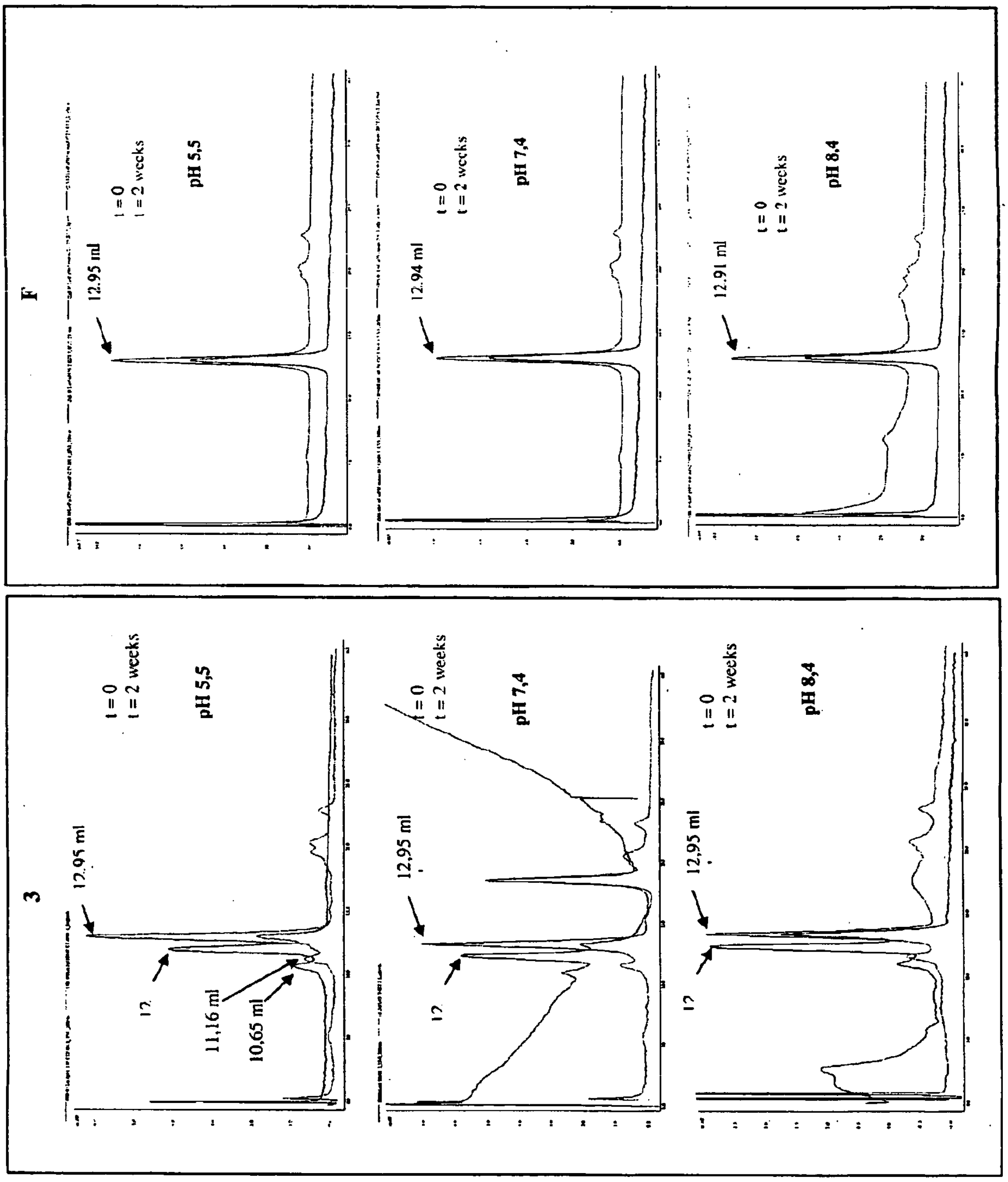


**Figure 3**: Analytical Gel Filtration of lot 3 and lot F at different times and pH.

Figure 4a: Analytical Gel Filtration of 5 different GBS 80 lots

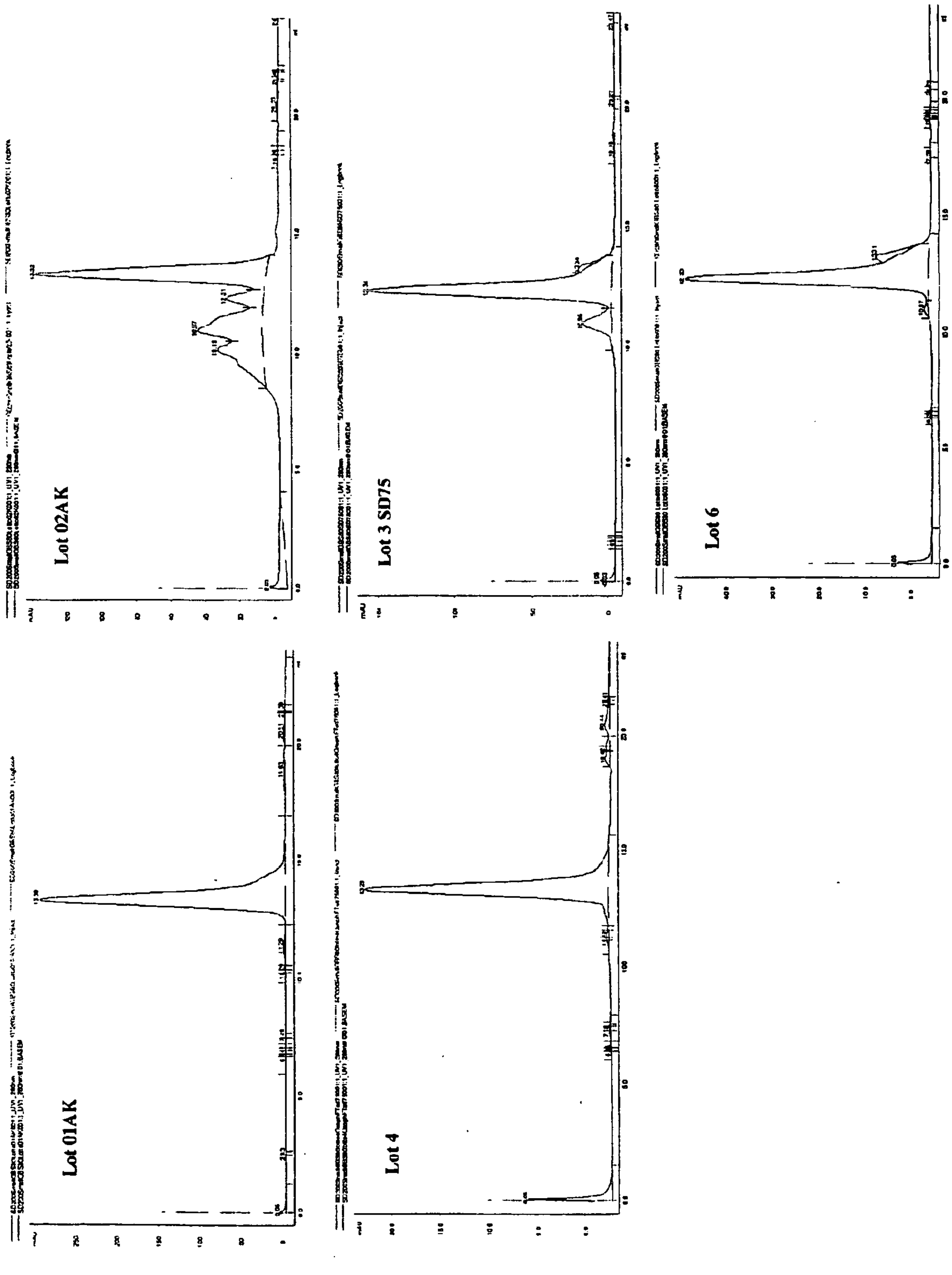

**Figure 4b**: Molecular weights of 5 different GBS80 lots as determined with MALDI-TOF spectrometry.

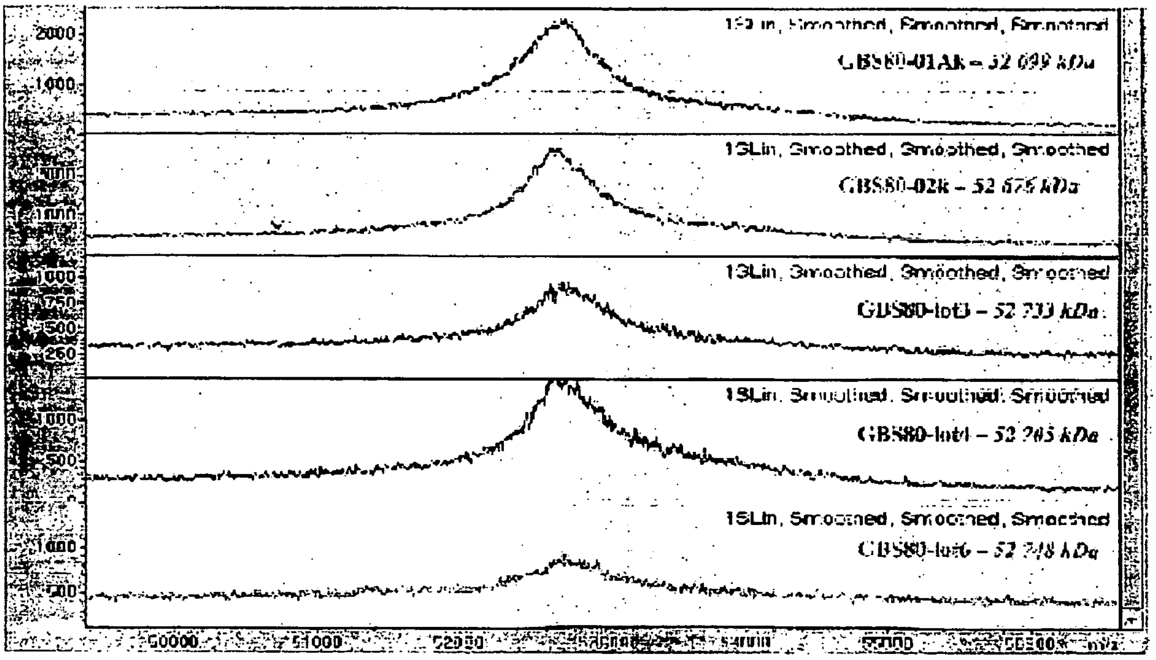

| Lot | Main conformer | MW (kDa) |
| --- | --- | --- |
| Lot 01AK | F | 52699 |
| Lot 02K | A | 52676 |
| Lot 3 | A | 52733 |
| Lot 4 | F | 52705 |
| Lot 6 | A | 52748 |

Figure 5: SDS-PAGE after digestion with different proteases, with and without detergent denaturation (RapiGest SF).

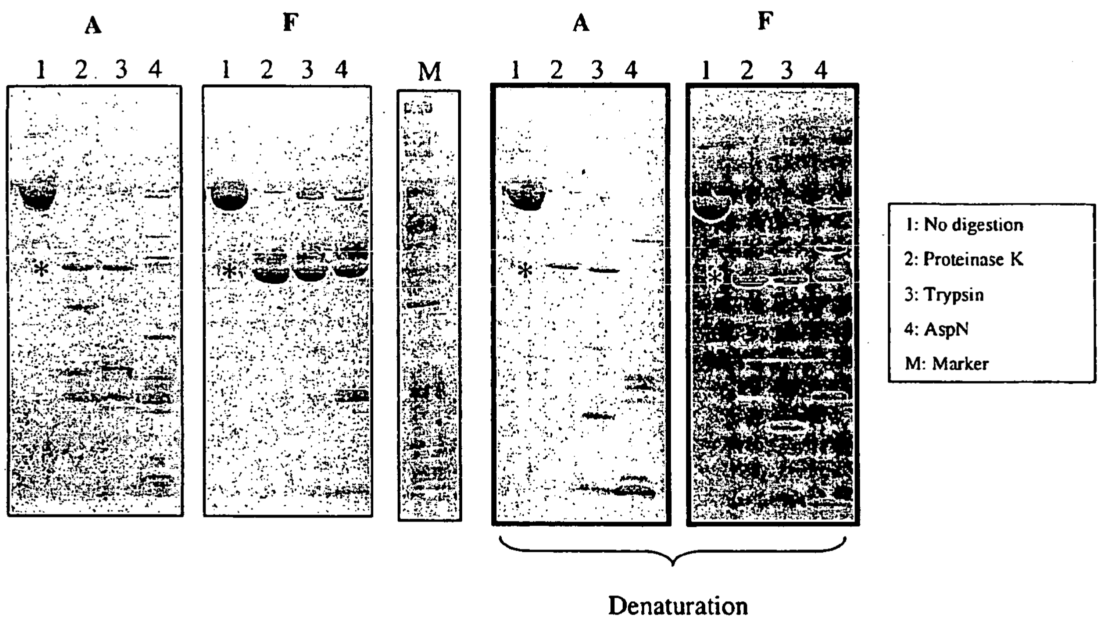

Figure 6: Active maternal immunization results

| | Lot | Protection: COH1 | | Protection: COH1 | |
|---|---|---|---|---|---|
| | | Alive/treated | % | Alive/treated | % |
| GBS 80 | A | 13/62 | 79 | 31/91 | 66 |
| GBS 80 | F | 19/68 | 73 | 9/88 | 90 |
| PBS | | 15/35 | 57 | 58/59 | 2 |

| Challenge | CFU | |
|---|---|---|
| | 160 | 1600 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0234771 A
- WO 05028618 A
- WO 04041157 A
- WO 89046699 A
- US 5693506 A
- US 5659122 A
- US 5608143 A
- US 4738 A
- US 921 A
- EP 0036776 A
- EP 0121775 A
- US 4689406 A
- US 45514331 B
- EP 0267851 A
- EP 0219237 A
- EP 0324647 A, Chey
- US 4336336 A
- EP 0244042 A
- EP 0127328 A
- EP 0036259 A
- EP 0063953 A
- WO 8404541 A
- EP 0136829 A
- EP 0136907 A
- US 4745056 A
- EP 0284044 A
- EP 0329203 A
- US 4876197 A
- US 4880734 A
- EP 0164556 A
- EP 0196056 A
- WO 88024066 A
- EP 0012873 A
- JP 62096086 B
- US 4588684 A
- EP 0060057 A
- US 4546083 A
- US 4870008 A
- EP 0324274 A
- WO 8902463 A
- US 4837148 A
- US 4929555 A
- US 10415182 B
- WO 04099242 A
- US 192046 A
- EP 0477508 A
- US 5306492 A
- WO 9842721 A
- EP 0372501 A
- EP 0378881 A
- EP 0427347 A
- WO 9317712 A
- WO 9403208 A
- WO 9858668 A
- EP 0471177 A
- WO 0056360 A
- WO 9101146 A
- WO 0061761 A
- WO 0172337 A
- US 6355271 B
- WO 0023105 A
- US 5057540 A
- WO 0502620 A
- WO 9633739 A
- EP 0109942 A
- US 4578269 A
- WO 9611711 A
- US 6352697 B
- WO 0007621 A
- US 6506386 B
- WO 0404762 A
- WO 9517211 A
- WO 9842375 A
- WO 9927960 A
- US 6090406 A
- US 5916588 A
- EP 0626169 A
- WO 9952549 A
- WO 0121207 A
- WO 0121152 A
- WO 0272012 A
- WO 0464715 A
- WO 0589837 A
- US 6692468 B
- WO 0007647 A
- WO 9917820 A
- US 5971953 A
- US 4060082 A
- EP 0520618 A
- WO 9801174 A
- WO 9014837 A
- WO 9511700 A
- US 6080725 A
- WO 05097181 A
- US 6630161 B
- WO 02097072 A
- US 4680338 A
- US 4988815 A

- WO 9215582 A
- US 4689338 A
- US 4929624 A
- US 5238944 A
- US 5266575 A
- US 5268376 A
- US 5346905 A
- US 5352784 A
- US 5389640 A
- US 5395937 A
- US 5482936 A
- US 5494916 A
- US 5525612 A
- US 6083505 A
- US 6440992 B
- US 6627640 B
- US 6656938 B
- US 6660735 B
- US 6660747 B
- US 6664260 B
- US 6664264 B
- US 6664265 B
- US 6667312 B
- US 6670372 B
- US 6677347 B
- US 6677348 B
- US 6677349 B
- US 6683088 B
- US 6703402 B
- US 6743920 B
- US 6800624 B
- US 6809203 B
- US 6888000 B
- US 6924293 B
- WO 2004060308 A
- WO 2004064759 A
- US 6924271 B
- US 20050070556 A
- US 5658731 A
- US 5011828 A
- WO 200487153 A
- US 6605617 B
- WO 0218383 A
- WO 20041018455 A
- WO 03082272 A
- US 2005022769 W
- WO 0226757 A
- WO 9962923 A
- WO 98401 A
- US 6207646 B
- US 6239116 B
- US 6429199 B
- WO 0195935 A
- WO 03035836 A
- WO 0122972 A
- GB 2220211 A
- WO 9421292 A
- WO 9400153 A
- WO 9517210 A
- WO 9626741 A
- WO 9319780 A
- WO 03011223 A
- US 6586409 B
- US 20050215517 A
- WO 0122992 A
- WO 9820734 A
- WO 9314778 A
- WO 9011092 A

**Non-patent literature cited in the description**

- **TETTELIN et al.** *Proc. Natl. Acad Sci. USA,* 2002
- Remington's Pharmaceutical Sciences. Mack Publishing Company, 1995
- Methods In Enzymology. Academic Press, Inc,
- Handbook of Experimental Immunology. Blackwell Scientific Publications, 1986, vol. I-IV
- **SAMBROOK et al.** Molecular Cloning: A Laboratory Manual. 1989
- Handbook of Surface and Colloidal Chemistry. CRC Press, 1997
- Short Protocols in Molecular Biology. John Wiley & Sons, 1999
- Molecular Biology Techniques: An Intensive Laboratory. Course. Academic Press, 1998
- PCR (Introduction to Biotechniques Series). Springer Verlag, 1997
- **PETERS ; DALRYMPLE et al.** Fields Virology. B.N. Raven Press
- Expression of Cloned Genes in Mammalian Cells. **SAMBROOK.** Molecular Cloning: A Laboratory Manual. 1989
- **MANIATIS et al.** *Science,* 1987, vol. 236, 1237
- **ALBERTS et al.** Molecular Biology of the Cell. 1989
- **DIJKEMA et al.** *EMBO J.,* 1985, vol. 4, 7611
- **GORMAN et al.** *Proc. Natl. Acad. Sci.,* 1982, vol. 79, 6777
- **BOSHART et al.** *Cell,* 1985, vol. 41, 5211
- **SASSONE-CORSI ; BORELLI.** *Trends Genet.,* 1986, vol. 2, 215
- **BIMSTIEL et al.** *Cell,* 1985, vol. 41, 349
- Termination and 3' end processing of eukaryotic RNA. **PROUDFOOT ; WHITELAW.** Transcription and splicing
- **PROUDFOOT.** *Trends Biochem. Sci.,* 1989, vol. 14, 1051

- Expression of cloned genes in cultured mammalian cells. **SAMBROOK et al.** Molecular Cloning: A Laboratory Manual. 1989
- **GLUZMAN.** *Cell,* 1981, vol. 23, 1751
- **KAUFMAN et al.** *Mol. Cell. Biol.,* 1989, vol. 9, 946
- **SHIMIZU et al.** *Mol. Cell. Biol.,* 1986, vol. 6, 10741
- **SUMMERS ; SMITH.** Texas Agricultural Experiment Station Bulletin No. 1555. 1987
- **LUCKOW ; SUMMERS.** *Virology,* 1989, vol. 17, 31
- **MILLER et al.** *Ann. Rev. Microbiol.,* 1988, vol. 42, 177
- The Regulation of Baculovirus Gene Expression. **FRIESEN et al.** The Molecular Biology of Baculoviruses. 1986
- **VLAK et al.** *J. Gen. Virol.,* 1988, vol. 69, 765
- **CARBONELL et al.** *Gene,* 1988, vol. 73, 409
- **MAEDA et al.** *Nature,* 1985, vol. 315, 592
- **LEBACQ-VERHEYDEN et al.** *Molec. Cell. Biol.,* 1988, vol. 8, 3129
- **SMITH et al.** *Proc. Nat'l Acad. Sci. USA,* 1985, vol. 82, 8404
- **MIYAJIMA et al.** *Gene,* 1987, vol. 58, 273
- **MARTIN et al.** *DNA,* 1988, vol. 7, 99
- **SMITH et al.** *Mol. Cell. Biol.,* 1983, vol. 3, 2156
- **MILLER et al.** *Bioessays,* 1989, vol. 4, 91
- Current Protocols in Microbiology. 1990, vol. 2, 10
- **CARBONELL et al.** *J. Virol.,* 1985, vol. 56, 153
- **WRIGHT.** *Nature,* 1986, vol. 321, 718
- **FRASER et al.** *In Vitro Cell. Dev. Biol.,* 1989, vol. 25, 225
- **ZENK.** *Phytochemistry,* 1991, vol. 30, 3861-3863
- **VAULCOMBE et al.** *Mol. Gen. Genet.,* 1987, vol. 209, 33-40
- **CHANDLER et al.** *Plant Molecular Biology,* 1984, vol. 3, 407-418
- **ROGERS.** *J. Biol. Chem.,* 1985, vol. 260, 3731-3738
- **ROTHSTEIN et al.** *Gene,* 1987, vol. 55, 353-356
- **WHITTIER et al.** *Nucleic Acids Research,* 1987, vol. 15, 2515-2535
- **WIRSEL et al.** *Molecular Microbiology,* 1989, vol. 3, 3-14
- **YU et al.** *Gene,* 1992, vol. 122, 247-253
- **R.L. JONES ; J. MACMILLIN.** Gibberellins: in: Advanced Plant Physiology. Pitman Publishing Limited, 1984, 21-52
- **SHEEN.** *Plant Cell,* 1990, vol. 2, 1027-1038
- **MAAS et al.** *EMBO J.,* 1990, vol. 9, 3447-3452
- **BENKEL ; HICKEY.** *Proc. Natl. Acad. Sci.,* 1987, vol. 84, 1337-1339
- **WILMINK ; DONS.** *Plant Mol. Biol. Reptr,* 1993, vol. 11 (2), 165-185
- **REED ; MANIATIS.** *Cell,* 1985, vol. 41, 95-105
- **CROSSWAY.** *Mol. Gen. Genet,* 1985, vol. 202, 179-185
- **KRENS et al.** *Nature,* 1982, vol. 296, 72-74
- **KLEIN et al.** *Nature,* 1987, vol. 327, 70-73
- **KNUDSEN ; MULLER.** *Planta,* 1991, vol. 185, 330-336
- **FRALEY et al.** *Proc. Natl. Acad. Sci. USA,* 1982, vol. 79, 1859-1863
- **FROMM et al.** *Proc. Natl Acad. Sci. USA,* 1985, vol. 82, 5824
- **RAIBAUD et al.** *Annu. Rev. Genet.,* 1984, vol. 18, 173
- **CHANG et al.** *Nature,* 1977, vol. 198, 1056
- **GOEDDEL et al.** *Nuc. Acids Res.,* 1980, vol. 8, 4057
- **YELVERTON et al.** *Nucl. Acids Res.,* 1981, vol. 9, 731
- **SHIMATAKE et al.** *Nature,* 1981, vol. 292, 128
- **AMANN et al.** *Gene,* 1983, vol. 25, 167
- **DE BOER et al.** *Proc. Natl. Acad. Sci.,* 1983, vol. 80, 21
- **STUDIER et al.** *J. Mol. Biol.,* 1986, vol. 189, 113
- **TABOR et al.** *Proc Natl. Acad. Sci.,* 1985, vol. 82, 1074
- **SHINE et al.** *Nature,* 1975, vol. 254, 34
- Genetic signals and nucleotide sequences in messenger RNA. **STEITZ et al.** Biological Regulation and Development: Gene Expression. 1979
- Expression of cloned genes in Escherichia coli. **SAMBROOK et al.** Molecular Cloning: A Laboratory Manual. 1989
- **NAGAI et al.** *Nature,* 1984, vol. 309, 8101
- **JIA et al.** *Gene,* 1987, vol. 60, 197
- **ALLEN et al.** *J. Biotechnol.,* 1987, vol. 5, 93
- **MAKOFF et al.** *J. Gen. Microbiol.,* 1989, vol. 135, 11
- **MILLER et al.** *Biotechnology,* 1989, vol. 7, 698
- **MASUI et al.** *Experimental Manipulation of Gene Expression,* 1983
- **GHRAYEB et al.** *EMBO J.,* 1984, vol. 3, 2437
- **OKA et al.** *Proc. Natl. Acad. Sci.,* 1985, vol. 82, 7212
- **PALVA et al.** *Proc. Natl. Acad. Sci. USA,* 1982, vol. 79, 5582
- **DAVIES et al.** *Annu. Rev. Microbiol.,* 1978, vol. 32, 469
- **AMANN et al.** *Gene,* 1985, vol. 40, 183
- **POWELL et al.** *Appl. Environ. Microbiol.,* 1988, vol. 54, 655

- **POWELL.** *Appl. Environ. Microbiol.,* 1988, vol. 54, 655
- **MASSON et al.** *FEMS Microbiol. Lett.,* 1989, vol. 60, 273
- **MILLER et al.** *Proc. Natl. Acad. Sci.,* 1988, vol. 85, 856
- **WANG et al.** *J. Bacteriol.,* 1990, vol. 172, 949
- **COHEN et al.** *Proc. Natl. Acad. Sci.,* 1973, vol. 69, 2110
- **DOWER et al.** *Nucleic Acids Res.,* 1988, vol. 16, 6127
- An improved method for transformation of Escherichia coli with ColEI-derived plasmids. **KUSHNER.** Genetic Engineering: Proceedings of the International Symposium on Genetic Engineering. 1978
- **MANDEL et al.** *J. Mol. Biol.,* 1970, vol. 53, 159
- **TAKETO.** *Biochim. Biophys. Acta,* 1988, vol. 949, 318
- **CHASSY et al.** *FEMS Microbiol. Lett.,* 1987, vol. 44, 173
- **FIEDLER et al.** *Anal. Biochem,* 1988, vol. 170, 38
- **AUGUSTIN et al.** *FEMS Microbiol. Lett.,* 1990, vol. 66, 203
- **BARANY et al.** *J. Bacteriol.,* 1980, vol. 144, 698
- Transformation of Streptococcus lactis by electroporation. **HARLANDER.** Streptococcal Genetics. 1987, 111
- **PERRY et al.** *Infect. Immun.,* 1981, vol. 32, 1295
- **SOMKUTI et al.** *Proc. 4th Evr. Cong. Biotechnology,* 1987, vol. 1, 412
- **MYANOHARA et al.** *Proc. Natl. Acad. Sci. USA,* 1983, vol. 80, 1
- **COHEN et al.** *Proc. Natl. Acad. Sci. USA,* 1980, vol. 77, 1078
- **HENIKOFF et al.** *Nature,* 1981, vol. 283, 835
- **HOLLENBERG et al.** *Curr. Topics Microbiol. Immunol.,* 1981, vol. 96, 119
- The Expression of Bacterial Antibiotic Resistance Genes in the Yeast Saccharomyces cerevisiae. **HOLLENBERG et al.** Plasmids of Medical, Environmental and Commercial Importance. 1979
- **MERCERAU-PUIGALON et al.** *Gene,* 1980, vol. 11, 163
- **PANTHIER et al.** *Curr. Genet.,* 1980, vol. 2, 109
- **BOTSTEIN et al.** *Gene,* 1979, vol. 8, 17-24
- **BRAKE et al.** *PNAS USA,* 1984, vol. 81, 4642-4646
- **STINCHCOMB et al.** *J. Mol. Biol.,* 1982, vol. 158, 157
- **ORR-WEAVER et al.** *Methods in Enzymol.,* 1983, vol. 101, 228-245
- **RINE et al.** *Proc. Natl. Acad. Sci. USA,* 1983, vol. 80, 6750
- **KURTZ et al.** *Mol. Cell. Biol.,* 1986, vol. 6, 142
- **KUNZE et al.** *J. Basic Microbiol.,* 1985, vol. 25, 141
- **GLEESON et al.** *J. Gen. Microbiol.,* 1986, vol. 132, 3459
- **ROGGENKAMP et al.** *Mol. Gen. Genet.,* 1986, vol. 202, 302
- **DAS et al.** *J. Bacteriol.,* 1984, vol. 158, 1165
- **DE LOUVENCOURT et al.** *J. Bacteriol.,* 1983, vol. 154, 737
- **VAN DEN BERG et al.** *Biol Technology,* 1990, vol. 8, 135
- **CREGG et al.** *Mol. Cell. Biol.,* 1985, vol. 5, 3376
- **HINNEN et al.** *Proc. Natl. Acad. Sci. USA,* 1978, vol. 75, 1929
- **ITO et al.** *J. Bacteriol.,* 1983, vol. 153, 163
- **BEACH ; NURSE.** *Nature,* 1981, vol. 300, 706
- **DAVIDOW et al.** *Curr. Genet.,* 1985, vol. 10, 39
- **GAILLARDIN et al.** *Curr. Genet.,* 1985, vol. 10, 49
- **DE LOUVENCOURT et al.** *J. Bacteriol.,* 1983, vol. 154, 1165
- **VAN DEN BERG et al.** *BiolTechnology,* 1990, vol. 8, 135
- **ROBERT K. SCOPES.** Protein Purification. Principles and Practice. Springer Verlag, 2000
- *Affinity Chromatography: Principles & Methods,* 2002, 18-102229
- Amersham Biosciences, and Doonan, Protein Purification Protocols. The Humana Press, 1996
- **SULKOWSKI.** *Trends in Biochem.,* 1985, vol. 3, 1
- Meth. Enzymol. 1990, vol. 182, 529
- *Gel Filtration: Principles and Methods,* 2002, 18-102218
- *Ion Exchange Chromatography: Principles and Methods,* 2002, 18-111421
- **SURMAN S et al.** *Proc. Natl. Acad. Sci. USA,* 2001, vol. 98, 4587-92
- **ADORINI L et al.** *J. Exp. Med.,* 1988, vol. 168, 2091
- **SO T. et al.** *Immunol. Let.,* 1996, vol. 49, 91-97
- **GILLIS S et al.** *J. Immunol.,* 1978, vol. 120, 2027
- **RAMSAY et al.** *Lancet,* 2001, vol. 357 (9251), 195-196
- **LINDBERG.** *Vaccine,* 1999, vol. 17 (2), 28-36
- **BUTTERY ; MOXON.** *J R Coll Physicians Lond,* 2000, vol. 34, 163-168
- **AHMAD ; CHAPNICK.** *Infect Dis Clin North Am,* 1999, vol. 13, 113-133

- **GOLDBLATT.** *J. Med. Microbiol.,* 1998, vol. 47, 563-567
- Conjugate Vaccines. vol. 10, 48-114
- **HERMANSON.** *Bioconjugate Techniques,* 1996, ISBN 0123423368
- *Research Disclosure, 453077,* January 2002
- **ROBINSON ; TORRES.** *Seminars in Immunology,* 1997, vol. 9, 271-283
- **DONNELLY et al.** *Annu Rev Immunol,* 1997, vol. 15, 617-648
- **SCOTT-TAYLOR ; DALGLEISH.** *Expert Opin Investig Drugs,* 2000, vol. 9, 471-480
- **APOSTOLOPOULOS ; PLEBANSKI.** *Curr Opin Mol. Ther,* 2000, vol. 2, 441-447
- **ILAN.** *Curr Opin Mol. Ther,* 1999, vol. 1, 116-120
- **DUBENSKY et al.** *Mol. Med,* 2000, vol. 6, 723-732
- **ROBINSON ; PERTMER.** *Adv Virus Res,* 2000, vol. 55, 1-74
- **DONNELLY et al.** *Am J Respir Crit Care Med,* 2000, vol. 162, 190-193
- **DAVIS.** *Mt. Sinai J. Med.,* 1999, vol. 66, 84-90
- Vaccine Design: The Subunit and Adjuvant Approach. Plenum Press, 1995
- **BARR et al.** *Advanced Drug Delivery Reviews,* 1998, vol. 32, 247-271
- **SJOLANDERET et al.** *Advanced Drug Delivery Reviews,* 1998, vol. 32, 321-338
- **PIZZA et al.** *Int J Med Microbiol,* 2000, vol. 290, 455-461
- **SINGH et al.** *J Cont Release,* 2001, vol. 70, 267-276
- **SIGNORELLI ; HADDEN.** *Int Immunopharmacol,* 2003, vol. 3 (8), 1177-86
- **COOPER.** *Pharm Biotechnol,* 1995, vol. 6, 559-80
- Vaccine Adjuvants: Preparation Methods and Research Protocols. Methods in Molecular Methods series. vol. 42
- **PODDA ; DEL GIUDICE.** *Expert Rev Vaccines,* 2003, vol. 2, 197-203
- **PODDA.** *Vacccine,* 2001, vol. 19, 2673-2680
- **ALLISON ; BYARS.** *Res Immunol,* 1992, vol. 143, 519-25
- **HARIHARAN et al.** *Cancer Res,* 1995, vol. 55, 3486-9
- **HAN et al.** *Impact of Vitamin E on Immune Function and Infectious Diseases in the Aged at Nutrition, Immune functions and Health EuroConference,* 09 June 2005
- **HAYDEN et al.** *J Clin Invest,* 1998, vol. 101 (3), 643-9
- **TASSIGNON et al.** *J Immunol Meth,* 2005, vol. 305, 188-98
- **MYERS et al.** *Cellular and molecular aspects of endotoxin reactions,* 1990, 145-156
- Vaccine Adjuvants: Preparation Methods and Research Protocols. Methods in Molecular Methods series. vol. 42, 273-282
- **JOHNSON et al.** *J Med Chem,* 1999, vol. 42, 4640-9
- **BALDRICK et al.** *Regulatory Toxicol Pharmacol,* 2002, vol. 35, 398-413
- **STANLEY.** *Clin Exp Dermatol,* 2002, vol. 27, 571-577
- **WU et al.** *Antiviral Res.,* 2004, vol. 64 (2), 79-83
- **VASILAKOS et al.** *Cell Immunol.,* 2000, vol. 204 (1), 64-74
- **JONES.** *Curr Opin Investig Drugs,* 2003, vol. 4, 214-218
- **JOHNSON et al.** *Bioorg Med Chem Lett,* 1999, vol. 9, 2273-2278
- **EVANS et al.** *Expert Rev Vaccines,* 2003, vol. 2, 219-229
- **ANDRIANOV et al.** *Biomaterials,* 1998, vol. 19, 109-115
- **PAYNE et al.** *Adv Drug Delivery Review,* 1998, vol. 31, 185-196
- **THOMPSON et al.** *Methods in Molecular Medicine,* 2003, vol. 94, 255-266
- **KANDIMALLA et al.** *Nucleic Acids Research,* 2003, vol. 31, 2393-2400
- **KRIEG.** *Nature Medicine,* 2003, vol. 9, 831-835
- **MCCLUSKIE et al.** *FEMS Immunology and Medical Microbiology,* 2002, vol. 32, 179-185
- **KANDIMALLA et al.** *Biochemical Society Transactions,* 2003, vol. 31, 654-658
- **BLACKWELL et al.** *J Immunol,* 2003, vol. 170, 4061-4068
- **KRIEG.** *Trends Immunol,* 2002, vol. 23, 64-65
- **KANDIMALLA et al.** *BBRC,* 2003, vol. 306, 948-953
- **BHAGAT et al.** *BBRC,* 2003, vol. 300, 853-861
- **THOMPSON et al.** The low-toxicity versions of LPS, MPL® adjuvant and RC529, are efficient adjuvants for CD4+ T cells. *J Leukoc Biol,* 2005, 78
- **MERALDI et al.** *Vaccine,* 2003, vol. 21, 2485-2491
- **PAJAK et al.** *Vaccine,* 2003, vol. 21, 836-842
- **WONG et al.** *J Clin Pharmacol,* 2003, vol. 43 (7), 735-42
- Vaccine Design: The Subunit and Adjuvant Approach. Plenum Press
- **KOHLER ; MILSTEIN.** *Nature,* 1975, vol. 256, 495-96
- Remington's Pharmaceutical Sciences. Mack Pub. Co, 1991
- **HUG ; SLEIGHT.** *Biochim. Biophys. Acta,* 1991, vol. 1097, 1-17
- **STRAUBINGER.** *Meth. Enzymol.,* 1983, vol. 101, 512-527

- **FEIGNER.** *Proc. Natl. Acad. Sci. USA,* 1987, vol. 84, 7413-7416
- **MALONE.** *Proc. Natl. Acad. Sci. USA,* 1989, vol. 86, 6077-6081
- **DEBS.** *J. Biol. Chem.,* 1990, vol. 265, 10189-10192
- **SZOKA.** *Proc. Natl. Acad. Sci. USA,* 1978, vol. 75, 4194-4198
- **STRAUBINGER.** *Meth. Immunol.,* 1983, vol. 101, 512-527
- **SZOKA.** *Proc. Nall. Acad. Sci. USA,* 1978, vol. 75, 4194-4198
- **PAPAHADJOPOULOS.** *Biochim. Biophys. Acta,* 1975, vol. 394, 483
- **WILSON.** *Cell,* 1979, vol. 17, 77
- **DEAMER ; BANGHAM.** *Biochim. Biophys. Acta,* 1976, vol. 443, 629
- **OSTRO.** *Biochem. Biophys. Res. Commun.,* 1977, vol. 76, 836
- **FRALEY.** *Proc. Natl. Acad. Sci. USA,* 1979, vol. 76, 3348
- **ENOCH ; STRITTMATTER.** *Proc. Natl. Acad. Sci. USA,* 1979, vol. 76, 145
- **FRALEY.** *J. Biol. Chem.,* 1980, vol. 255, 10431
- **SZOKA ; PAPAHADJOPOULOS.** *Proc. Natl. Acad. Sci. USA,* 1978, vol. 75, 145
- **SCHAEFER- RIDDER.** *Science,* 1982, vol. 215, 166
- **BRESLOW.** *Annu Rev. Biochem,* 1985, vol. 54, 699
- **LAW.** *Adv. Exp Med. Biol.,* 1986, vol. 151, 162
- **CHEN.** *J Biol Chem,* 1986, vol. 261, 12918
- **KANE.** *Proc Natl Acad Sci USA,* 1980, vol. 77, 2465
- **UTERMANN.** *Hum Genet,* 1984, vol. 65, 232
- *Meth. Enzymol.,* 1986, 128
- **PITAS.** *J. Biochem.,* 1980, vol. 255, 5454-5460
- **MAHEY.** *J Clin. Invest,* 1979, vol. 64, 743-750
- **ATKINSON.** *Annu Rev Biophys Chem,* 1986, vol. 15, 403
- **RADDING.** *Biochim BiophysActa,* 1958, vol. 30, 443